# EUROPEAN PATENT APPLICATION

(11) **EP 1 443 055 A2**
(43) Date of publication of application: **04.08.2004**
(21) Application number: 04000756.9
(22) Date of filing: 27.05.1999
(51) Int. Cl.: C07K 14/54, C12N 15/24, C07H 21/04, A61K 38/20

(54) **Interleukins-21 and 22**

(30) Priority: 29.05.1998 US 87340 P; 10.09.1998 US 99805 P; 30.04.1999 US 131965 P
(62) Divisional of application: 99925886.6
(71) Applicant: Human Genome Sciences, Inc., Rockville, MD 20850 (US)
(72) Inventor: Ruben, Steven M., Brookeville, MD 20833 (US); Ebner, Reinhard, Gaithersburg, MD 20878 (US)
(74) Representative: VOSSIUS & PARTNER

(57) **Abstract**

Described is the human protein designated interleukin-22 (IL-22) and the polynucleotide encoding this protein. Also described are vectors, host cells, antibodies, and recombinant methods producing this human protein. Further, described are diagnostic methods and pharmaceutical compositions useful for diagnosing and treating disorders related to this human protein.

SEQUENCE LISTING

<110> Ebner, Reinhard
      Ruben, Steven

<120> Interleukins-21 and 22

<130> E2897 EP/1 S3

<150> 99 925 886.6
<151> 2000-12-21

<150> 60/169,837
<151> 1999-12-09

<150> 09/320,713
<151> 1999-05-27

<150> 60/087,340
<151> 1998-05-29

<150> 60/099,805
<151> 1998-09-10

<150> 60/131,965
<151> 1999-04-30

<150> WO 99/61617
<151> 1999-05-27

<160> 32

<170> PatentIn version 3.0

<210> 1
<211> 705
<212> DNA
<213> HOMO SAPIENS

<220>
<221> CDS
<222> (2)..(262)

<400> 1
g gca cga gtg gac acg gat gag gac cgc tat cca cag aag ctg gcc ttc      49
  Ala Arg Val Asp Thr Asp Glu Asp Arg Tyr Pro Gln Lys Leu Ala Phe
  1               5                   10                  15

gcc gag tgc ctg tgc aga ggc tgt atc gat gca cgg acg ggc cgc gag      97
Ala Glu Cys Leu Cys Arg Gly Cys Ile Asp Ala Arg Thr Gly Arg Glu
            20                  25                  30

aca gct gcg ctc aac tcc gtg cgg ctg ctc cag agc ctg ctg gtg ctg      145
Thr Ala Ala Leu Asn Ser Val Arg Leu Leu Gln Ser Leu Leu Val Leu
        35                  40                  45

cgc cgc cgg ccc tgc tcc cgc gac ggc tcg ggg ctc ccc aca cct ggg      193
Arg Arg Arg Pro Cys Ser Arg Asp Gly Ser Gly Leu Pro Thr Pro Gly
    50                  55                  60

gcc ttt gcc ttc cac acc gag ttc atc cac gtc ccc gtc ggc tgc acc     241
Ala Phe Ala Phe His Thr Glu Phe Ile His Val Pro Val Gly Cys Thr
65                  70                  75                  80

tgc gtg ctg ccc cgt tca gtg tgaccgccaa ggccgtgggg cccttagact     292
Cys Val Leu Pro Arg Ser Val
                85

ggacacgtgt gctccccaga gggcaccccc tatttatgtg tatttattgt tatttatatg     352

cctcccccaa cactaccctt ggggtctggg cattccccgt gtctggagga cagcccccca     412

ctgttctcct catctccagc ctcagtagtt gggggtwgaa ggagctcagc acctcttcca     472

gcccttaaag ctgcagaaaa ggtgtcacac ggctgcctgt accttggytc cctgtcctgc     532

tcccggcttc ccttacccta tcactggcct caggcccccg caggctgcct cttcccaacc     592

tccttggaag tacccctgtt tcttaaacaa ttatttaagt gtacgtgtat tattaaactg     652

atgaacacaa aaaaaaaaaa aaaaaaaaaa aaaaaaaaaa aaaaaaaaaa aaa     705

<210>  2
<211>  87
<212>  PRT
<213>  HOMO SAPIENS

<400>  2

Ala Arg Val Asp Thr Asp Glu Asp Arg Tyr Pro Gln Lys Leu Ala Phe
1               5                   10                  15

Ala Glu Cys Leu Cys Arg Gly Cys Ile Asp Ala Arg Thr Gly Arg Glu
            20                  25                  30

Thr Ala Ala Leu Asn Ser Val Arg Leu Leu Gln Ser Leu Leu Val Leu
        35                  40                  45

Arg Arg Arg Pro Cys Ser Arg Asp Gly Ser Gly Leu Pro Thr Pro Gly
    50                  55                  60

Ala Phe Ala Phe His Thr Glu Phe Ile His Val Pro Val Gly Cys Thr
65                  70                  75                  80

Cys Val Leu Pro Arg Ser Val
                85

<210>  3
<211>  1641
<212>  DNA
<213>  HOMO SAPIENS

<220>

<221>  CDS
<222>  (3)..(482)

<400>  3
gg aat tcg gca cga gct cgt gcc gtg ctc agt gcc ttc cac cac acg        47
   Asn Ser Ala Arg Ala Arg Ala Val Leu Ser Ala Phe His His Thr
   1               5                   10                  15

ctg cag ctg ggg ccg cgt gag cag gcg cgc aac gcg agc tgc ccg gca        95
Leu Gln Leu Gly Pro Arg Glu Gln Ala Arg Asn Ala Ser Cys Pro Ala
                20                  25                  30

ggg ggc agg ccc gcc gac cgc cgc ttc cgg ccg ccc acc aac ctg cgc       143
Gly Gly Arg Pro Ala Asp Arg Arg Phe Arg Pro Pro Thr Asn Leu Arg
            35                  40                  45

agc gtg tcg ccc tgg gcc tac aga atc tcc tac gac ccg gcg agg tac       191
Ser Val Ser Pro Trp Ala Tyr Arg Ile Ser Tyr Asp Pro Ala Arg Tyr
        50                  55                  60

ccc agg tac ctg cct gaa gcc tac tgc ctg tgc cgg ggc tgc ctg acc       239
Pro Arg Tyr Leu Pro Glu Ala Tyr Cys Leu Cys Arg Gly Cys Leu Thr
    65                  70                  75

ggg ctg ttc ggc gag gag gac gtg cgc ttc cgc agc gcc cct gtc tac       287
Gly Leu Phe Gly Glu Glu Asp Val Arg Phe Arg Ser Ala Pro Val Tyr
80                  85                  90                  95

atg ccc acc gtc gtc ctg cgc cgc acc ccc gcc tgc gcc ggc ggc cgt       335
Met Pro Thr Val Val Leu Arg Arg Thr Pro Ala Cys Ala Gly Gly Arg
                100                 105                 110

tcc gtc tac acc gag gcc tac gtc acc atc ccc gtg ggc tgc acc tgc       383
Ser Val Tyr Thr Glu Ala Tyr Val Thr Ile Pro Val Gly Cys Thr Cys
            115                 120                 125

gtc ccc gag ccg gag aag gac gca gac agc atc aac tcc agc atc gac       431
Val Pro Glu Pro Glu Lys Asp Ala Asp Ser Ile Asn Ser Ser Ile Asp
        130                 135                 140

aaa cag ggc gcc aag ctc ctg ctg ggc ccc aac gac gcg ccc gct ggc       479
Lys Gln Gly Ala Lys Leu Leu Leu Gly Pro Asn Asp Ala Pro Ala Gly
    145                 150                 155

ccc tgaggccggt cctgccccgg gaggtctccc cggcccgcat cccgaggcgc            532
Pro
160

ccaagctgga gccgcctgga gggctcggtc ggcgacctct gaagagagtg caccgagcaa     592

accaagtgcc ggagcaccag cgccgccttt ccatggagac tcgtaagcag cttcatctga     652

cacgggcatc cctggcttgc ttttagctac aagcaagcag cgtggctgga agctgatggg     712

aaacgacccg gcacgggcat cctgtgtgcg gcccgcatgg agggtttgga aaagttcacg     772

gaggctccct gaggagcctc tcagatcggc tgctgcgggt gcagggcgtg actcaccgct     832

gggtgcttgc caaagagata gggacgcata tgctttttaa agcaatctaa aaataataat     892

aagtatagcg actatatacc tacttttaaa atcaactgtt ttgaatagag gcagagctat 952

tttatattat caaatgagag ctactctgtt acatttctta acatataaac atcgttttta 1012

cttcttctgg tagaattttt taaagcataa ttggaatcct tggataaatt ttgtagctgg 1072

tacactctgg cctgggtctc tgaattcagc ctgtcaccga tggctgactg atgaaatgga 1132

cacgtctcat ctgacccact cttccttcca ctgaaggtct tcacgggcct ccaggtggac 1192

caaagggatg cacaggcggc tcgcatgccc cagggccagc taagagttcc aaagatctca 1252

gatttggttt tagtcatgaa tacataaaca gtctcaaact cgcacaattt tttccccctt 1312

ttgaaagcca ctggggccaa tttgtggtta agaggtggtg agataagaag tggaacgtga 1372

catctttgcc agttgtcaga agaatccaag caggtattgg cttagttgta agggctttag 1432

gatcaggctg aatatgagga caaagtgggc cacgttagca tctgcagaga tcaatctgga 1492

ggcttctgtt tctgcattct gccacgagag ctaggtcctt gatcttttct ttagattgaa 1552

agtctgtctc tgaacacaat tatttgtaaa agttagtagt tcttttttaa atcattaaaa 1612

gaggcttgct gaaaaaaaaa aaaaaaaaa 1641

<210> 4
<211> 160
<212> PRT
<213> HOMO SAPIENS

<400> 4

Asn Ser Ala Arg Ala Arg Ala Val Leu Ser Ala Phe His His Thr Leu
1               5                   10                  15

Gln Leu Gly Pro Arg Glu Gln Ala Arg Asn Ala Ser Cys Pro Ala Gly
            20                  25                  30

Gly Arg Pro Ala Asp Arg Arg Phe Arg Pro Pro Thr Asn Leu Arg Ser
        35                  40                  45

Val Ser Pro Trp Ala Tyr Arg Ile Ser Tyr Asp Pro Ala Arg Tyr Pro
    50                  55                  60

Arg Tyr Leu Pro Glu Ala Tyr Cys Leu Cys Arg Gly Cys Leu Thr Gly
65                  70                  75                  80

Leu Phe Gly Glu Glu Asp Val Arg Phe Arg Ser Ala Pro Val Tyr Met
                85                  90                  95

Pro Thr Val Val Leu Arg Arg Thr Pro Ala Cys Ala Gly Gly Arg Ser
            100                 105                 110

Val Tyr Thr Glu Ala Tyr Val Thr Ile Pro Val Gly Cys Thr Cys Val
        115                 120                 125

Pro Glu Pro Glu Lys Asp Ala Asp Ser Ile Asn Ser Ser Ile Asp Lys
    130                 135                 140

Gln Gly Ala Lys Leu Leu Leu Gly Pro Asn Asp Ala Pro Ala Gly Pro
145                 150                 155                 160

<210>  5
<211>  155
<212>  PRT
<213>  HOMO SAPIENS

<400>  5

Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155

<210>  6
<211>  158
<212>  PRT
<213>  MUS MUSCULUS

<400>  6

Met Ser Pro Gly Arg Ala Ser Ser Val Ser Leu Met Leu Leu Leu Leu
1               5                   10                  15

Leu Ser Leu Ala Ala Thr Val Lys Ala Ala Ala Ile Ile Pro Gln Ser
            20                  25                  30

Ser Ala Cys Pro Asn Thr Glu Ala Lys Asp Phe Leu Gln Asn Val Lys
        35                  40                  45

Val Asn Leu Lys Val Phe Asn Ser Leu Gly Ala Lys Val Ser Ser Arg
    50                  55                  60

Arg Pro Ser Asp Tyr Leu Asn Arg Ser Thr Ser Pro Trp Thr Leu His
65                  70                  75                  80

Arg Asn Glu Asp Pro Asp Arg Tyr Pro Ser Val Ile Trp Glu Ala Gln
                85                  90                  95

Cys Arg His Gln Arg Cys Val Asn Ala Glu Gly Lys Leu Asp His His
            100                 105                 110

Met Asn Ser Val Leu Ile Gln Gln Glu Ile Leu Val Leu Lys Arg Glu
        115                 120                 125

Pro Glu Ser Cys Pro Phe Thr Phe Arg Val Glu Lys Met Leu Val Gly
    130                 135                 140

Val Gly Cys Thr Cys Val Ala Ser Ile Val Arg Gln Ala Ala
145                 150                 155

<210> 7
<211> 151
<212> PRT
<213> VIRAL

<400> 7

Met Thr Phe Arg Met Thr Ser Leu Val Leu Leu Leu Leu Leu Ser Ile
1               5                   10                  15

Asp Cys Ile Val Lys Ser Glu Ile Thr Ser Ala Gln Thr Pro Arg Cys
            20                  25                  30

Leu Ala Ala Asn Asn Ser Phe Pro Arg Ser Val Met Val Thr Leu Ser
        35                  40                  45

Ile Arg Asn Trp Asn Thr Ser Ser Lys Arg Ala Ser Asp Tyr Tyr Asn
    50                  55                  60

Arg Ser Thr Ser Pro Trp Thr Leu His Arg Asn Glu Asp Gln Asp Arg
65                  70                  75                  80

Tyr Pro Ser Val Ile Trp Glu Ala Lys Cys Arg Tyr Leu Gly Cys Val
                85                  90                  95

Asn Ala Asp Gly Asn Val Asp Tyr His Met Asn Ser Val Pro Ile Gln
            100                 105                 110

Gln Glu Ile Leu Val Val Arg Lys Gly His Gln Pro Cys Pro Asn Ser
        115                 120                 125

Phe Arg Leu Glu Lys Met Leu Val Thr Val Gly Cys Thr Cys Val Thr

130                 135                 140

Pro Ile Val His Asn Val Asp
145                 150

<210>  8
<211>  180
<212>  PRT
<213>  HOMO SAPIENS

<400>  8

Met Asp Trp Pro His Asn Leu Leu Phe Leu Leu Thr Ile Ser Ile Phe
1               5                   10                  15

Leu Gly Leu Gly Gln Pro Arg Ser Pro Lys Ser Lys Arg Lys Gly Gln
            20                  25                  30

Gly Arg Pro Gly Pro Leu Ala Pro Gly Pro His Gln Val Pro Leu Asp
        35                  40                  45

Leu Val Ser Arg Met Lys Pro Tyr Ala Arg Met Glu Glu Tyr Glu Arg
    50                  55                  60

Asn Ile Glu Glu Met Val Ala Gln Leu Arg Asn Ser Ser Glu Leu Ala
65                  70                  75                  80

Gln Arg Lys Cys Glu Val Asn Leu Gln Leu Trp Met Ser Asn Lys Arg
                85                  90                  95

Ser Leu Ser Pro Trp Gly Tyr Ser Ile Asn His Asp Pro Ser Arg Ile
            100                 105                 110

Pro Val Asp Leu Pro Glu Ala Arg Cys Leu Cys Leu Gly Cys Val Asn
        115                 120                 125

Pro Phe Thr Met Gln Glu Asp Arg Ser Met Val Ser Val Pro Val Phe
    130                 135                 140

Ser Gln Val Pro Val Arg Arg Arg Leu Cys Pro Pro Pro Pro Arg Thr
145                 150                 155                 160

Gly Pro Cys Arg Gln Arg Ala Val Met Glu Thr Ile Ala Val Gly Cys
                165                 170                 175

Thr Cys Ile Phe
            180

<210>  9
<211>  45
<212>  DNA
<213>  OLIGONUCLEOTIDE

<220>
<221>  primer_bind
<222>  (1)..(45)
<223>  5' primer containing a BamHI restriction site followed by
       several nucleotides of the amino terminal coding
       sequence of mature IL-21 sequence

<400>  9
gatcgcggat ccgacacgga tgaggaccgc tatccacaga agctg                    45


<210>  10
<211>  41
<212>  DNA
<213>  OLIGONUCLEOTIDE

<220>
<221>  primer_bind
<222>  (1)..(41)
<223>  3' primer containing an HindIII restriciton site followed by
       several nucleotides complementary to the 3' end of the
       coding sequence of IL-21 DNA sequence.


<400>  10
cccaagcttt cacactgaac ggggcagcac gcaggtgcag c                        41


<210>  11
<211>  35
<212>  DNA
<213>  OLIGONUCLEOTIDE

<220>
<221>  primer_bind
<222>  (1)..(35)
<223>  5' primer containing a BamHI restriction site, a "C" residue
       to preserve the reading frame, and 16 nucleotides of the
       sequence of the complete IL-21 protein.


<400>  11
cgccgcggat ccgccatccg cacgagtgga cacgg                               35


<210>  12
<211>  29
<212>  DNA
<213>  OLIGONUCLEOTIDE

<220>
<221>  primer_bind
<222>  (1)..(29)
<223>  3' primer containing an Asp718 restriction site, and 20
       nucleotides complementary to the 3' noncoding
       sequence of IL-21.


<400>  12
cgcggtaccc actgaacggg gcagcacgc                                      29


<210>  13
<211>  733
<212>  DNA
<213>  HOMO SAPIENS

<400> 13
gggatccgga gcccaaatct tctgacaaaa ctcacacatg cccaccgtgc ccagcacctg 60

aattcgaggg tgcaccgtca gtcttcctct tccccccaaa acccaaggac accctcatga 120

tctcccggac tcctgaggtc acatgcgtgg tggtggacgt aagccacgaa gaccctgagg 180

tcaagttcaa ctggtacgtg gacggcgtgg aggtgcataa tgccaagaca aagccgcggg 240

aggagcagta caacagcacg taccgtgtgg tcagcgtcct caccgtcctg caccaggact 300

ggctgaatgg caaggagtac aagtgcaagg tctccaacaa agccctccca acccccatcg 360

agaaaaccat ctccaaagcc aaagggcagc cccgagaacc acaggtgtac accctgcccc 420

catcccggga tgagctgacc aagaaccagg tcagcctgac ctgcctggtc aaaggcttct 480

atccaagcga catcgccgtg gagtgggaga gcaatgggca gccggagaac aactacaaga 540

ccacgcctcc cgtgctggac tccgacggct ccttcttcct ctacagcaag ctcaccgtgg 600

acaagagcag gtggcagcag gggaacgtct tctcatgctc cgtgatgcat gaggctctgc 660

acaaccacta cacgcagaag agcctctccc tgtctccggg taaatgagtg cgacggccgc 720

gactctagag gat 733

<210> 14
<211> 5
<212> PRT
<213> HOMO SAPIENS

<220>
<221> SITE
<222> (1)..(5)
<223> Xaa is equal to any amino acid

<400> 14

Trp Ser Xaa Trp Ser
1               5

<210> 15
<211> 86
<212> DNA
<213> OLIGONUCLEOTIDE

<220>
<221> primer_bind
<222> (1)..(86)
<223> 5' primer containing 14 tandem copies of the GAS-binding
site, 18bp of complementary sequence to SV40 early
promotor sequence, and is flanked with an XhoI
restriction site.

<400> 15

gcgcctcgag atttccccga aatctagatt tccccgaaat gatttccccg aaatgatttc 60

cccgaaatat ctgccatctc aattag 86

<210> 16
<211> 27
<212> DNA
<213> OLIGONUCLEOTIDE

<220>
<221> primer_bind
<222> (1)..(27)
<223> 3' primer containing a complementary sequence to SV40 promotor
and flanked with an Hin dIII site.

<400> 16
   gcggcaagct ttttgcaaag cctaggc 27

<210> 17
<211> 271
<212> DNA
<213> HOMO SAPIENS

<400> 17
ctcgagattt ccccgaaatc tagatttccc cgaaatgatt tccccgaaat gatttccccg 60

aaatatctgc catctcaatt agtcagcaac catagtcccg cccctaactc cgcccatccc 120

gcccctaact ccgcccagtt ccgcccattc tccgccccat ggctgactaa ttttttttat 180

ttatgcagag gccgaggccg cctcggcctc tgagctattc cagaagtagt gaggaggctt 240

ttttggaggc ctaggctttt gcaaaaagct t 271

<210> 18
<211> 32
<212> DNA
<213> OLIGONUCLEOTIDE

<220>
<221> primer_bind
<222> (1)..(32)
<223> 5' PCR primer.

<400> 18
gcgctcgagg gatgacagcg atagaacccc gg 32

<210> 19
<211> 31
<212> DNA
<213> OLIGONUCLEOTIDE

<220>
<221> primer_bind

<222>  (1)..(31)
<223>  3' PCR primer.


<400>  19
gcgaagcttc gcgactcccc ggatccgcct c                                    31


<210>  20
<211>  12
<212>  DNA
<213>  OLIGONUCLEOTIDE

<220>
<221>  primer_bind
<222>  (1)..(12)
<223>  NF-kappaB binding site.


<400>  20
ggggactttc cc                                                         12


<210>  21
<211>  73
<212>  DNA
<213>  OLIGONUCLEOTIDE

<220>
<221>  primer_bind
<222>  (1)..(73)
<223>  5' primer containing 4 tandem copeies of the NF-kappaB
       binding site, 18bp of complementary sequence to 5' end  of
       SV40 promotor sequence, flanked by an XhoI restriction site.


<400>  21
gcggcctcga ggggactttc ccggggactt tccggggact ttccgggact ttccatcctg     60

ccatctcaat tag                                                        73


<210>  22
<211>  27
<212>  DNA
<213>  OLIGONUCLEOTIDE

<220>
<221>  primer_bind
<222>  (1)..(27)
<223>  3' primer containing sequence complementary to the 3' end of the
       SV40 promotor and flanked by an HindIII restriction site.


<400>  22
   gcggcaagct ttttgcaaag cctaggc                                          27


<210>  23
<211>  256

<212>  DNA
<213>  HOMO SAPIENS

<400>  23
ctcgagggga ctttcccggg gactttccgg ggactttccg ggactttcca tctgccatct      60

caattagtca gcaaccatag tcccgcccct aactccgccc atcccgcccc taactccgcc     120

cagttccgcc cattctccgc cccatggctg actaattttt tttatttatg cagaggccga     180

ggccgcctcg gcctctgagc tattccagaa gtagtgagga ggcttttttg gaggcctagg     240

cttttgcaaa aagctt                                                     256


<210>  24
<211>  371
<212>  DNA
<213>  HOMO SAPIENS

<220>
<221>  misc_feature
<222>  (1)..(1)
<223>  n is equal to any a, t, c, or g


<220>
<221>  misc_feature
<222>  (11)..(11)
<223>  n is equal to any a, t, c, or g


<220>
<221>  misc_feature
<222>  (16)..(16)
<223>  n is equal to any a, t, c, or g


<220>
<221>  misc_feature
<222>  (31)..(31)
<223>  n is equal to any a, t, c, or g


<220>
<221>  misc_feature
<222>  (37)..(37)
<223>  n is equal to any a, t, c, or g


<220>
<221>  misc_feature
<222>  (79)..(79)
<223>  n is equal to any a, t, c, or g


<220>
<221>  misc_feature
<222>  (154)..(154)
<223>  n is equal to any a, t, c, or g

<220>
<221>  misc_feature
<222>  (293)..(293)
<223>  n is equal to any a, t, c, or g


<220>
<221>  misc_feature
<222>  (320)..(320)
<223>  n is equal to any a, t, c, or g


<220>
<221>  misc_feature
<222>  (322)..(322)
<223>  n is equal to any a, t, c, or g


<220>
<221>  misc_feature
<222>  (329)..(329)
<223>  n is equal to any a, t, c, or g


<220>
<221>  misc_feature
<222>  (337)..(337)
<223>  n is equal to any a, t, c, or g


<220>
<221>  misc_feature
<222>  (344)..(344)
<223>  n is equal to any a, t, c, or g


<220>
<221>  misc_feature
<222>  (362)..(362)
<223>  n is equal to any a, t, c, or g


<400>  24
naattcggca nagggngaaa cgacccggca ngcgatncct gtgtgcggcc cgcatggagg      60

gtttggaaaa gttcacggng gctccctgag gacctgcgag aatcgggctg ctgcgggtgc     120

aaggcgtgga ctcaccgctg ggtgcttgcc aaanaggata gggacgcata tgctttttaa     180

agcaatctaa aaataataat aagtatagcg actatatacc tacttttaaa atcaactgtt     240

ttgaatagag gcagagctta ttttatatta tccaaatgag agctactctg ttnacatttt     300

ctttaaacat tttaaacatn gnttttttna cttcttnctg ggtnggattt tttttaaagg     360

cntaattggg a                                                          371

<210>  25
<211>  498
<212>  DNA
<213>  HOMO SAPIENS

<220>
<221>  misc_feature
<222>  (17)..(17)
<223>  n is equal to any a, t, g, or c


<220>
<221>  misc_feature
<222>  (114)..(114)
<223>  n is equal to any a, t, g, or c


<220>
<221>  misc_feature
<222>  (143)..(143)
<223>  n is equal to any a, t, g, or c


<220>
<221>  misc_feature
<222>  (183)..(183)
<223>  n is equal to any a, t, g, or c


<220>
<221>  misc_feature
<222>  (209)..(209)
<223>  n is equal to any a, t, g, or c


<220>
<221>  misc_feature
<222>  (242)..(242)
<223>  n is equal to any a, t, g, or c


<220>
<221>  misc_feature
<222>  (245)..(245)
<223>  n is equal to any a, t, g, or c


<220>
<221>  misc_feature
<222>  (251)..(251)
<223>  n is equal to any a, t, g, or c


<220>
<221>  misc_feature
<222>  (270)..(270)
<223>  n is equal to any a, t, g, or c


<220>

<221>  misc_feature
<222>  (284)..(284)
<223>  n is equal to any a, t, g, or c


<220>
<221>  misc_feature
<222>  (321)..(321)
<223>  n is equal to any a, t, g, or c


<220>
<221>  misc_feature
<222>  (326)..(326)
<223>  n is equal to any a, t, g, or c


<220>
<221>  misc_feature
<222>  (334)..(334)
<223>  n is equal to any a, t, g, or c


<220>
<221>  misc_feature
<222>  (336)..(336)
<223>  n is equal to any a, t, g, or c


<220>
<221>  misc_feature
<222>  (348)..(348)
<223>  n is equal to any a, t, g, or c


<220>
<221>  misc_feature
<222>  (352)..(352)
<223>  n is equal to any a, t, g, or c


<220>
<221>  misc_feature
<222>  (364)..(364)
<223>  n is equal to any a, t, g, or c


<220>
<221>  misc_feature
<222>  (367)..(367)
<223>  n is equal to any a, t, g, or c


<220>
<221>  misc_feature
<222>  (374)..(374)
<223>  n is equal to any a, t, g, or c


<220>

<221>  misc_feature
<222>  (376)..(376)
<223>  n is equal to any a, t, g, or c

<220>
<221>  misc_feature
<222>  (397)..(397)
<223>  n is equal to any a, t, g, or c

<220>
<221>  misc_feature
<222>  (406)..(406)
<223>  n is equal to any a, t, g, or c

<220>
<221>  misc_feature
<222>  (410)..(410)
<223>  n is equal to any a, t, g, or c

<220>
<221>  misc_feature
<222>  (422)..(422)
<223>  n is equal to any a, t, g, or c

<220>
<221>  misc_feature
<222>  (428)..(428)
<223>  n is equal to any a, t, g, or c

<220>
<221>  misc_feature
<222>  (442)..(442)
<223>  n is equal to any a, t, g, or c

<220>
<221>  misc_feature
<222>  (449)..(449)
<223>  n is equal to any a, t, g, or c

<220>
<221>  misc_feature
<222>  (451)..(451)
<223>  n is equal to any a, t, g, or c

<220>
<221>  misc_feature
<222>  (462)..(462)
<223>  n is equal to any a, t, g, or c

<220>

<221>  misc_feature
<222>  (470)..(470)
<223>  n is equal to any a, t, g, or c


<220>
<221>  misc_feature
<222>  (473)..(473)
<223>  n is equal to any a, t, g, or c


<220>
<221>  misc_feature
<222>  (486)..(486)
<223>  n is equal to any a, t, g, or c


<220>
<221>  misc_feature
<222>  (492)..(492)
<223>  n is equal to any a, t, g, or c


<400>  25
aattcggcag agccagnccg gagaaggacg cagacagcat caactccagc atcgacaaac      60

agggcgccaa gctcctgctg ggccccaacg acgcgcccgc tggcccctga aggncggttc     120

ctgccccggg aggtctcccc ggncccgcat cccgaggcgc ccaagctgga gccgcctgga     180

ggnttcggtc ggcgactctg aagagagtnc accgagcaaa ccaagtgccg gagcaacagc     240

gncgnctttt ncatggagat tcgtaagcan ttttcatttg acanggggat ccctggtttg     300

tttttagtta caagcaagca nntggnttga agtngntggg gaaaggancc gnagggattc     360

tgtnttnggg gccntntgga gggttttgga aaatttnagg gggttnctgn gggtttttta     420

anattggntt tttttagggt tnaagggtnn nttaacttgg gngtttttcn aanngttggg     480

ggattntttt tnaagatt                                                   498


<210>  26
<211>  178
<212>  DNA
<213>  HOMO SAPIENS

<220>
<221>  misc_feature
<222>  (100)..(100)
<223>  n is equal to any a, t, g, or c


<220>
<221>  misc_feature
<222>  (110)..(110)
<223>  n is equal to any a, t, g, or c

<220>
<221>  misc_feature
<222>  (136)..(136)
<223>  n is equal to any a, t, g, or c


<220>
<221>  misc_feature
<222>  (143)..(143)
<223>  n is equal to any a, t, g, or c


<220>
<221>  misc_feature
<222>  (146)..(146)
<223>  n is equal to any a, t, g, or c


<220>
<221>  misc_feature
<222>  (150)..(150)
<223>  n is equal to any a, t, g, or c


<220>
<221>  misc_feature
<222>  (164)..(164)
<223>  n is equal to any a, t, g, or c


<400>  26
ccttcagcaa gcctctttta atgatttaaa aaagaacttc taacttttac aaataattgt      60

gttcagagac agactttcaa tctaaagaaa agatcaaggn cctagctctn gtggcagaat     120

gcagaaacag aagccnccag atnganctcn gcagatgcta acgnggccca ctttgtcc       178


<210>  27
<211>  264
<212>  DNA
<213>  HOMO SAPIENS

<220>
<221>  misc_feature
<222>  (23)..(23)
<223>  n is equal to any a, t, g, or c


<220>
<221>  misc_feature
<222>  (131)..(131)
<223>  n is equal to any a, t, g, or c


<220>
<221>  misc_feature
<222>  (188)..(188)
<223>  n is equal to any a, t, g, or c

<220>
<221> misc_feature
<222> (228)..(228)
<223> n is equal to any a, t, g, or c

<400> 27
ggcagagcca agctcctgct ggngccccaa cgacgcgccc gctggcccct aaggccggtt 60

cctgccccgg aaggtctccc cggcccgcat cccgaggcgc ccaagctgga gccgcctgga 120

gggcttcggt ncggcgaacc tctgaaagag aagtgccacc gagcaaacca agtgccggta 180

gcaccagngc cgcctttcca tggagactcg taagcagctt catctganac gggaatccct 240

ggtttgcttt tagctacaag caag 264

<210> 28
<211> 1067
<212> DNA
<213> HOMO SAPIENS

<220>
<221> CDS
<222> (34)..(624)

<400> 28
gctccaagcc cagcctgccc cgctgccgcc acc atg acg ctc ctc ccc ggc ctc 54
Met Thr Leu Leu Pro Gly Leu
1               5

ctg ttt ctg acc tgg ctg cac aca tgc ctg gcc cac cat gac ccc tcc 102
Leu Phe Leu Thr Trp Leu His Thr Cys Leu Ala His His Asp Pro Ser
        10                  15                  20

ctc agg ggg cac ccc cac agt cac ggt acc cca cac tgc tac tcg gct 150
Leu Arg Gly His Pro His Ser His Gly Thr Pro His Cys Tyr Ser Ala
    25                  30                  35

gag gaa ctg ccc ctc ggc cag gcc ccc cca cac ctg ctg gct cga ggt 198
Glu Glu Leu Pro Leu Gly Gln Ala Pro Pro His Leu Leu Ala Arg Gly
40                  45                  50                  55

gcc aag tgg ggg cag gct ttg cct gta gcc ctg gtg tcc agc ctg gag 246
Ala Lys Trp Gly Gln Ala Leu Pro Val Ala Leu Val Ser Ser Leu Glu
                60                  65                  70

gca gca agc cac agg ggg agg cac gag agg ccc tca gct acg acc cag 294
Ala Ala Ser His Arg Gly Arg His Glu Arg Pro Ser Ala Thr Thr Gln
            75                  80                  85

tgc ccg gtg ctg cgg ccg gag gag gtg ttg gag gca gac acc cac cag 342
Cys Pro Val Leu Arg Pro Glu Glu Val Leu Glu Ala Asp Thr His Gln
        90                  95                  100

cgc tcc atc tca ccc tgg aga tac cgg gtg gac acg gat gag gac cgc 390
Arg Ser Ile Ser Pro Trp Arg Tyr Arg Val Asp Thr Asp Glu Asp Arg
    105                 110                 115

tat cca cag aag ctg gcc ttc gcc gag tgc ctg tgc aga ggc tgt atc 438
Tyr Pro Gln Lys Leu Ala Phe Ala Glu Cys Leu Cys Arg Gly Cys Ile
120                 125                 130                 135

gat gca cgg acg ggc cgc gag aca gct gcg ctc aac tcc gtg cgg ctg 486
Asp Ala Arg Thr Gly Arg Glu Thr Ala Ala Leu Asn Ser Val Arg Leu
                140                 145                 150

ctc cag agc ctg ctg gtg ctg cgc cgc cgg ccc tgc tcc cgc gac ggc 534
Leu Gln Ser Leu Leu Val Leu Arg Arg Arg Pro Cys Ser Arg Asp Gly
            155                 160                 165

tcg ggg ctc ccc aca cct ggg gcc ttt gcc ttc cac acc gag ttc atc 582
Ser Gly Leu Pro Thr Pro Gly Ala Phe Ala Phe His Thr Glu Phe Ile
        170                 175                 180

cac gtc ccc gtc ggc tgc acc tgc gtg ctg ccc cgt tca gtg 624
His Val Pro Val Gly Cys Thr Cys Val Leu Pro Arg Ser Val
    185                 190                 195

tgaccgccaa ggccgtgggg cccttagact ggacacgtgt gctccccaga gggcaccccc 684

tatttatgtg tatttattgt tatttatatg cctcccccaa cactaccctt ggggtctggg 744

cattccccgt gtctggagga cagcccccca ctgttctcct catctccagc ctcagtagtt 804

gggggtwgaa ggagctcagc acctcttcca gcccttaaag ctgcagaaaa ggtgtcacac 864

ggctgcctgt accttggytc cctgtcctgc tcccggcttc ccttacccta tcactggcct 924

caggcccccg caggctgcct cttcccaacc tccttggaag tacccctgtt tcttaaacaa 984

ttatttaagt gtacgtgtat tattaaactg atgaacacaa aaaaaaaaaa aaaaaaaaaa 1044

aaaaaaaaaa aaaaaaaaaa aaa 1067

<210> 29
<211> 197
<212> PRT
<213> HOMO SAPIENS

<400> 29

Met Thr Leu Leu Pro Gly Leu Leu Phe Leu Thr Trp Leu His Thr Cys
1               5                   10                  15

Leu Ala His His Asp Pro Ser Leu Arg Gly His Pro His Ser His Gly
            20                  25                  30

Thr Pro His Cys Tyr Ser Ala Glu Glu Leu Pro Leu Gly Gln Ala Pro
        35                  40                  45

Pro His Leu Leu Ala Arg Gly Ala Lys Trp Gly Gln Ala Leu Pro Val
    50                  55                  60

Ala Leu Val Ser Ser Leu Glu Ala Ala Ser His Arg Gly Arg His Glu
65                  70                  75                  80

Arg Pro Ser Ala Thr Thr Gln Cys Pro Val Leu Arg Pro Glu Glu Val
                85                  90                  95

Leu Glu Ala Asp Thr His Gln Arg Ser Ile Ser Pro Trp Arg Tyr Arg
            100                 105                 110

Val Asp Thr Asp Glu Asp Arg Tyr Pro Gln Lys Leu Ala Phe Ala Glu
        115                 120                 125

Cys Leu Cys Arg Gly Cys Ile Asp Ala Arg Thr Gly Arg Glu Thr Ala
    130                 135                 140

Ala Leu Asn Ser Val Arg Leu Leu Gln Ser Leu Leu Val Leu Arg Arg
145                 150                 155                 160

Arg Pro Cys Ser Arg Asp Gly Ser Gly Leu Pro Thr Pro Gly Ala Phe
                165                 170                 175

Ala Phe His Thr Glu Phe Ile His Val Pro Val Gly Cys Thr Cys Val
            180                 185                 190

Leu Pro Arg Ser Val
        195

<210>  30
<211>  332
<212>  DNA
<213>  HOMO SAPIENS

<220>
<221>  misc_feature
<222>  (162)..(162)
<223>  n is equal to any a, t, g, or c

<220>
<221>  misc_feature
<222>  (194)..(194)
<223>  n is equal to any a, t, g, or c

<220>
<221>  misc_feature
<222>  (214)..(214)
<223>  n is equal to any a, t, g, or c

<220>
<221>  misc_feature
<222>  (260)..(260)
<223>  n is equal to any a, t, g, or c


<220>
<221>  misc_feature
<222>  (277)..(277)
<223>  n is equal to any a, t, g, or c


<220>
<221>  misc_feature
<222>  (290)..(290)
<223>  n is equal to any a, t, g, or c


<220>
<221>  misc_feature
<222>  (305)..(305)
<223>  n is equal to any a, t, g, or c


<220>
<221>  misc_feature
<222>  (314)..(314)
<223>  n is equal to any a, t, g, or c


<400>  30
tggacacgta tgaggaccgc tatccacaga agctggcctt cgccgagtgc ctgtgcagag      60

gctgtatcga tgcacggacg ggccgcgaga cagctgcgct caactccgtg cggctgctcc     120

agagcctgac tggtgctgcg ccgccggccc tgactacccg cnacggacta cgggggctac     180

cccacacctg gggncctttg accttccaca ccgnagttac atgccacgta ccccgttcgg     240

gctgtcacct gacgtgctgn ccccgtttac agtgtgnacc gaccgtaggn ccgtggggtc     300

ccctnagtac tggnacacgt gtgatacccc ag                                   332


<210>  31
<211>  522
<212>  DNA
<213>  HOMO SAPIENS

<220>
<221>  CDS
<222>  (1)..(522)

<400>  31
ggc tgc gcg gac cgg ccg gag gag cta ctg gag cag ctg tac ggg cgc       48
Gly Cys Ala Asp Arg Pro Glu Glu Leu Leu Glu Gln Leu Tyr Gly Arg
1               5                   10                  15

ctg gcg gcc ggc gtg ctc agt gcc ttc cac cac acg ctg cag ctg ggg       96
Leu Ala Ala Gly Val Leu Ser Ala Phe His His Thr Leu Gln Leu Gly

20                  25                  30

ccg cgt gag cag gcg cgc aac gcg agc tgc ccg gca ggg ggc agg ccc        144
Pro Arg Glu Gln Ala Arg Asn Ala Ser Cys Pro Ala Gly Gly Arg Pro
        35                  40                  45

gcc gac cgc cgc ttc cgg ccg ccc acc aac ctg cgc agc gtg tcg ccc        192
Ala Asp Arg Arg Phe Arg Pro Pro Thr Asn Leu Arg Ser Val Ser Pro
    50                  55                  60

tgg gcc tac aga atc tcc tac gac ccg gcg agg tac ccc agg tac ctg        240
Trp Ala Tyr Arg Ile Ser Tyr Asp Pro Ala Arg Tyr Pro Arg Tyr Leu
65                  70                  75                  80

cct gaa gcc tac tgc ctg tgc cgg ggc tgc ctg acc ggg ctg ttc ggc        288
Pro Glu Ala Tyr Cys Leu Cys Arg Gly Cys Leu Thr Gly Leu Phe Gly
                85                  90                  95

gag gag gac gtg cgc ttc cgc agc gcc cct gtc tac atg ccc acc gtc        336
Glu Glu Asp Val Arg Phe Arg Ser Ala Pro Val Tyr Met Pro Thr Val
            100                 105                 110

gtc ctg cgc cgc acc ccc gcc tgc gcc ggc ggc cgt tcc gtc tac acc        384
Val Leu Arg Arg Thr Pro Ala Cys Ala Gly Gly Arg Ser Val Tyr Thr
        115                 120                 125

gag gcc tac gtc acc atc ccc gtg ggc tgc acc tgc gtc ccc gag ccg        432
Glu Ala Tyr Val Thr Ile Pro Val Gly Cys Thr Cys Val Pro Glu Pro
    130                 135                 140

gag aag gac gca gac agc atc aac tcc agc atc gac aaa cag ggc gcc        480
Glu Lys Asp Ala Asp Ser Ile Asn Ser Ser Ile Asp Lys Gln Gly Ala
145                 150                 155                 160

aag ctc ctg ctg ggc ccc aac gac gcg ccc gct ggc ccc tga                522
Lys Leu Leu Leu Gly Pro Asn Asp Ala Pro Ala Gly Pro
                165                 170

<210>  32
<211>  173
<212>  PRT
<213>  HOMO SAPIENS

<400>  32

Gly Cys Ala Asp Arg Pro Glu Glu Leu Leu Glu Gln Leu Tyr Gly Arg
1               5                   10                  15

Leu Ala Ala Gly Val Leu Ser Ala Phe His His Thr Leu Gln Leu Gly
            20                  25                  30

Pro Arg Glu Gln Ala Arg Asn Ala Ser Cys Pro Ala Gly Gly Arg Pro
        35                  40                  45

Ala Asp Arg Arg Phe Arg Pro Pro Thr Asn Leu Arg Ser Val Ser Pro
    50                  55                  60

Trp Ala Tyr Arg Ile Ser Tyr Asp Pro Ala Arg Tyr Pro Arg Tyr Leu
65                  70                  75                  80

Pro Glu Ala Tyr Cys Leu Cys Arg Gly Cys Leu Thr Gly Leu Phe Gly
                85                  90                  95

Glu Glu Asp Val Arg Phe Arg Ser Ala Pro Val Tyr Met Pro Thr Val
            100                 105                 110

Val Leu Arg Arg Thr Pro Ala Cys Ala Gly Gly Arg Ser Val Tyr Thr
        115                 120                 125

Glu Ala Tyr Val Thr Ile Pro Val Gly Cys Thr Cys Val Pro Glu Pro
    130                 135                 140

Glu Lys Asp Ala Asp Ser Ile Asn Ser Ser Ile Asp Lys Gln Gly Ala
145                 150                 155                 160

Lys Leu Leu Leu Gly Pro Asn Asp Ala Pro Ala Gly Pro
                165                 170

## Description

### Field of the Invention

The present invention relates to two novel human genes, each of which encodes a polypeptide which is a member of the Interleukin family. More specifically, the present invention relates to a polynucleotide encoding a novel human polypeptide named Interleukin-21, or "IL-21". The present invention also relates to a polynucleotide encoding a novel human polypeptide named Interleukin-22, or "IL-22". This invention also relates to IL-21 and IL-22 polypeptides, as well as vectors, host cells, antibodies directed to IL-21 and IL-22 polypeptides, and recombinant methods for producing the same. Also provided are diagnostic methods for detecting disorders related to the immune system, and therapeutic methods for treating such disorders. The invention further relates to screening methods for identifying agonists and antagonists of IL-21 and IL-22 activity.

### Background of the Invention

Cytokines typically exert their respective biochemical and physiological effects by binding to specific receptor molecules. Receptor binding then stimulates specific signal transduction pathways (Kishimoto, T., *et al.*, *Cell* **76:**253-262 (1994)). The specific interactions of cytokines with their receptors are often the primary regulators of a wide variety of cellular processes including activation, proliferation, and differentiation (Arai, K. -I, *et al.*, *Ann. Rev. Biochem*. **59**:783-836 (1990); Paul, W. E. and Seder, R. A., *Cell* **76:**241-251 (1994)).

Human interleukin (IL)-17, a closely related homolog of the molecules of the present invention, was only recently identified. IL-17 is a 155 amino acid polypeptide which was molecularly cloned from a CD4+ T-cell cDNA library (Yao, Z., *et al*., *J*. *Immunol*. **155:**5483-5486 (1995)). The IL-17 polypeptide contains an N-terminal signal peptide and contains approximately 72% identity at the amino acid level with a T-cell trophic herpesvirus saimiri (HVS) gene designated HVS13. High levels of IL-17 are secreted from CD4-positive primary peripheral blood leukocytes (PBL) upon stimulation (Yao, Z., *et al., Immunity* **3:**811-821 (1995)). Treatment of fibroblasts with IL-17, HVS13, or another murine homologue, designated CTLA8, activate signal transduction pathways and result in the stimulation of the NF-kappaB transcription factor family, the secretion of IL-6, and the costimulation of T-cell proliferation (Yao, Z., *et al., Immunity* **3:**811-821 (1995)).

An HVS 13-Fc fusion protein was used to isolate a murine IL-17 receptor molecule which does not appear to belong to any of the previously described cytokine receptor families (Yao, Z., *et al., Immunity* **3:**811-821 (1995)). The murine IL-17 receptor (mIL-17R) is predicted to encode a type I transmembrane protein of 864 amino acids with an apparent molecular mass of 97.8 kDa. mIL-17R is predicted to possess an N-terminal signal peptide with a cleavage site between alanine-31 and serine-32. The molecule also contains a 291 amino acid extracellular domain, a 21 amino acid transmembrane domain, and a 521 amino acid cytoplasmic tail. A soluble recombinant IL-17R molecule consisting of 323 amino acids of the extracellular domain of IL-17R fused to the Fc portion of human immunoglobulin IgG1 was able to significantly inhibit IL-17-induced IL-6 production by murine NIH-3T3 cells (*supra*).

Interestingly, the expression of the IL-17 gene is highly restricted. It is typically observed primarily in activated T-lymphocyte memory cells (Broxmeyer, H. *J. Exp. Med.* **183**:2411-2415 (1996); Fossiez, F., *et al., J. Exp. Med*. **183:**2593-2603 (1996)). Conversely, the IL-17 receptor appears to be expressed in a large number of cells and tissues (Rouvier, E., *et al., J. Immunol*. **150:**5445-5456 (1993); Yao, Z., *et al., J. Immunol*. **155:**5483-5486 (1995)). It remains to be seen, however, if IL-17 itself can play an autocrine role in the expression of IL-17. IL-17 has been implicated as a causative agent in the expression of IL-6, IL-8, G-CSF, Prostaglandin E (PGE₂), and intracellular adhesion molecule (ICAM)-1 (Fossiez, F., *supra*; Yao, Z., *et al., Immunity* **3:**811-821 (1995)). Each of these molecules possesses highly relevant and potentially therapeutically valuable properties. For instance, IL-6 is involved in the regulation of hematopoietic stem and progenitor cell growth and expansion (Ikebuchi, K., *et al., Proc. Natl. Acad. Sci. USA* **84**:9035-9039 (1987); Gentile, P. and Broxmeyer, H. E. *Ann. N.Y. Acad. Sci.* USA **628**:74-83 (1991)). IL-8 exhibits a myelosuppressive activity for stem cells and immature subsets of myeloid progenitors (Broxmeyer, H. E., *et al., Ann. Hematol.* **71**:235-246 (1995); Daly, T. J., *et al., J. Biol. Chem*. **270:**23282-23292 (1995)). G-CSF acts both early and late to activate and stimulate hematopoiesis in general, and more specifically on neutrophil hematopoiesis, while PGE₂ enhances erythropoiesis, suppresses lymphopoiesis and myelopoiesis in general, and strongly suppresses monocytopoiesis (Broxmeyer, H. E. *Amer. J. Ped. Hematol.*/*Oncol.* **14:**22-30 (1992); Broxmeyer, H. E. and Williams, D. E. *CRC Crit. Rev. Oncol.*/*Hematol.* **8:**173-226 (1988)).

Thus, there is a need for polypeptides that function as immunoregulatory molecules and, thereby, modulate the transfer of an extracellular signal ultimately to the nucleus of the cell, since disturbances of such regulation may be involved in disorders relating to cellular activation, hemostasis, angiogenesis, tumor metastasis, cellular migration and ovulation, as well as neurogenesis. Therefore, there is a need for identification and characterization of such human polypeptides which can play a role in detecting, preventing, ameliorating or correcting such disorders.

### Summary of the Invention

The present invention relates to novel polynucleotides and the encoded polypeptides of IL-21 and IL-22. Moreover, the present invention relates to vectors, host cells, antibodies, and recombinant methods for producing the polypeptides and polynucleotides. Also provided are diagnostic methods for detecting disorders related to the polypeptides, and therapeutic methods for treating such disorders. The invention further relates to screening methods for identifying binding partners of IL-21 and IL-22.

### Brief Description of the Drawings

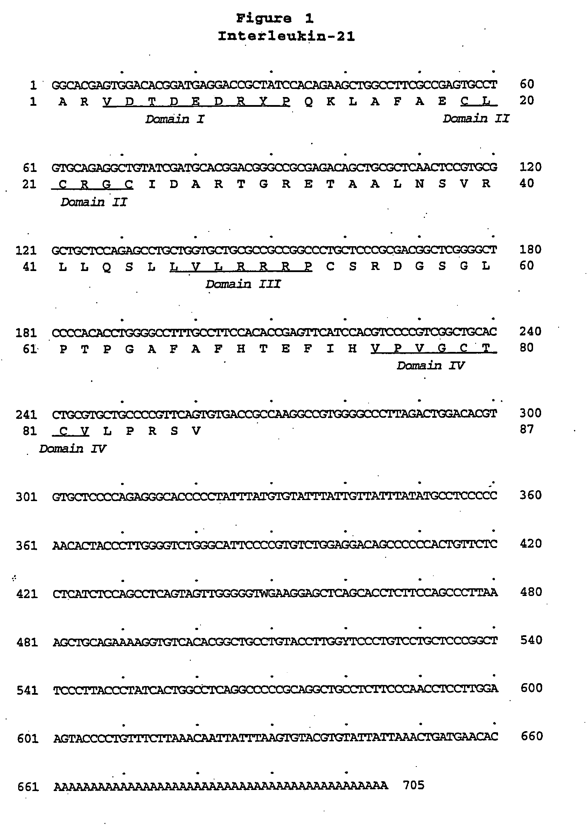

**Figure 1** shows the partial nucleotide sequence (SEQ ID NO:1) and the deduced amino acid sequence (SEQ ID NO:2) of IL-21. The locations of conserved Domains I-IV (see below) are underlined and labeled as such.

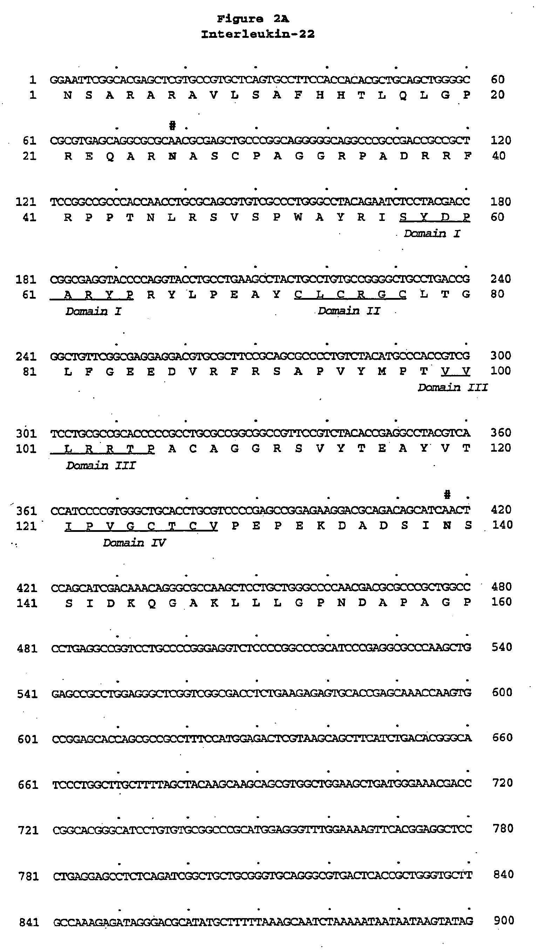

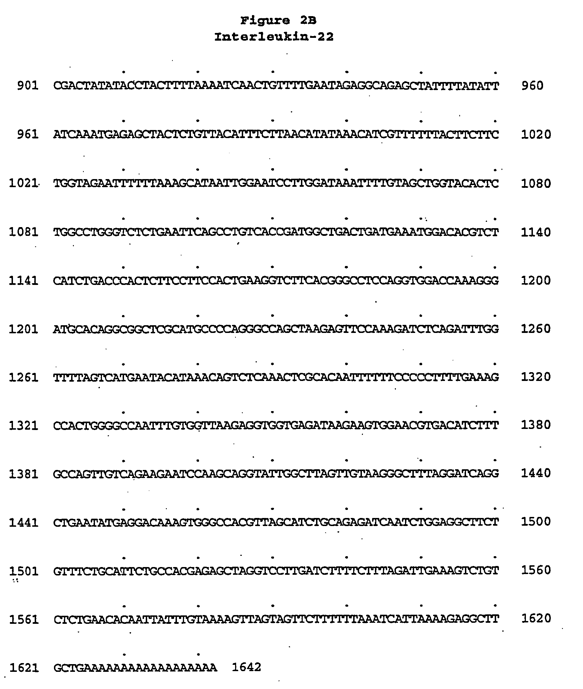

**Figures 2A and 2B** show the nucleotide sequence (SEQ ID NO:3) and the deduced amino acid sequence (SEQ ID NO:4) of IL-22. The locations of conserved Domains I-IV (see below) are underlined and labeled as such. The locations of two potential N-linked glycosylation sites are identified by a bolded asparagine symbol (N) accompanied by a bolded pound sign (#) located above the initial nucleotide of the codon encoding the corresponding asparagine.

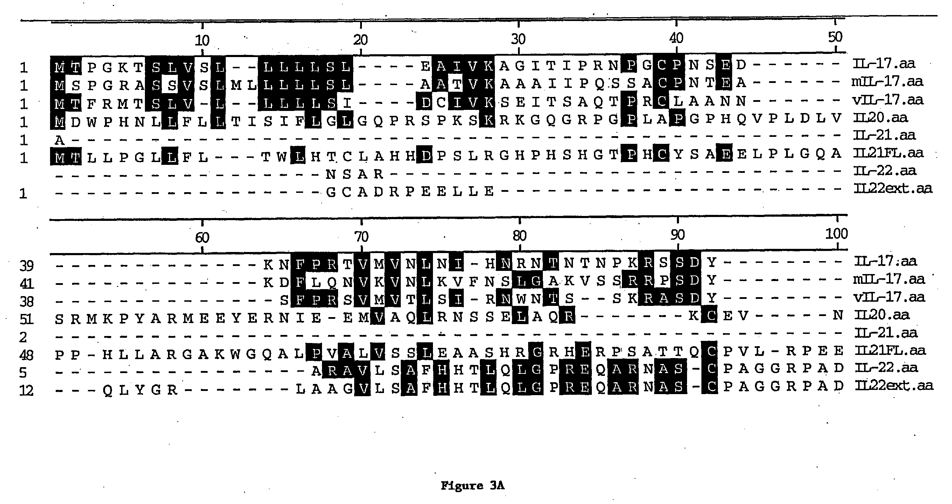

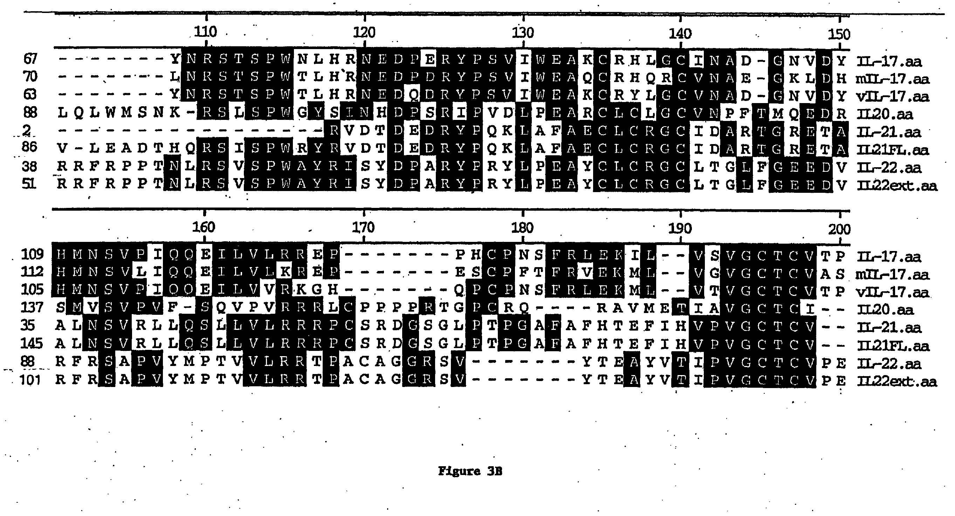

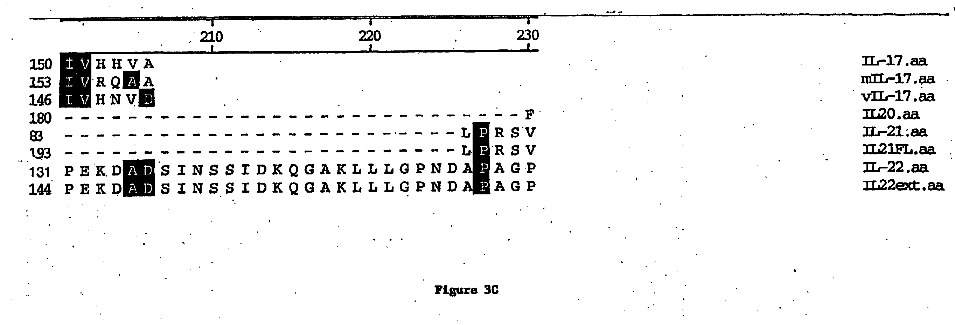

**Figures 3A, 3B, and 3C** show the regions of identity between the amino acid sequences of: (1) human Interleukin-17 (designated IL-17.aa in the figure; GenBank Accession No. U32659; SEQ ID NO:5); (2) mouse Interleukin-17 (designated mIL-17.aa in the figure; GenBank Accession No. U43088; SEQ ID NO:6); (3) viral Interleukin-17 (designated vIL-17.aa in the figure; GenBank Accession No. X64346; SEQ ID NO:7); (4) IL-20 (designated IL20.aa in the figure and disclosed in copending U.S. Provisional Application Serial No. 60/060,140; filed September 26, 1997; SEQ ID NO:8); (5) a partial-length IL-21 protein (SEQ ID NO:2); (6) the full-length IL-21 protein (designated IL-21FL.aa in the figure); (7) a partial-length IL-22 protein (designated IL-22.aa in the figure), and (8) an IL-22 protein (designated IL22ext.aa in the figure), as determined by aligning the sequences using the MegAlign component of the computer program DNA*Star (DNASTAR, Inc., 1228 S. Park St., Madison, WI 53715 USA) using the default parameters.

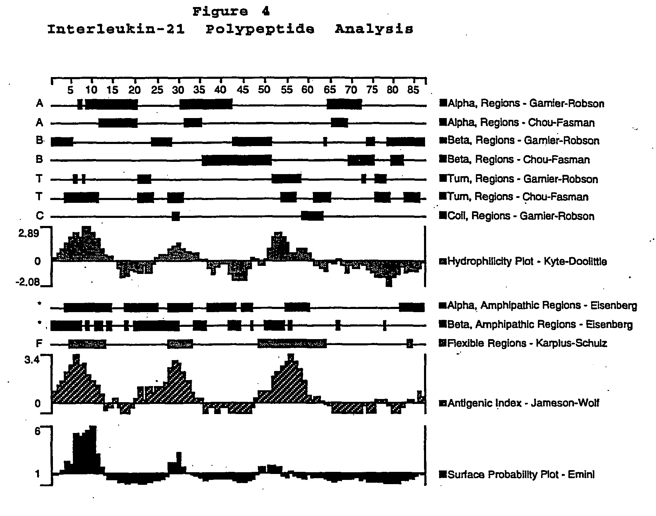

**Figure 4** shows an analysis of the partial IL-21 amino acid sequence (SEQ ID NO:2). Alpha, beta, turn and coil regions; hydrophilicity and hydrophobicity; amphipathic regions; flexible regions; antigenic index and surface probability are shown. In the "Antigenic Index" or "Jameson-Wolf" graph, the positive peaks indicate locations of the highly antigenic regions of the IL-21 protein, that is, regions from which epitope-bearing peptides of the invention can be determined. Polypeptides and polynucleotides encoding polypeptides comprising the domains defined by these graphs are contemplated by the present invention.

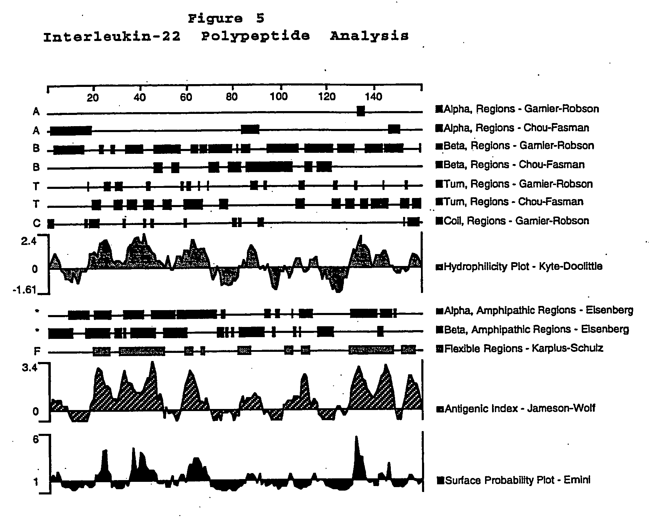

**Figure 5** shows an analysis of the IL-22 amino acid sequence. Alpha, beta, turn and coil regions; hydrophilicity and hydrophobicity; amphipathic regions; flexible regions; antigenic index and surface probability are shown. In the "Antigenic Index" or "Jameson-Wolf" graph, the positive peaks indicate locations of the highly antigenic regions of the IL-22 protein, that is, regions from which epitope-bearing peptides of the invention can be determined. Polypeptides and polynucleotides encoding polypeptides comprising the domains defined by these graphs are contemplated by the present invention.
   The data presented in Figure 5 are also represented in tabular form in Table II. The columns are labeled with the headings "Res", "Position", and Roman Numerals I-XIII. The column headings refer to the following features of the amino acid sequence presented in Figure 5 and Table II: "Res": amino acid residue of SEQ ID NO:4 or Figures 2A and 2B; "Position": position of the corresponding residue within SEQ ID NO:4 or Figures 2A and 2B; I: Alpha, Regions - Gamier-Robson; II: Alpha, Regions - Chou-Fasman; III: Beta, Regions - Garnier-Robson; IV: Beta, Regions - Chou-Fasman; V: Turn, Regions - Garnier-Robson; VI: Turn, Regions - Chou-Fasman; VII: Coil, Regions - Garnier-Robson; VIII: Hydrophilicity Plot - Kyte-Doolittle; IX: Alpha, Amphipathic Regions - Eisenberg; X: Beta, Amphipathic Regions - Eisenberg; XI: Flexible Regions - Karplus-Schulz; XII: Antigenic Index - Jameson-Wolf; and XIII: Surface Probability Plot - Emini.

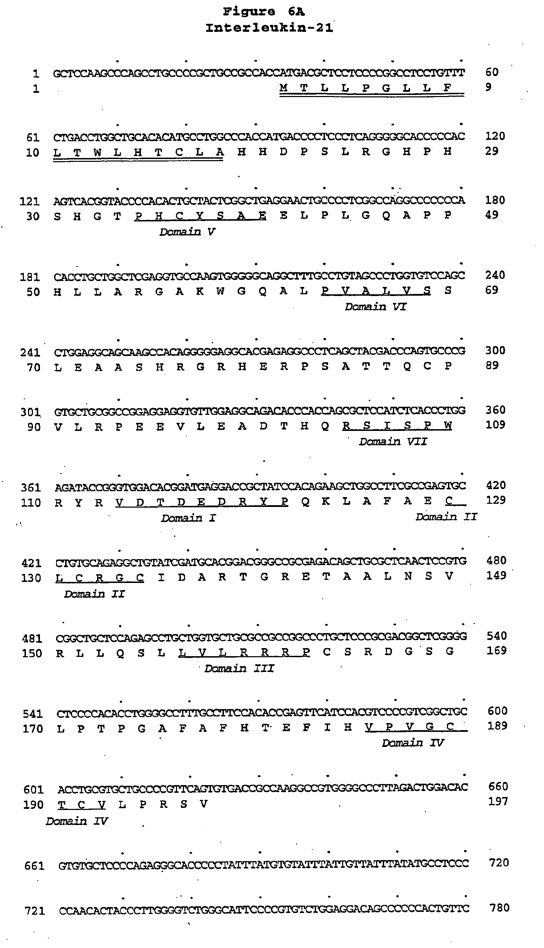

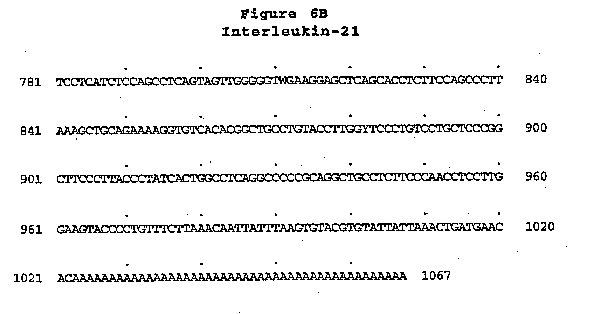

**Figures 6A and 6B** show the nucleotide sequence (SEQ ID NO:28) and the deduced amino acid sequence (SEQ ID NO:29) of the full-length IL-21. The locations of conserved Domains I-IV (identical to those shown in Figure 1) and of conserved Domains V-VII are underlined and labeled as such. A predicted signal peptide from methionine-1 to alanine-18 is double underlined.

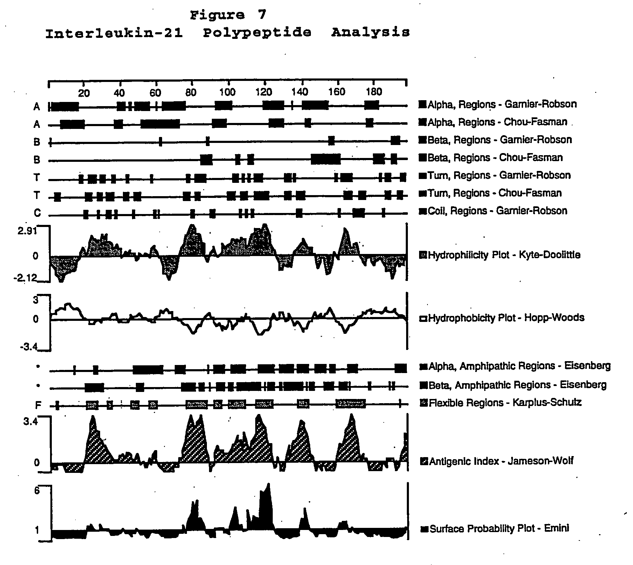

**Figure 7** shows an analysis of a full-length IL-21 amino acid sequence. Alpha, beta, turn and coil regions; hydrophilicity and hydrophobicity; amphipathic regions; flexible regions; antigenic index and surface probability are shown. In the "Antigenic Index" or "Jameson-Wolf" graph, the positive peaks indicate locations of the highly antigenic regions of a full-length IL-21 protein, that is, regions from which epitope-bearing peptides of the invention can be determined. Polypeptides and polynucleotides encoding polypeptides comprising the domains defined by these graphs are contemplated by the present invention.
   The data presented in Figure 7 are also represented in tabular form in Table I. The columns are labeled with the headings "Res", "Position", and Roman Numerals I-XIV. The column headings refer to the following features of the amino acid sequence presented in Figure 7 and Table I: "Res": amino acid residue of SEQ ID NO:29 or Figures 6A and 6B; "Position": position of the corresponding residue within SEQ ID NO:29 or Figures 6A and 6B; I: Alpha, Regions - Gamier-Robson; II: Alpha, Regions - Chou-Fasman; III: Beta, Regions - Gamier-Robson; IV: Beta, Regions - Chou-Fasman; V: Turn, Regions - Garnier-Robson; VI: Turn, Regions - Chou-Fasman; VII: Coil, Regions - Gamier-Robson; VIII: Hydrophilicity Plot - Kyte-Doolittle; IX: Hydrophobicity Plot - Hopp-Woods; X: Alpha, Amphipathic Regions - Eisenberg; XI: Beta, Amphipathic Regions - Eisenberg; XII: Flexible Regions - Karplus-Schulz; XIII: Antigenic Index - Jameson-Wolf; and XIV: Surface Probability Plot - Emini.

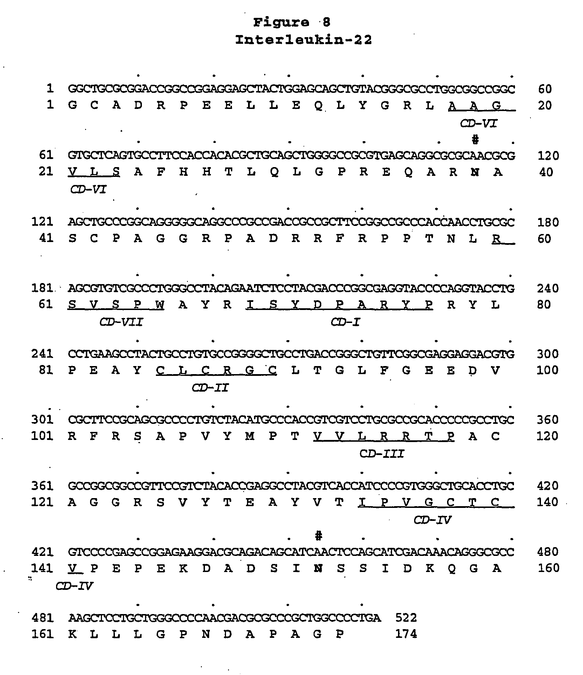

**Figure 8** shows the nucleotide sequence (SEQ ID NO:31) and the deduced amino acid sequence (SEQ ID NO:32) of an IL-22. The locations of conserved Domains I-IV and VI-VII are underlined and labeled as such. The locations of two potential N-linked glycosylation sites are identified by a bolded asparagine symbol (N) accompanied by a bolded pound sign (#) located above the initial nucleotide of the codon encoding the corresponding asparagine. The two potential N-linked glycosylation sites are located at Asn-39 (N-39, A-40, S-41) and Asn-152 (N-152, S-153, S-154) of SEQ ID NO:32.

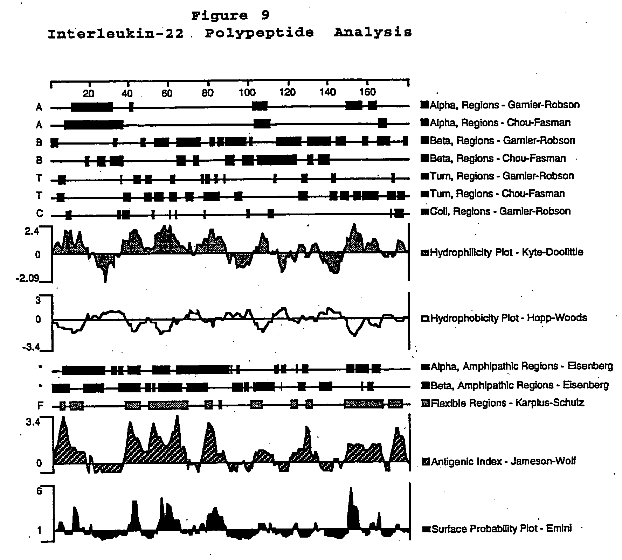

**Figure 9** shows an analysis of the IL-22 amino acid sequence provided in Figure 8 and SEQ ID NO:32. Alpha, beta, turn and coil regions; hydrophilicity and hydrophobicity; amphipathic regions; flexible regions; antigenic index and surface probability are shown. In the "Antigenic Index" or "Jameson-Wolf" graph, the positive peaks indicate locations of the highly antigenic regions of the IL-22 protein, that is, regions from which epitope-bearing peptides of the invention can be determined. Polypeptides and polynucleotides encoding polypeptides comprising the domains defined by these graphs are contemplated by the present invention.

The data presented in Figure 9 are also represented in tabular form in Table III. The columns are labeled with the headings "Res", "Position", and Roman Numerals I-XIV. The column headings refer to the following features of the amino acid sequence presented in Figure 9 and Table III: "Res": amino acid residue of SEQ ID NO:32 or Figure 8; "Position": position of the corresponding residue within SEQ ID NO:32 or Figure 8; I: Alpha, Regions - Gamier-Robson; II: Alpha, Regions - Chou-Fasman; III: Beta, Regions - Garnier-Robson; IV: Beta, Regions - Chou-Fasman; V: Turn, Regions - Gamier-Robson; VI: Turn, Regions - Chou-Fasman; VII: Coil, Regions - Garnier-Robson; VIII: Hydrophilicity Plot - Kyte-Doolittle; IX: Hydrophobicity Plot - Hopp-Woods; X: Alpha, Amphipathic Regions - Eisenberg; XI: Beta, Amphipathic Regions - Eisenberg; XII: Flexible Regions - Karplus-Schulz; XIII: Antigenic Index - Jameson-Wolf; and XIV: Surface Probability Plot - Emini.

### Detailed Description

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide. However, a nucleic acid contained in a clone that is a member of a library (e.g., a genomic or cDNA library) that has not been isolated from other members of the library (e.g., in the form of a homogeneous solution containing the clone and other members of the library) or which is contained on a chromosome preparation (e.g., a chromosome spread), is not "isolated" for the purposes of this invention.

In the present invention, a "secreted" IL-21 or IL-22 protein refers to a protein. capable of being directed to the ER, secretory vesicles, or the extracellular space as a result of a signal sequence, as well as an IL-21 or IL-22 protein released into the extracellular space without necessarily containing a signal sequence. If the IL-21 or IL-22 secreted protein is released into the extracellular space, the IL-21 or IL-22 secreted protein can undergo extracellular processing to produce a "mature" IL-21 or IL-22 protein. Release into the extracellular space can occur by many mechanisms, including exocytosis and proteolytic cleavage.

As used herein , an IL-21 or IL-22 "polynucleotide" refers to a molecule having a nucleic acid sequence contained in SEQ ID NO:1 or in SEQ ID NO:3, respectively, or the cDNA contained within the respective clones deposited with the ATCC. For example, the IL-21 or IL-22 polynucleotide can contain the nucleotide sequence of the full-length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, an IL-21 or IL-22 "polypeptide" refers to a molecule having the translated amino acid sequence generated from the polynucleotide as broadly defined.

As used herein , an IL-21 "polynucleotide" refers to a molecule having a nucleic acid sequence contained in SEQ ID NO:1 or in SEQ ID NO:28, or the cDNA contained within the respective clones deposited with the ATCC. For example, the IL-21 polynucleotide can contain the nucleotide sequence of the full-length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, an IL-21 "polypeptide" refers to a molecule having the translated amino acid sequence generated from the polynucleotide as broadly defined.

As used herein , an IL-22 "polynucleotide" refers to a molecule having a nucleic acid sequence contained in SEQ ID NO:3 or in SEQ ID NO:31, or the cDNA contained within the respective clones deposited with the ATCC. For example, the IL-22 polynucleotide can contain the nucleotide sequence of the full-length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, an IL-22 "polypeptide" refers to a molecule having the translated amino acid sequence generated from the polynucleotide as broadly defined.

A representative clone containing all or most of the sequence for SEQ ID NO:1 (designated HTGED19) was deposited with the American Type Culture Collection ("ATCC") on March 5, 1998, and was given the ATCC Deposit Number 209666. In addition, a representative clone containing all or most of the sequence for SEQ ID NO:3 (designated HFPBX96) was also deposited with the ATCC on March 5, 1998, and was given the ATCC Deposit Number 209665. The ATCC is located at 10801 University Blvd., Manassas, VA 20110-2209, USA. The ATCC deposit was made pursuant to the terms of the Budapest Treaty on the international recognition of the deposit of microorganisms for purposes of patent procedure.

An IL-21 "polynucleotide" also includes those polynucleotides capable of hybridizing, under stringent hybridization conditions, to sequences contained in SEQ ID NO:1 or SEQ ID NO:28, the complements thereof, or the cDNA within the deposited clone. Further, an IL-22 "polynucleotide" also includes those polynucleotides capable of hybridizing, under stringent hybridization conditions, to sequences contained in SEQ ID NO:3 or SEQ ID NO:31, the complements thereof, or the cDNA within the deposited clone. "Stringent hybridization conditions" refers to an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

Also contemplated are nucleic acid molecules that hybridize to the IL-21 and the IL-22 polynucleotides at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 µg/ml salmon sperm blocking DNA; followed by washes at 50°C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a polyA+ stretch or the complement thereof (e.g., practically any double-stranded cDNA clone).

The IL-21 and IL-22 polynucleotides can be composed of any polyribonucleotide or polydeoxyribonucleotide, which may be unmodified RNA or DNA or modified RNA or DNA. For example, the IL-21 and IL-22 polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the IL-21 polynucleotides can be composed of triple-stranded regions comprising RNA or DNA or both RNA and DNA. IL-21 polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

IL-21 and IL-22 polypeptides can be composed of amino acids joined to each other by peptide bonds or modified peptide bonds, i.e., peptide isosteres, and may contain amino acids other than the 20 gene-encoded amino acids. The IL-21 and IL-22 polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in the IL-21 and IL-22 polypeptides, including the peptide backbone, the amino acid side-chains and the amino or carboxyl termini. It will be appreciated that the same type of modification may be present in the same or varying degrees at several sites in a given IL-21 or IL-22 polypeptide. Also, a given IL-21 or IL-22 polypeptide may contain many types of modifications. IL-21 or IL-22 polypeptides may be branched, for example, as a result of ubiquitination, and they may be cyclic, with or without branching. Cyclic, branched, and branched cyclic IL-21 and IL-22 polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pp. 1-12 (1983); Seifter, *et al., Meth. Enzymol*. **182:**626-646 (1990); Rattan, *et al., Ann. NY Acad. Sci.* **663:**48-62 (1992)).

"SEQ ID NO:1" and "SEQ ID NO:28" refer to an IL-21 polynucleotide sequence while "SEQ ID NO:2" and SEQ ID NO:29 refer to an IL-21 polypeptide sequence. Likewise, "SEQ ID NO:3" and SEQ ID NO:31 refer to an IL-22 polynucleotide sequence while "SEQ ID NO:4" and SEQ ID NO:32 refer to an IL-22 polypeptide sequence.

An IL-21 polypeptide "having biological activity" refers to polypeptides exhibiting activity similar, but not necessarily identical to, an activity of an IL-21 polypeptide, including mature forms, as measured in a particular biological assay, with or without dose-dependency. In addition, an IL-22 polypeptide "having biological activity" refers to polypeptides exhibiting activity similar, but not necessarily identical to, an activity of an IL-22 polypeptide, including mature forms, as measured in a particular biological assay, with or without dose-dependency. In the case where dose-dependency does exist, it need not be identical to that of the IL-21 or IL-22 polypeptide, but rather substantially similar to the dose-dependence in a given activity as compared to the IL-21 or IL-22 polypeptides (i.e., the candidate polypeptide will exhibit greater activity or not more than about 25-fold less and, preferably, not more than about tenfold less activity, and most preferably, not more than about three-fold less activity relative to the IL-21 polypeptide).

### IL-21 and IL-22 Polynucleotides and Polypeptides

Clone HTGED 19, encoding IL-21, was isolated from a cDNA library derived from apoptotic T-cells. This clone contains the entire coding region identified as SEQ ID NO:2. The deposited clone contains a cDNA having a total of 705 nucleotides, which encodes a partial predicted open reading frame of 87 amino acid residues (see Figure 1). The partial open reading frame begins at a point in the complete IL-21 ORF such that the "G" in position 1 of SEQ ID NO:1 is actually in position 3 of a coding triplet. As such, the partial predicted IL-21 polypeptide sequence is shown beginning in-frame with an alanine residue at position 1 of SEQ ID NO:2. The alanine residue at position 1 of SEQ ID NO:2 is encoded by nucleotides 2-4 of the nucleotide sequence shown as SEQ ID NO:1. The ORF shown as SEQ ID NO:2 ends at a stop codon at nucleotide position 263-265 of the nucleotide sequence shown as SEQ ID NO:1. The predicted molecular weight of the partial IL-21 protein should be about 9,558 Daltons.

An initial BLAST analysis of the expression of the IL-21 cDNA sequence against the HGS EST database has also revealed a highly specific expression of this cDNA clone. In such an analysis, the HTGED 19 cDNA sequence appears to be found only in apoptotic T-cells. Thus, IL-21 appears to be expressed in a highly restricted pattern limited to apoptotic T-cells, and, for example, other subpopulatons of lymphocytes or other cells in a state of activation or quiescence.

Clone HTGED19, encoding IL-21, was used to screen a panel of bacterial artificial chromosomes containing various segments of human genomic DNA (Research Genetics, Inc.). A positive clone was sequenced to identify potential splice donor and acceptor sites. Analysis of several sites revealed an upstream partial ORF that, when placed immediately 5' and in frame with the existing IL-21 DNA sequence, generated a complete ORF which encodes a polypeptide with additional sequence identity to the IL-17 family (See Figures 3A, 3B, and 3C). A clone of the full-length IL-21 ORF has been constructed by combination of the IL-21 exons PCR-amplified from the HTGED 19 genomic clone. The clone has been deposited with the ATCC as ATCC Deposit No. PTA-69 on May 14, 1999. The nucleotide sequence of the full-length IL-21 clone contains the entire coding region identified as SEQ ID NO:29. The resultant clone contains an insert having a total of 1067 nucleotides, which encodes a predicted open reading frame of 197 amino acid residues (see Figures 6A and 6B). The open reading frame begins at nucleotide position 34 in the complete IL-21 polynucleotide shown as SEQ ID NO:28 (Figures 6A and 6B). The ORF ends at a stop codon at nucleotide position 625-627 of the nucleotide sequence shown as SEQ ID NO:28 (Figures 6A and 6B). The predicted molecular weight of the IL-21 polypeptide shown in Figures 6A and 6B and as SEQ ID NO:29 should be about 21,764 Daltons.

Further BLAST analysis of the expression of the full-length IL-21 cDNA sequence against the HGS EST database has also revealed a highly specific expression of this cDNA clone. In such an analysis, the full-length HTGED19 cDNA sequence appears to be found only in apoptotic T-cells. Thus, IL-21 appears to be expressed in a highly restricted pattern limited to apoptotic T-cells, and, for example, other subpopulatons of lymphocytes or other cells in a state of activation or quiescence.

A PCR product comprising exons 1 and 2 (based on the genomic organization predicted above) has been amplified using a 12 week old early stage cDNA library as template DNA. This PCR product confirms that at least exons 1 and 2 of the genomic organization predicted above exists as messenger RNA in at least 12 week old early stage human embyo.

Clone HFPBX96, encoding IL-22, was isolated from a cDNA library derived from epileptic frontal cortex. This clone contains the entire coding region identified as SEQ ID NO:4. The deposited clone contains a cDNA having a total of 1,642 nucleotides, which encodes a partial predicted open reading frame of 160 amino acid residues (see Figures 2A and 2B). The partial open reading frame begins at a point in the complete IL-22 ORF such that the "G" in position 1 of SEQ ID NO:3 is actually in position two of a coding triplet. As such, the partial predicted IL-22 polypeptide sequence is shown beginning in-frame with an asparagine residue at position 1 of SEQ ID NO:4. The asparagine residue at position 1 of SEQ ID NO:4 is encoded by nucleotides 3-5 of the nucleotide sequence shown as SEQ ID NO:3. The ORF shown as SEQ ID NO:4 ends at a stop codon at nucleotide position 483-485 of the nucleotide sequence shown as SEQ ID NO:3. The predicted molecular weight of the partial IL-22 protein should be about 17,436 Daltons.

Clone HFPBX96, encoding IL-22, was used to screen a human fetal brain cDNA library containing approximately one million cDNA clones (Genome Systems, Inc.). A positive clone was sequenced to identify 59 nucleotides of additional 5' sequence. The cDNA clone has been deposited with the ATCC as ATCC Deposit No. PTA-70 on May 14, 1999. Analysis of the extended IL-22 ORF reveals a polypeptide with additional sequence identity to the IL-17 family (see Figures 3A, 3B, and 3C). The nucleotide sequence of the extended, but still apparently partial-length IL-22 clone contains the entire coding region identified as SEQ ID NO:31. The resultant clone contains an insert having a total of 522 nucleotides, which encodes a predicted open reading frame of 174 amino acid residues (see Figure 8). The open reading frame begins at nucleotide position 1 in the complete IL-22 polynucleotide shown as SEQ ID NO:31 (Figure 8). The ORF ends at a stop codon at nucleotide position 520-522 of the nucleotide sequence shown as SEQ ID NO:31 (Figure 8). The predicted molecular weight of the IL-22 polypeptide shown in Figure 8 and as SEQ ID NO:31 is about 19,636 Daltons.

Using BLAST and MegAlign analyses, SEQ ID NO:2, SEQ ID NO:4, SEQ ID NO:29, and SEQ ID NO:32 were each found to be highly homologous to several members of the Interleukin family. Particularly, SEQ ID NO:2, SEQ ID NO:4, SEQ ID NO:29, and SEQ ID NO:32 contain at least four domains homologous to the translation products of the human mRNA for Interleukin (IL)-20 (copending U.S. Provisional Application Serial No. 60/060,140; filed September 26, 1997; SEQ ID NO:8), IL-17 (GenBank Accession No. U32659; SEQ ID NO:5; see also Figures 3A, 3B, and 3C), the murine mRNA for Interleukin (IL)-17 (GenBank Accession No. U43088; SEQ ID NO:6; see also Figures 3A, 3B, and 3C), and the human viral mRNA for Interleukin (IL)-17 (GenBank Accession No. X64346; SEQ ID NO:7; see also Figures 3A, 3B, and 3C).

Specifically, the molecules of the present invention, in particular, SEQ ID NO:2, SEQ ID NO:4, SEQ ID NO:29, and SEQ ID NO:32 share a high degree of sequence identity with IL-20, IL-17, mIL-17, and vIL-17 in the following conserved domains: (a) a predicted NXDPXRYP domain (where X represents any amino acid) located at about amino acids valine-3 to proline-11 of SEQ ID NO:2, serine-57 to proline-64 of SEQ ID NO:4, valine-113 to proline-121 of SEQ ID NO:29, serine-70 to proline-77 of SEQ ID NO:32, and asparagine-79 to proline-86 of the human IL-17 amino acid sequence (SEQ ID NO:5); (b) a predicted CLCXGC domain (where X represents any amino acid) located at about amino acids cysteine-19 to cysteine-24 of SEQ ID NO:2, cysteine-72 to cysteine-77 of SEQ ID NO:4, cysteine-129 to cysteine-134 of SEQ ID NO:29, cysteine-85 to cysteine-90 of SEQ ID NO:32, and cysteine-94 to cysteine-99 of the human IL-17 amino acid sequence (SEQ ID NO:5); (c) a predicted LVLRRXP domain (where X represents any amino acid) located at about amino acids leucine-46 to proline-52 of SEQ ID NO:2, valine-99 to proline-105 of SEQ ID NO:4, leucine-156 to proline-162 of SEQ ID NO:29, valine-112 to proline-118 of SEQ ID NO:32, and leucine-120 to proline-126 of the human IL-17 amino acid sequence (SEQ ID NO:5); and (d) a predicted VXVGCTCV domain (where X represents any amino acid) located. at about amino acids valine-75 to valine-82 of SEQ ID NO:2, isoleucine-121 to valine-128 of SEQ ID NO:4, valine-187 to valine-192 of SEQ ID NO:29, isoleucine-134 to valine-141 of SEQ ID NO:32, and valine-140 to valine-147 of the human IL-17 amino acid sequence (SEQ ID NO:5).

In addition, the full-length IL-21 molecule shown in Figures 6A and 6B (SEQ ID NO:29) and the IL-22 molecule shown in Figure 8 (SEQ ID NO:32) exhibit several additional conserved domains when compared with IL-20 and the other members of the IL-17 family as shown in Figures 3A, 3B, and 3C). These conserved Domains are underlined in Figures 6A and 6B and in Figure 8 and are labeled as conserved Domains V, VI, and VII. Specifically, the molecules of the present invention, in particular, SEQ ID NO:29 and SEQ ID NO:32, share a high degree of sequence identity with IL-20, IL-17, mIL-17, and vIL-17 in the following conserved domains: (a) a predicted PXCXSAE domain (where X represents any amino acid) located at about amino acids proline-34 to glutamic acid-40 of SEQ ID NO:29; (b) a predicted PXXLVS domain (where X represents any amino acid) located at about amino acids proline-63 to serine-68 of SEQ ID NO:29 and at about amino acids alanine-18 to serine-23 of SEQ ID NO:32; and (c) a predicted RSXSPW domain (where X represents any amino acid) located at about amino acids arginine-104 to tryptophan-109 of SEQ ID NO:29 and at about amino acids arginine-60 to tryptophan-65 of SEQ ID NO:32. These polypeptide fragments of IL-21 and IL-22 are specifically contemplated in the present invention. Because each of these IL-17 and IL-17-like molecules is thought to be important immunoregulatory molecules, the homology between these IL-17 and IL-17-like molecules and IL-21 and IL-22 suggests that IL-21 and IL-22 may also be important immunoregulatory molecules.

Moreover, based on their apparent sequence identities with IL-17 and IL-20 (see Figures 3A, 3B, and 3C), the full-length IL-21 and IL-22 polypeptides are each likely to have an amino terminal secretory signal peptide leader sequence. Since the present invention appears to be partial cDNA clones of the IL-21 (SEQ ID NOs:1 and 2) and IL-22 (SEQ ID NOs:3 and 4) molecules (in addition to the full-length IL-21 molecule shown as SEQ ID NOs:28 and 29 and the IL-22 molecule shown as SEQ ID NOs:31 and 32), it is also contemplated that the translation products of SEQ ID NOs:2, 4, and 32 of the present invention will be caused to enter the cellular secretory pathway by virtue of being expressed as a fusion proteins comprising several different portions of the N-terminus of the IL-20 molecule of copending U.S. Provisional Application Serial No. 60/060,140 fused to the known coding sequence of the IL-21 or IL-22 molecules of the present invention. Such expression constructs will secrete hybrid IL-20/IL-21 or IL-20/IL-22 molecules from the host cell.

In one embodiment, the mature IL-21 protein used in these fusion proteins encompasses about amino acids 12-87 of SEQ ID NO:2, while the IL-20/21 fusion protein encompasses about the 104 or 113 N-terminal amino acids of IL-20 encoded in frame with about amino acids 12-87 of the IL-21 of SEQ ID NO:2. In other embodiments, an IL-20/21 fusion protein encompasses about the 104 or 113 N-terminal amino acids of IL-20 encoded in frame with about amino acids 3-87 of the IL-21 protein of SEQ ID NO:2. These polypeptide fragments of IL-21 are specifically contemplated in the present invention.

In another embodiment, the mature IL-22 protein used to generate these fusion proteins encompasses about amino acids 1-160 of SEQ ID NO:4, while the IL-20/22 fusion protein encompasses about the 95, 104 or 113 N-terminal amino acids of IL-20 encoded in frame with about amino acids 1-160 of the IL-22 of SEQ ID NO:4. In other embodiments, the IL-22 protein used to generate these fusion proteins encompasses about amino acids 47-160 of SEQ ID NO:4, while the IL-20/22 fusion protein encompasses about the 95, 104 or 113 N-terminal amino acids of IL-20 encoded in frame with about amino acids 1-160 of the IL-22 of SEQ ID NO:4. In still other embodiments, the IL-22 protein used to generate these fusion proteins encompasses about amino acids 56-160 of SEQ ID NO:4, while the IL-20/22 fusion protein encompasses about the 95, 104 or 113 N-terminal amino acids of IL-20 encoded in frame with about amino acids 1-160 of the IL-22 of SEQ ID NO:4. In yet other embodiments, the IL-22 protein used to generate these fusion proteins encompasses about amino acids 65-160 of SEQ ID NO:4, while the IL-20/22 fusion protein encompasses about the 95, 104 or 113 N-terminal amino acids of IL-20 encoded in frame with about amino acids 1-160 of the IL-22 of SEQ ID NO:4. These polypeptide fragments of IL-22 are specifically contemplated in the present invention.

In yet another embodiment, the mature IL-22 protein used to generate these fusion proteins encompasses about amino acids 1-173 of SEQ ID NO:32, while the IL-20/22 fusion protein encompasses about the 95, 104 or 113 N-terminal amino acids of IL-20 encoded in frame with about amino acids 1-173 of the IL-22 of SEQ ID NO:32. These polypeptide fragments of IL-22 are specifically contemplated in the present invention.

The IL-21 and IL-22 nucleotide sequences identified as SEQ ID NO:1 and SEQ ID NO:3, respectively, were assembled from partially homologous ("overlapping") sequences obtained from the deposited clones. The IL-21 nucleotide sequence identified as SEQ ID NO:28 was assembled from partially homologous ("overlapping") sequences obtained from the deposited clone and a genomic DNA clone. The IL-22 nucleotide sequence identified as SEQ ID NO:32 was assembled from partially homologous ("overlapping") sequences obtained from the deposited clones (ATCC Deposit No. 209665 and ATCC Deposit No. PTA-70). The overlapping sequences specific to the partial IL-21 and IL-22 molecules of the invention and the full-length IL-21 molecule of the invention were each assembled into single contiguous sequences of high redundancy (usually three to five overlapping sequences at each nucleotide position), resulting in four final sequences identified as SEQ ID NO:1, SEQ ID NO:3, SEQ ID NO:28, and SEQ ID NO:31.

Therefore, SEQ ID NO:1 and the translated SEQ ID NO:2; SEQ ID NO:3 and the translated SEQ ID NO:4; SEQ ID NO:31 and the translated SEQ ID NO:32; and SEQ ID NO:28 and the translated SEQ ID NO:29, are sufficiently accurate and otherwise suitable for a variety of uses well known in the art and described further below. For instance, SEQ ID NO:1, SEQ ID NO:3, SEQ ID NO:28, and SEQ ID NO:31 are useful for designing nucleic acid hybridization probes that will detect nucleic acid sequences contained in SEQ ID NO:1, SEQ ID NO:3, SEQ ID NO:28, and SEQ ID NO:31, or the cDNA contained in the respective deposited cDNA clones. These probes will also hybridize to nucleic acid molecules in biological samples, thereby enabling a variety of forensic and diagnostic methods of the invention. Similarly, polypeptides identified from SEQ ID NO:2 and SEQ ID NO:29 may be used to generate antibodies which bind specifically to IL-21 and polypeptides identified from SEQ ID NO:4 and SEQ ID NO:32 may be used to generate antibodies which bind specifically to IL-22.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:1 and the predicted translated amino acid sequence identified as SEQ ID NO:2, and the generated nucleotide sequence identified as SEQ ID NO:28 and the predicted translated amino acid sequence identified as SEQ ID NO:29, but also a sample of plasmid DNA containing a human cDNA of IL-21 deposited with the ATCC. In addition, the present invention also provides not only the generated nucleotide sequence identified as SEQ ID NO:3 and the predicted translated amino acid sequence identified as SEQ ID NO:4, and the generated nucleotide sequence identified as SEQ ID NO:3 and the predicted translated amino acid sequence identified as SEQ ID NO:4, but also a sample of plasmid DNA containing a human cDNA of IL-22 deposited with the ATCC. Accordingly, the nucleotide sequence of the deposited IL-21 and IL-22 clones can be readily determined by sequencing the deposited clone in accordance with known methods. The predicted IL-21 and IL-22 amino acid sequences can then be verified from such deposits. Moreover, the amino acid sequence of the protein encoded by the deposited clone can also be directly determined by peptide sequencing or by expressing the protein in a suitable host cell containing the deposited human IL-21 or IL-22 cDNAs, collecting the protein, and determining its sequence.

The present invention also relates to the IL-21 gene corresponding to SEQ ID NO:1, SEQ ID NO:2, SEQ ID NO:28, SEQ ID NO:29 or the deposited clone which encodes a partial IL-21. The present invention further relates to the IL-22 gene corresponding to SEQ ID NO:3, SEQ ID NO:4, SEQ ID NO:31, SEQ ID NO:32 or the deposited clone which encodes IL-22. The IL-21 and IL-22 genes can be isolated in accordance with known methods using the sequence information disclosed herein. Such methods include preparing probes or primers from the disclosed sequences and identifying or amplifying the IL-21 and IL-22 genes from appropriate sources of genomic material.

Also provided in the present invention are species homologs of IL-21 and IL-22. Species homologs may be isolated and identified by making suitable probes or primers from the sequences provided herein and screening a suitable nucleic acid source for the desired homolog.

The IL-21 and IL-22 polypeptides can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The IL-21 and IL-22 polypeptides may be in the form of the secreted protein, including the mature form, or may be a part of a larger protein, such as a fusion protein. It is often advantageous to include an additional amino acids which comprise secretory or leader sequences, pro-sequences, sequences which aid in purification, such as multiple histidine residues, or an additional sequence for stability during recombinant production.

IL-21 and IL-22 polypeptides are preferably provided in an isolated form, and preferably are substantially purified. A recombinantly produced version of an IL-21 or IL-22 polypeptide, including the secreted polypeptide, can be substantially purified by the one-step method described in the publication by Smith and Johnson (*Gene* 67:31-40 (1988)). IL-21 and IL-22 polypeptides also can be purified from natural or recombinant sources using antibodies of the invention raised against the IL-21 and IL-22 proteins, respectively, in methods which are well known in the art.

### Polynucleotide and Polypeptide Variants

"Variant" refers to a polynucleotide or polypeptide differing from the IL-21 and IL-22 polynucleotides or polypeptides, but retaining essential properties thereof. Generally, variants are overall closely similar, and, in many regions, identical to the IL-21 and IL-22 polynucleotide or polypeptide.

By a polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence encoding the IL-21 or IL-22 polypeptides. In other words, to obtain a polynucleotide having a nucleotide sequence at least 95% identical to a reference nucleotide sequence, up to 5% of the nucleotides in the reference sequence may be inserted, deleted or substituted with another nucleotide. The query sequence may be an entire sequence shown of SEQ ID NO:1, SEQ ID NO:3, SEQ ID NO:28, SEQ ID NO:31, the ORF (open reading frame) of either IL-21 or IL-22, or any fragment specified as described herein.

As a practical matter, whether any particular nucleic acid molecule or polypeptide is at least 90%, 95%, 96%, 97%, 98% or 99% identical to (or 10%, 5%, 4%, 3%, 2% or 1% different from) a nucleotide sequence of the presence invention can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brutlag and colleagues (*Comp*. *App. Biosci.* 6:237-245 (1990)). In a sequence alignment the query and subject sequences are both DNA sequences. An RNA sequence can be compared by converting (uridine residues (U) to thymidine residues (T). The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

If the subject sequence is shorter than the query sequence because of 5' or 3' deletions, but not because of internal deletions, a manual correction must be made to the results. This is because the FASTDB algorithm does not account for 5' and 3' truncations of the subject sequence when calculating percent identity. For subject sequences truncated at the 5' or 3' ends, relative to the the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence ((number of bases at the 5' and 3' ends not matched)/(total number of bases in the query sequence)), so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequnce are manually corrected for. No other manual corrections are to made for the purposes of the present invention.

By a polypeptide having an amino acid sequence which is, at least, for example, 95% "identical" to (or 5% different from) a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (insertions and deletions are collectively referred to as "indels" in the art) or substituted with another amino acid. These alterations of the reference sequence may occur at the amino- or carboxy-terminal positions of the reference amino acid sequence or anywhere between those terminal positions, interspersed either individually among residues in the reference sequence or in one or more contiguous groups within the reference sequence.

As a practical matter, whether any particular polypeptide is at least 90%, 95%, 96%, 97%, 98% or 99% identical to (or 10%, 5%, 4%, 3%, 2% or 1% different from), for instance, the amino acid sequences shown in SEQ ID NO:2 or SEQ ID NO:29, or that shown in SEQ ID NO:4 or SEQ ID NO:32, or to the amino acid sequence encoded by deposited cDNA clones, can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag and colleagues (*Comp. App. Biosci*. 6:237-245 (1990)). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1; Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is becuase the FASTDB program does not account for N- and C-terminal truncations of the subject sequence when calculating global percent identity. For subject sequences truncated at the N- and C-termini, relative to the the query sequence, the percent identity is corrected by calculating the number of residues of the query sequence that are N-and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence), so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The IL-21 and IL-22 variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. IL-21 and IL-22 polynucleotide variants can be produced for a variety of reasons, e.g., to optimize codon expression for a particular host (change codons in the human mRNA to those preferred by a bacterial host such as *E. coli*).

Naturally occurring IL-21 and IL-22 variants are called "allelic variants," and refer to one of several alternate forms of a gene occupying a given locus on a chromosome of an organism (*Genes II*, Lewin, B., ed., John Wiley & Sons, New York (1985)). These allelic variants can vary at either the polynucleotide and/or polypeptide level. Alternatively, non-naturally occurring variants may be produced by mutagenesis techniques or by direct synthesis.

Using known methods of protein engineering and recombinant DNA technology, variants may be generated to improve or alter the characteristics of the IL-21 and IL-22 polypeptides. For instance, one or more amino acids can be deleted from the N-terminus or C-terminus of the secreted protein without substantial loss of biological function. Ron and coworkers reported variant KGF proteins having heparin binding activity even after deleting 3, 8, or 27 amino-terminal amino acid residues (*J. Biol. Chem*. 268:2984-2988 (1993)). Similarly, Interferon gamma exhibited up to ten times higher activity after deleting 8-10 amino acid residues from the carboxy terminus of this protein (Dobeli, *et al., J. Biotechnol*. 7:199-216 (1988)).

In the present case, since the IL-21 and IL-22 proteins of the invention are highly related to the Interleukin-17-like polypeptide family, deletions of N-terminal amino acids up to the cysteine at position 19 of SEQ ID NO:2 and up to the cysteine at position 29 of SEQ ID NO:4 may retain some biological activity. Polypeptides having further N-terminal deletions including the cysteine-19 residue in SEQ ID NO:2 and the cysteine-29 residue in SEQ ID NO:4 would not be expected to retain such biological activities because it is likely that these residues are required for forming a disulfide bridge to provide structural stability which is needed for receptor binding and signal transduction.

However, even if deletion of one or more amino acids from the N-terminus of a protein results in modification or loss of one or more biological functions of the protein, other biological activities may still be retained. Thus, the ability of the shortened protein to induce and/or bind to antibodies which recognize the complete or mature IL-21 or IL-22 proteins generally will be retained when less than the majority of the residues of the complete or mature IL-21 or IL-22 proteins are removed from the N-termini of the respective proteins. Whether a particular polypeptide lacking N-terminal residues of a complete protein retains such immunologic activities can readily be determined by routine methods described herein and otherwise known in the art.

Accordingly, the present invention further provides polypeptides having one or more residues deleted from the amino terminus of the amino acid sequence of the IL-21 polypeptide shown in SEQ ID NO:2, up to the cysteine residue at position number 19, and polynucleotides encoding such polypeptides. In addition, the present invention further provides polypeptides having one or more residues deleted from the amino terminus of the amino acid sequence of the IL-22 polypeptide shown in SEQ ID NO:4, up to the cysteine residue at position number 29, and polynucleotides encoding such polypeptides. In particular, the present invention provides polypeptides comprising the amino acid sequence of residues n¹-87 of SEQ ID NO:2, where n¹ is an integer in the range of 1 to 18, and 19 is the position of the first residue from the N-terminus of the complete IL-21 polypeptide (shown in SEQ ID NO:2) believed to be required for the receptor binding activity of the IL-21 protein. Likewise, the present invention provides polypeptides comprising the amino acid sequence of residues n²-160 of SEQ ID NO:4, where n² is an integer in the range of 1 to 28, and 29 is the position of the first residue from the N-terminus of the complete IL-22 polypeptide (shown in SEQ ID NO:4) believed to be required for the receptor binding activity of the IL-22 protein.

More in particular, the invention provides polynucleotides encoding polypeptides comprising, or alternatively consisting of, the amino acid sequence of residues 1-87, 2-87, 3-87, 4-87, 5-87, 6-87, 7-87, 8-87, 9-87, 10-87, 11-87, 12-87, 13-87, 14-87, 15-87, 16-87, 17-87, 18-87, and 19-87 of SEQ ID NO:2. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

The invention also provides polynucleotides encoding polypeptides comprising, or alternatively consisting of, the amino acid sequence of residues 1-160, 2-160, 3-160, 4-160, 5-160, 6-160, 7-160, 8-160, 9-160, 10-160, 11-160, 12-160, 13-160, 14-160, 15-160, 16-160, 17-160, 18-160, 19-160, 20-160, 21-160, 22-160, 23-160, 24-160, 25-160, 26-160, 27-160, 28-160, and 29-160 of SEQ ID NO:4. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

In addition, since the IL-21 and IL-22 proteins of the invention are highly related to the IL-17-like polypeptide family, deletions of C-terminal amino acids up to the leucine at postion 83 of SEQ ID NO:2 and up to the proline at position 129 of SEQ ID NO:4 may retain some biological activity. Polypeptides having further C-terminal deletions including the leucine residue at position 83 of SEQ ID NO:2 and the proline at position 129 of SEQ ID NO:4 would not be expected to retain such biological activities since these residues are in the beginning of the conserved domain required for biological activities.

However, even if deletion of one or more amino acids from the C-terminus of a protein results in modification of loss of one or more biological functions of the protein, other biological activities may still be retained. Thus, the ability of the shortened protein to induce and/or bind to antibodies which recognize the complete or mature IL-21 and IL-22 proteins generally will be retained when less than the majority of the residues of the complete or mature IL-21 and IL-22 proteins are removed from the C-terminus. Whether a particular polypeptide lacking C-terminal residues of a complete protein retains such immunologic activities can readily be determined by routine methods described herein and otherwise known in the art.

Accordingly, the present invention further provides polypeptides having one or more residues removed from the carboxy terminus of the amino acid sequence of the IL-21 polypeptide shown in SEQ ID NO:2, up to the leucine residue at position 83 of SEQ ID NO:2, and polynucleotides encoding such polypeptides. In addition, the present invention further provides polypeptides having one or more residues removed from the carboxy terminus of the amino acid sequence of the IL-22 polypeptide shown in SEQ ID NO:4, up to the proline residues at position 129 of SEQ ID NO:4. In particular, the present invention provides polypeptides having the amino acid sequence of residues 1-m¹ of the amino acid sequence in SEQ ID NO:2, where m¹ is any integer in the range of 83 to 87, and residue 82 is the position of the first residue from the C-terminus of the complete IL-21 polypeptide (shown in SEQ ID NO:2) believed to be required for activity of the IL-21 protein. In addition, the present invention also provides polypeptides having the amino acid sequence of residues 1-m² of the amino acid sequence in SEQ ID NO:4, where m² is any integer in the range of 129 to 160, and residue 128 is the position of the first residue from the C-terminus of the complete IL-22 polypeptide (shown in SEQ ID NO:4) believed to be required for activity of the IL-22 protein.

More in particular, the invention provides polynucleotides encoding polypeptides comprising, or alternatively consisting of, the amino acid sequence of residues 1-83, 1-84, 1-85, 1-86, and 1-87 of SEQ ID NO:2. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

The present invention also provides polynucleotides encoding polypeptides comprising, or alternatively consisting of, the amino acid sequence of residues 1-129, 1-130, 1-131, 1-132, 1-133, 1-134, 1-135, 1-136, 1-137, 1-138, 1-139, 1-140, 1-141, 1-142, 1-143, 1-144, 1-145, 1-146, 1-147, 1-148, 1-149, 1-150, 1-151, 1-152, 1-153, 1-154, 1-155, 1-156, 1-157, 1-158, 1-159, and 1-160 of SEQ ID NO:4. Polypeptides encoded by these polynucleotides are also provided: The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

The invention also provides polypeptides having one or more amino acids deleted from both the amino and the carboxyl termini of IL-21, which may be described generally as having residues n¹-m¹ of SEQ ID NO:2, where n¹ and m¹ are integers as described above. Likewise, the invention also provides polypeptides having one or more amino acids deleted from both the amino and the carboxyl termini of IL-22, which may be described generally as having residues n²-m² of SEQ ID NO:4, where n² and m² are integers as described above.

Moreover, ample evidence demonstrates that variants often retain a biological activity similar to that of the naturally occurring protein. For example, Gayle and coworkers conducted extensive mutational analysis of human cytokine IL-1a (*J. Biol*. *Chem*. 268:22105-22111 (1993)). They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]" (see, Abstract). In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking Nor C-terminal residues of a protein retains such immunogenic activities can readily be determined by routine methods described herein and otherwise known in the art.

As mentioned above, even if deletion of one or more amino acids from the N-terminus of a protein results in modification or loss of one or more biological functions of the protein, other biological activities may still be retained. Thus, the ability of the shortened protein to induce and/or bind to antibodies which recognize the complete or mature IL-21 or IL-22 proteins generally will be retained when less than the majority of the residues of the complete or mature IL-21 or IL-22 proteins are removed from the N-termini of the respective proteins. Whether a particular polypeptide lacking N-terminal residues of a complete protein retains such immunologic activities can readily be determined by routine methods described herein and otherwise known in the art.

Accordingly, the present invention further provides polypeptides having one or more residues deleted from the amino terminus of the amino acid sequence of the IL-21 polypeptide shown in SEQ ID NO:2, up to the valine residue at position number 82, and polynucleotides encoding such polypeptides. In addition, the present invention further provides polypeptides having one or more residues deleted from the amino terminus of the amino acid sequence of the IL-22 polypeptide shown in SEQ ID NO:4, up to the aspartic acid residue at position number 155, and polynucleotides encoding such polypeptides. In particular, the present invention provides polypeptides comprising the amino acid sequence of residues n³-87 of SEQ ID NO:2, where n³ is an integer in the range of 1 to 82, and 83 is the position of the first residue from the N-terminus of the complete IL-21 polypeptide (shown in SEQ ID NO:2) believed to be required for immunogenic activity of the IL-21 protein. Likewise, the present invention provides polypeptides comprising the amino acid sequence of residues n⁴-160 of SEQ ID NO:4, where n⁴ is an integer in the range of 1 to 155, and 156 is the position of the first residue from the N-terminus of the complete IL-22 polypeptide (shown in SEQ ID NO:4) believed to be required for immunogenic activity of the IL-22 protein.

More in particular, the invention provides polynucleotides encoding polypeptides comprising, or alternatively consisting of, the amino acid sequence of residues R-2 to V-87; V-3 to V-87; D-4 to V-87; T-5 to V-87; D-6 to V-87; E-7 to V-87; D-8 to V-87; R-9 to V-87; Y-10 to V-87; P-1 to V-87; Q-12 to V-87; K-13 to V-87; L-14 to V-87; A-15 to V-87; F-16 to V-87; A-17 to V-87; E-18 to V-87; C-19 to V-87; L-20 to V-87; C-21 to V-87; R-22 to V-87; G-23 to V-87; C-24 to V-87; I-25 to V-87; D-26 to V-87; A-27 to V-87; R-28 to V-87; T-29 to V-87; G-30 to V-87; R-31 to V-87; E-32 to V-87; T-33 to V-87; A-34 to V-87; A-35 to V-87; L-36 to V-87; N-37 to V-87; S-38 to V-87; V-39 to V-87; R-40 to V-87; L-41 to V-87; L-42 to V-87; Q-43 to V-87; S-44 to V-87; L-45 to V-87; L-46 to V-87; V-47 to V-87; L-48 to V-87; R-49 to V-87; R-50 to V-87; R-51 to V-87; P-52 to V-87; C-53 to V-87; S-54 to V-87; R-55 to V-87; D-56 to V-87; G-57 to V-87; S-58 to V-87; G-59 to V-87; L-60 to V-87; P-61 to V-87; T-62 to V-87; P-63 to V-87; G-64 to V-87; A-65 to V-87; F-66 to V-87; A-67 to V-87; F-68 to V-87; H-69 to V-87; T-70 to V-87; E-71 to V-87; F-72 to V-87; I-73 to V-87; H-74 to V-87; V-75 to V-87; P-76 to V-87; V-77 to V-87; G-78 to V-87; C-79 to V-87; T-80 to V-87; C-81 to V-87; and V-82 to V-87 of SEQ ID NO:2. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

Further, the invention provides polynucleotides encoding polypeptides comprising, or alternatively consisting of, the amino acid sequence of residues S-2 to P-160; A-3 to P-160; R-4 to P-160; A-5 to P-160; R-6 to P-160; A-7 to P-160; V-8 to P-160; L-9 to P-160; S-10 to P-160; A-11 to P-160; F-12 to P-160; H-13 to P-160; H-14 to P-160; T-15 to P-160; L-16 to P-160; Q-17 to P-160; L-18 to P-160; G-19 to P-160; P-20 to P-160; R-21 to P-160; E-22 to P-160; Q-23 to P-160; A-24 to P-160; R-25 to P-160; N-26 to P-160; A-27 to P-160; S-28 to P-160; C-29 to P-160; P-30 to P-160; A-31 to P-160; G-32 to P-160; G-33 to P-160; R-34 to P-160; P-35 to P-160; A-36 to P-160; D-37 to P-160; R-38 to P-160; R-39 to P-160; F-40 to P-160; R-41 to P-160; P-42 to P-160; P-43 to P-160; T-44 to P-160; N-45 to P-160; L-46 to P-160; R-47 to P-160; S-48 to P-160; V-49 to P-160; S-50 to P-160; P-51 to P-160; W-52 to P-160; A-53 to P-160; Y-54 to P-160; R-55 to P-160; I-56 to P-160; S-57 to P-160; Y-58 to P-160; D-59 to P-160; P-60 to P-160; A-61 to P-160; R-62 to P-160; Y-63 to P-160; P-64 to P-160; R-65 to P-160; Y-66 to P-160; L-67 to P-160; P-68 to P-160; E-69 to P-160; A-70 to P-160; Y-71 to P-160; C-72 to P-160; L-73 to P-160; C-74 to P-160; R-75 to P-160; G-76 to P-160; C-77 to P-160; L-78 to P-160; T-79 to P-160; G-80 to P-160; L-81 to P-160; F-82 to P-160; G-83 to P-160; E-84 to P-160; E-85 to P-160; D-86 to P-160; V-87 to P-160; R-88 to P-160; F-89 to P-160; R-90 to P-160; S-91 to P-160; A-92 to P-160; P-93 to P-160; V-94 to P-160; Y-95 to P-160; M-96 to P-160; P-97 to P-160; T-98 to P-160; V-99 to P-160; V-100 to P-160; L-101 to P-160; R-102 to P-160; R-103 to P-160; T-104 to P-160; P-105 to P-160; A-106 to P-160; C-107 to P-160; A-108 to P-160; G-109 to P-160; G-110 to P-160; R-111 to P-160; S-112 to P-160; V-113 to P-160; Y-114 to P-160; T-115 to P-160; E-116 to P-160; A-117 to P-160; Y-118 to P-160; V-119 to P-160; T-120 to P-160; I-121 to P-160; P-122 to P-160; V-123 to P-160; G-124 to P-160; C-125 to P-160; T-126 to P-160; C-127 to P-160; V-128 to P-160; P-129 to P-160; E-130 to P-160; P-131 to P-160; E-132 to P-160; K-133 to P-160; D-134 to P-160; A-135 to P-160; D-136 to P-160; S-137 to P-160; 1-138 to P-160; N-139 to P-160; S-140 to P-160; S-141 to P-160; 1-142 to P-160; D-143 to P-160; K-144 to P-160; Q-145 to P-160; G-146 to P-160; A-147 to P-160; K-148 to P-160; L-149 to P-160; L-150 to P-160; L-151 to P-160; G-152 to P-160; P-153 to P-160; N-154 to P-160; and D-155 to P-160 of SEQ ID NO:4. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

Also as mentioned above, even if deletion of one or more amino acids from the C-terminus of a protein results in modification of loss of one or more biological functions of the protein, other biological activities may still be retained. Thus, the ability of the shortened protein to induce and/or bind to antibodies which recognize the complete or mature IL-21 and IL-22 proteins generally will be retained when less than the majority of the residues of the complete or mature IL-21 and IL-22 proteins are removed from the C-terminus. Whether a particular polypeptide lacking C-terminal residues of a complete protein retains such immunologic activities can readily be determined by routine methods described herein and otherwise known in the art.

Accordingly, the present invention further provides polypeptides having one or more residues removed from the carboxy terminus of the amino acid sequence of the IL-21 polypeptide shown in SEQ ID NO:2, up to the aspartic acid residue at position 6 of SEQ ID NO:2, and polynucleotides encoding such polypeptides. In addition, the present invention further provides polypeptides having one or more residues removed from the carboxy terminus of the amino acid sequence of the IL-22 polypeptide shown in SEQ ID NO:4, up to the arginine residues at position 6 of SEQ ID NO:4. In particular, the present invention provides polypeptides having the amino acid sequence of residues 1-m³ of the amino acid sequence in SEQ ID NO:2, where m³ is any integer in the range of 6 to 87, and residue 5 is the position of the first residue from the C-terminus of the complete IL-21 polypeptide (shown in SEQ ID NO:2) believed to be required for immunogenic activity of the IL-21 protein. In addition, the present invention also provides polypeptides having the amino acid sequence of residues 1-m⁴ of the amino acid sequence in SEQ ID NO:4, where m⁴ is any integer in the range of 6 to 160, and residue 5 is the position of the first residue from the C-terminus of the complete IL-22 polypeptide (shown in SEQ ID NO:4) believed to be required for immunogenic activity of the IL-22 protein.

More in particular, the invention provides polynucleotides encoding polypeptides comprising, or alternatively consisting of, the amino acid sequence of residues A-1 to S-86; A-1 to R-85; A-1 to P-84; A-1 to L-83; A-1 to V-82; A-1 to C-81; A-1 to T-80; A-1 to C-79; A-1 to G-78; A-1 to V-77; A-1 to P-76; A-1 to V-75; A-1 to H-74; A-1 to 1-73; A-1 to F-72; A-1 to E-71; A-1 to T-70; A-1 to H-69; A-1 to F-68; A-1 to A-67; A-1 to F-66; A-1 to A-65; A-1 to G-64; A-1 to P-63; A-1 to T-62; A-1 to P-61; A-1 to L-60; A-1 to G-59; A-1 to S-58; A-1 to G-57; A-1 to D-56; A-1 to R-55; A-1 to S-54; A-1 to C-53; A-1 to P-52; A-1 to R-51; A-1 to R-50; A-1 to R-49; A-1 to L-48; A-1 to V-47; A-1 to L-46; A-1 to L-45; A-1 to S-44; A-1 to Q-43; A-1 to L-42; A-1 to L-41; A-1 to R-40; A-1 to V-39; A-1 to S-38; A-1 to N-37; A-1 to L-36; A-1 to A-35; A-1 to A-34; A-1 to T-33; A-1 to E-32; A-1 to R-31; A-1 to G-30; A-1 to T-29; A-1 to R-28; A-1 to A-27; A-1 to D-26; A-1 to 1-25; A-1 to C-24; A-1 to G-23; A-1 to R-22; A-1 to C-21; A-1 to L-20; A-1 to C-19; A-1 to E-18; A-1 to A-17; A-1 to F-16; A-1 to A-15; A-1 to L-14; A-1 to K-13; A-1 to Q-12; A-1 to P-11; A-1 to Y-10; A-1 to R-9; A-1 to D-8; A-1 to E-7; and A-1 to D-6 of SEQ ID NO:2. Polynucleotides encoding these polypeptides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

Moreover, the invention also provides polynucleotides encoding polypeptides comprising, or alternatively consisting of, the amino acid sequence of residues N-1 to G-159; N-1 to A-158; N-1 to P-157; N-1 to A-156; N-1 to D-155; N-1 to N-154; N-1 to P-153; N-1 to G-152; N-1 to L-151; N-1 to L-150; N-1 to L-149; N-1 to K-148; N-1 to A-147; N-1 to G-146; N-1 to Q-145; N-1 to K-144; N-1 to D-143; N-1 to I-142; N-1 to S-141; N-1 to S-140; N-1 to N-139; N-1 to I-138; N-1 to S-137; N-1 to D-136; N-1 to A-135; N-1 to D-134; N-1 to K-133; N-1 to E-132; N-1 to P-131; N-1 to E-130; N-1 to P-129; N-1 to V-128; N-1 to C-127; N-1 to T-126; N-1 to C-125; N-1 to G-124; N-1 to V-123; N-1 to P-122; N-1 to I-121; N-1 to T-120; N-1 to V-119; N-1 to Y-118; N-1 to A-117; N-1 to E-116; N-1 to T-115; N-1 to Y-114; N-1 to V-113; N-1 to S-112; N-1 to R-111; N-1 to G-110; N-1 to G-109; N-1 to A-108; N-1 to C-107; N-1 to A-106; N-1 to P-105; N-1 to T-104; N-1 to R-103; N-1 to R-102; N-1 to L-101; N-1 to V-100; N-1 to V-99; N-1 to T-98; N-1 to P-97; N-1 to M-96; N-1 to Y-95; N-1 to V-94; N-1 to P-93; N-1 to A-92; N-1 to S-91; N-1 to R-90; N-1 to F-89; N-1 to R-88; N-1 to V-87; N-1 to D-86; N-1 to E-85 ; N-1 to E-84; N-1 to G-83; N-1 to F-82; N-1 to L-81; N-1 to G-80; N-1 to T-79; N-1 to L-78; N-1 to C-77; N-1 to G-76; N-1 to R-75; N-1 to C-74; N-1 to L-73; N-1 to C-72; N-1 to Y-71; N-1 to A-70; N-1 to E-69; N-1 to P-68; N-1 to L-67; N-1 to Y-66; N-1 to R-65; N-1 to P-64; N-1 to Y-63; N-1 to R-62; N-1 to A-61; N-1 to P-60; N-1 to D-59; N-1 to Y-58; N-1 to S-57; N-1 to I-56; N-1 to R-55; N-1 to Y-54; N-1 to A-53; N-1 to W-52; N-1 to P-51; N-1 to S-50; N-1 to V-49; N-1 to S-48; N-1 to R-47; N-1 to L-46; N-1 to N-45; N-1 to T-44; N-1 to P-43; N-1 to P-42; N-1 to R-41; N-1 to F-40; N-1 to R-39; N-1 to R-38; N-1 to D-37; N-1 to A-36; N-1 to P-35; N-1 to R-34; N-1 to G-33; N-1 to G-32; N-1 to A-31; N-1 to P-30; N-1 to C-29; N-1 to S-28; N-1 to A-27; N-1 to N-26; N-1 to R-25; N-1 to A-24; N-1 to Q-23; N-1 to E-22; N-1 to R-21; N-1 to P-20; N-1 to G-19; N-1 to L-18; N-1 to Q-17; N-1 to L-16; N-1 to T-15; N-1 to H-14; N-1 to H-13; N-1 to F-12; N-1 to A-11; N-1 to S-10; N-1 to L-9; N-1 to V-8; N-1 to A-7; and N-1 to R-6 of SEQ ID NO:4. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

The invention also provides polypeptides having one or more amino acids deleted from both the amino and the carboxyl termini of IL-21, which may be described generally as having residues n³-m³ of SEQ ID NO:2, where n³ and m³ are integers as described above. Likewise, the invention also provides polypeptides having one or more amino acids deleted from both the amino and the carboxyl termini of IL-22, which may be described generally as having residues n⁴-m⁴ of SEQ ID NO:4, where n⁴ and m⁴ are integers as described above.

Moreover, any polypeptide having one or more amino acids deleted from both the amino and the carboxyl termini of IL-22, described specifically as having residues n⁴-m⁴ of SEQ ID NO:4 (where n⁴ and m⁴ are integers as described above) may be excluded from the invention. In particular, any polypeptide having one or more amino acids deleted from both the amino and the carboxyl termini of IL-22 and which is defined by residues n⁴-m⁴ of SEQ ID NO:4, where n" is equal to 21, 22, 23, 24 or 25 and m⁴ is equal to 271, 272, 273, 274, 275 or 276 may be excluded from the invention.

Also as mentioned above, even if deletion of one or more amino acids from the N-terminus of a protein results in modification or loss of one or more biological functions of the protein, other biological activities may still be retained. Thus, the ability of the shortened protein to induce and/or bind to antibodies which recognize the full-length or mature IL-21 polypeptides generally will be retained when less than the majority of the residues of the full-length or mature IL-21 polypeptides are removed from the N-terminus. Whether a particular polypeptide lacking N-terminal residues of a complete or full-length polypeptide retains such immunologic activities can readily be determined by routine methods described herein and otherwise known in the art.

Accordingly, the present invention further provides polypeptides having one or more residues deleted from the amino terminus of the amino acid sequence of the IL-21 polypeptide shown in SEQ ID NO:29, up to the valine residue at position number 192, and polynucleotides encoding such polypeptides. In particular, the present invention provides polypeptides comprising the amino acid sequence of residues n⁵-197 of SEQ ID NO:29, where n⁵ is an integer in the range of 1 to 192, and 193 is the position of the first residue from the N-terminus of the full-length IL-21 polypeptide (shown in SEQ ID NO:29) believed to be required for immunogenic activity of the IL-21 protein.

More in particular, the invention provides polynucleotides encoding polypeptides comprising, or alternatively consisting of, the amino acid sequence of residues T-2 to V-197; L-3 to V-197; L-4 to V-197; P-5 to V-197; G-6 to V-197; L-7 to V-197; L-8 to V-197; F-9 to V-197; L-10 to V-197; T-11 to V-197; W-12 to V-197; L-13 to V-197; H-14 to V-197; T-15 to V-197; C-16 to V-197; L-17 to V-197; A-18 to V-197; H-19 to V-197; H-20 to V-197; D-21 to V-197; P-22 to V-197; S-23 to V-197; L-24 to V-197; R-25 to V-197; G-26 to V-197; H-27 to V-197; P-28 to V-197; H-29 to V-197; S-30 to V-197; H-31 to V-197; G-32 to V-197; T-33 to V-197; P-34 to V-197; H-35 to V-197; C-36 to V-197; Y-37 to V-197; S-38 to V-197; A-39 to V-197; E-40 to V-197; E-41 to V-197; L-42 to V-197; P-43 to V-197; L-44 to V-197; G-45 to V-197; Q-46 to V-197; A-47 to V-197; P-48 to V-197; P-49 to V-197; H-50 to V-197; L-51 to V-197; L-52 to V-197; A-53 to V-197; R-54 to V-197; G-55 to V-197; A-56 to V-197; K-57 to V-197; W-58 to V-197; G-59 to V-197; Q-60 to V-197; A-61 to V-197; L-62 to V-197; P-63 to V-197; V-64 to V-197; A-65 to V-197; L-66 to V-197; V-67 to V-197; S-68 to V-197; S-69 to V-197; L-70 to V-197; E-71 to V-197; A-72 to V-197; A-73 to V-197; S-74 to V-197; H-75 to V-197; R-76 to V-197; G-77 to V-197; R-78 to V-197; H-79 to V-197; E-80 to V-197; R-81 to V-197; P-82 to V-197; S-83 to V-197; A-84 to V-197; T-85 to V-197; T-86 to V-197; Q-87 to V-197; C-88 to V-197; P-89 to V-197; V-90 to V-197; L-91 to V-197; R-92 to V-197; P-93 to V-197; E-94 to V-197; E-95 to V-197; V-96 to V-197; L-97 to V-197; E-98 to V-197; A-99 to V-197; D-100 to V-197; T-101 to V-197; H-102 to V-197; Q-103 to V-197; R-104 to V-197; S-105 to V-197; I-106 to V-197; S-107 to V-197; P-108 to V-197; W-109 to V-197; R-110 to V-197; Y-111 to V-197; R-112 to V-197; V-113 to V-197; D-114 to V-197; T-115 to V-197; D-116 to V-197; E-117 to V-197; D-118 to V-197; R-119 to V-197; Y-120 to V-197; P-121 to V-197; Q-122 to V-197; K-123 to V-197; L-124 to V-197; A-125 to V-197; F-126 to V-197; A-127 to V-197; E-128 to V-197; C-129 to V-197; L-130 to V-197; C-131 to V-197; R-132 to V-197; G-133 to V-197; C-134 to V-197; I-135 to V-197; D-136 to V-197; A-137 to V-197; R-138 to V-197; T-139 to V-197; G-140 to V-197; R-141 to V-197; E-142 to V-197; T-143 to V-197; A-144 to V-197; A-145 to V-197; L-146 to V-197; N-147 to V-197; S-148 to V-197; V-149 to V-197; R-150 to V-197; L-151 to V-197; L-152 to V-197; Q-153 to V-197; S-154 to V-197; L-155 to V-197; L-156 to V-197; V-157 to V-197; L-158 to V-197; R-159 to V-197; R-160 to V-197; R-161 to V-197; P-162 to V-197; C-163 to V-197; S-164 to V-197; R-165 to V-197; D-166 to V-197; G-167 to V-197; S-168 to V-197; G-169 to V-197; L-170 to V-197; P-171 to V-197; T-172 to V-197; P-173 to V-197; G-174 to V-197; A-175 to V-197; F-176 to V-197; A-177 to V-197; F-178 to V-197; H-179 to V-197; T-180 to V-197; E-181 to V-197; F-182 to V-197; I-183 to V-197; H-184 to V-197; V-185 to V-197; P-186 to V-197; V-187 to V-197; G-188 to V-197; C-189 to V-197; T-190 to V-197; C-191 to V-197; and V-192 to V-197 of SEQ ID NO:29. Polypeptides encoded by these polynucleotides also are provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

Also as mentioned above, even if deletion of one or more amino acids from the C-terminus of a protein results in modification of loss of one or more biological functions of the protein, other biological activities may still be retained. Thus, the ability of the shortened polypeptide to induce and/or bind to antibodies which recognize the full-length or mature IL-21 polypeptide generally will be retained when less than the majority of the residues of the full-length or mature IL-21 polypeptides are removed from the C-terminus. Whether a particular polypeptide lacking C-terminal residues of a complete protein retains such immunologic activities can readily be determined by routine methods described herein and otherwise known in the art.

Accordingly, the present invention further provides polypeptides having one or more residues removed from the carboxy terminus of the amino acid sequence of the IL-21 polypeptide shown in SEQ ID NO:29, up to the glycine residue at position 6 of SEQ ID NO:29, and polynucleotides encoding such polypeptides. In particular, the present invention provides polypeptides having the amino acid sequence of residues 1-m⁵ of the amino acid sequence in SEQ ID NO:29, where m⁵ is any integer in the range of 6 to 196, and residue 5 is the position of the first residue from the C-terminus of the full-length IL-21 polypeptide (shown in SEQ ID NO:29) believed to be required for immunogenic activity of the IL-21 protein.

More in particular, the invention provides polynucleotides encoding polypeptides comprising, or alternatively consisting of, the amino acid sequence of residues M-1 to S-196; M-1 to R-195; M-1 to P-194; M-1 to L-193; M-1 to V-192; M-1 to C-191; M-1 to T-190; M-1 to C-189; M-1 to G-188; M-1 to V-187; M-1 to P-186; M-1 to V-185; M-1 to H-184; M-1 to I-183; M-1 to F-182; M-1 to E-181; M-1 to T-180; M-1 to H-179; M-1 to F-178; M-1 to A-177; M-1 to F-176; M-1 to A-175; M-1 to G-174; M-1 to P-173; M-1 to T-172; M-1 to P-171; M-1 to L-170; M-1 to G-169; M-1 to S-168; M-1 to G-167; M-1 to D-166; M-1 to R-165; M-1 to S-164; M-1 to C-163; M-1 to P-162; M-1 to R-161; M-1 to R-160; M-1 to R-159; M-1 to L-158; M-1 to V-157; M-1 to L-156; M-1 to L-155; M-1 to S-154; M-1 to Q-153; M-1 to L-152; M-1 to L-151; M-1 to R-150; M-1 to V-149; M-1 to S-148; M-1 to N-147; M-1 to L-146; M-1 to A-145; M-1 to A-144; M-1 to T-143; M-1 to E-142; M-1 to R-141; M-1 to G-140; M-1 to T-139; M-1 to R-138; M-1 to A-137; M-1 to D-136; M-1 to I-135; M-1 to C-134; M-1 to G-133; M-1 to R-132; M-1 to C-131; M-1 to L-130; M-1 to C-129; M-1 to E-128; M-1 to A-127; M-1 to F-126; M-1 to A-125; M-1 to L-124; M-1 to K-123; M-1 to Q-122; M-1 to P-121; M-1 to Y-120; M-1 to R-119; M-1 to D-118; M-1 to E-117; M-1 to D-116; M-1 to T-115; M-1 to D-114; M-1 to V-113; M-1 to R-112; M-1 to Y-111; M-1 to R-110; M-1 to W-109; M-1 to P-108; M-1 to S-107; M-1 to I-106; M-1 to S-105; M-1 to R-104; M-1 to Q-103; M-1 to H-102; M-1 to T-101; M-1 to D-100; M-1 to A-99; M-1 to E-98; M-1 to L-97; M-1 to V-96; M-1 to E-95; M-1 to E-94; M-1 to P-93; M-1 to R-92; M-1 to L-91; M-1 to V-90; M-1 to P-89; M-1 to C-88; M-1 to Q-87; M-1 to T-86; M-1 to T-85; M-1 to A-84; M-1 to S-83; M-1 to P-82; M-1 to R-81; M-1 to E-80; M-1 to H-79; M-1 to R-78; M-1 to G-77; M-1 to R-76; M-1 to H-75; M-1 to S-74; M-1 to A-73; M-1 to A-72; M-1 to E-71; M-1 to L-70; M-1 to S-69; M-1 to S-68; M-1 to V-67; M-1 to L-66; M-1 to A-65; M-1 to V-64; M-1 to P-63; M-1 to L-62; M-1 to A-61; M-1 to Q-60; M-1 to G-59; M-1 to W-58; M-1 to K-57; M-1 to A-56; M-1 to G-55; M-1 to R-54; M-1 to A-53; M-1 to L-52; M-1 to L-51; M-1 to H-50; M-1 to P-49; M-1 to P-48; M-1 to A-47; M-1 to Q-46; M-1 to G-45; M-1 to L-44; M-1 to P-43; M-1 to L-42; M-1 to E-41; M-1 to E-40; M-1 to A-39; M-1 to S-38; M-1 to Y-37; M-1 to C-36; M-1 to H-35; M-1 to P-34; M-1 to T-33; M-1 to G-32; M-1 to H-31; M-1 to S-30; M-1 to H-29; M-1 to P-28; M-1 to H-27; M-1 to G-26; M-1 to R-25; M-1 to L-24; M-1 to S-23; M-1 to P-22; M-1 to D-21; M-1 to H-20; M-1 to H-19; M-1 to A-18; M-1 to L-17; M-1 to C-16; M-1 to T-15; M-1 to H-14; M-1 to L-13; M-1 to W-12; M-1 to T-11; M-1 to L-10; M-1 to F-9; M-1 to L-8; M-1 to L-7; and M-1 to G-6 of SEQ ID NO:29. Polypeptides encoded by these polynucleotides also are provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

The invention also provides polypeptides having one or more amino acids deleted from both the amino and the carboxyl termini of IL-21, which may be described generally as having residues n⁵-m⁵ of SEQ ID NO:29, where n⁵ and m⁵ are integers as described above. Polynucleotides encoding such polypeptides are also provided.

Moreover, any polypeptide having one or more amino acids deleted from both the amino and the carboxyl termini of IL-21, described specifically as having residues n⁵-m⁵ of SEQ ID NO:29 (where n⁵ and m⁵ are integers as described above) may be excluded from the invention.

Also as mentioned above, even if deletion of one or more amino acids from the N-terminus of a protein results in modification or loss of one or more biological functions of the protein, other biological activities may still be retained. Thus, the ability of the shortened protein to induce and/or bind to antibodies which recognize the full-length, partial-length or mature IL-22 polypeptides generally will be retained when less than the majority of the residues of the full-length, partial-length or mature IL-22 polypeptides are removed from the N-terminus. Whether a particular polypeptide lacking N-terminal residues of a complete or full-length polypeptide retains such immunologic activities can readily be determined by routine methods described herein and otherwise known in the art.

Accordingly, the present invention further provides polypeptides having one or more residues deleted from the amino terminus of the amino acid sequence of the IL-22 polypeptide shown in SEQ ID NO:32, up to the aspartic acid residue at position number 168, and polynucleotides encoding such polypeptides. In particular, the present invention provides polypeptides comprising the amino acid sequence of residues n⁶-173 of SEQ ID NO:32, where n⁶ is an integer in the range of 1 to 168, and 168 is the position of the first residue from the N-terminus of the IL-22 polypeptide (shown in SEQ ID NO:32) believed to be required for immunogenic activity of the IL-22 protein.

More in particular, the invention provides polynucleotides encoding polypeptides comprising, or alternatively consisting of, the amino acid sequence of residues C-2 to P-173; A-3 to P-173; D-4 to P-173; R-5 to P-173; P-6 to P-173; E-7 to P-173; E-8 to P-173; L-9 to P-173; L-10 to P-173; E-11 to P-173; Q-12 to P-173; L-13 to P-173; Y-14 to P-173; G-15 to P-173; R-16 to P-173; L-17 to P-173; A-18 to P-173; A-19 to P-173; G-20 to P-173; V-21 to P-173; L-22 to P-173; S-23 to P-173; A-24 to P-173; F-25 to P-173; H-26 to P-173; H-27 to P-173; T-28 to P-173; L-29 to P-173; Q-30 to P-173; L-31 to P-173; G-32 to P-173; P-33 to P-173; R-34 to P-173; E-35 to P-173; Q-36 to P-173; A-37 to P-173; R-38 to P-173; N-39 to P-173; A-40 to P-173; S-41 to P-173; C-42 to P-173; P-43 to P-173; A-44 to P-173; G-45 to P-173; G-46 to P-173; R-47 to P-173; P-48 to P-173; A-49 to P-173; D-50 to P-173; R-51 to P-173; R-52 to P-173; F-53 to P-173; R-54 to P-173; P-55 to P-173; P-56 to P-173; T-57 to P-173; N-58 to P-173; L-59 to P-173; R-60 to P-173; S-61 to P-173; V-62 to P-173; S-63 to P-173; P-64 to P-173; W-65 to P-173; A-66 to P-173; Y-67 to P-173; R-68 to P-173; I-69 to P-173; S-70 to P-173; Y-71 to P-173; D-72 to P-173; P-73 to P-173; A-74 to P-173; R-75 to P-173; Y-76 to P-173; P-77 to P-173; R-78 to P-173; Y-79 to P-173; L-80 to P-173; P-81 to P-173; E-82 to P-173; A-83 to P-173; Y-84 to P-173; C-85 to P-173; L-86 to P-173; C-87 to P-173; R-88 to P-173; G-89 to P-173; C-90 to P-173; L-91 to P-173; T-92 to P-173; G-93 to P-173; L-94 to P-173; F-95 to P-173; G-96 to P-173; E-97 to P-173; E-98 to P-173; D-99 to P-173; V-100 to P-173; R-101 to P-173; F-102 to P-173; R-103 to P-173; S-104 to P-173; A-105 to P-173; P-106 to P-173; V-107 to P-173; Y-108 to P-173; M-109 to P-173; P-110 to P-173; T-111 to P-173; V-112 to P-173; V-113 to P-173; L-114 to P-173; R-115 to P-173; R-116 to P-173; T-117 to P-173; P-118 to P-173; A-119 to P-173; C-120 to P-173; A-121 to P-173; G-122 to P-173; G-123 to P-173; R-124 to P-173; S-125 to P-173; V-126 to P-173; Y-127 to P-173; T-128 to P-173; E-129 to P-173; A-130 to P-173; Y-131 to P-173; V-132 to P-173; T-133 to P-173; I-134 to P-173; P-135 to P-173; V-136 to P-173; G-137 to P-173; C-138 to P-173; T-139 to P-173; C-140 to P-173; V-141 to P-173; P-142 to P-173; E-143 to P-173; P-144 to P-173; E-145 to P-173; K-146 to P-173; D-147 to P-173; A-148 to P-173; D-149 to P-173; S-150 to P-173; I-151 to P-173; N-152 to P-173; S-153 to P-173; S-154 to P-173; I-155 to P-173; D-156 to P-173; K-157 to P-173; Q-158 to P-173; G-159 to P-173; A-160 to P-173; K-161 to P-173; L-162 to P-173; L-163 to P-173; L-164 to P-173; G-165 to P-173; P-166 to P-173; N-167 to P-173; and D-168 to P-173 of SEQ ID NO:32. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-21 polypeptides described above. The present invention also encompasses the above polynucleotide sequences fused to a heterologous polynucleotide sequence.

Also as mentioned above, even if deletion of one or more amino acids from the C-terminus of a protein results in modification of loss of one or more biological functions of the protein, other biological activities may still be retained. Thus, the ability of the shortened polypeptide to induce and/or bind to antibodies which recognize the full-length, partial-length or mature IL-22 polypeptide generally will be retained when less than the majority of the residues of the full-length, partial-length or mature IL-22 polypeptides are removed from the C-terminus. Whether a particular polypeptide lacking C-terminal residues of a complete protein retains such immunologic activities can readily be determined by routine methods described herein and otherwise known in the art.

Accordingly, the present invention further provides polypeptides having one or more residues removed from the carboxy terminus of the amino acid sequence of the IL-22 polypeptide shown in SEQ ID NO:32, up to the proline residue at position 6 of SEQ ID NO:32, and polynucleotides encoding such polypeptides. In particular, the present invention provides polypeptides having the amino acid sequence of residues 1-m⁶ of the amino acid sequence in SEQ ID NO:32, where m⁶ is any integer in the range of 6 to 173, and residue 6 is the position of the first residue from the C-terminus of the IL-22 polypeptide (shown in SEQ ID NO:32) believed to be required for immunogenic activity of the IL-22 protein.

More in particular, the invention provides polynucleotides encoding polypeptides comprising, or alternatively consisting of, the amino acid sequence of residues G-1 to G-172; G-1 to A-171; G-1 to P-170; G-1 to A-169; G-1 to D-168; G-1 to N-167; G-1 to P-166; G-1 to G-165; G-1 to L-164; G-1 to L-163; G-1 to L-162; G-1 to K-161; G-1 to A-160; G-1 to G-159; G-1 to Q-158; G-1 to K-157; G-1 to D-156; G-1 to I-155; G-1 to S-154; G-1 to S-153; G-1 to N-152; G-1 to I-151; G-1 to S-150; G-1 to D-149; G-1 to A-148; G-1 to D-147; G-1 to K-146; G-1 to E-145; G-1 to P-144; G-1 to E-143; G-1 to P-142; G-1 to V-141; G-1 to C-140; G-1 to T-139; G-1 to C-138; G-1 to G-137; G-1 to V-136; G-1 to P-135; G-1 to I-134; G-1 to T-133; G-1 to V-132; G-1 to Y-131; G-1 to A-130; G-1 to E-129; G-1 to T-128; G-1 to Y-127; G-1 to V-126; G-1 to S-125; G-1 to R-124; G-1 to G-123; G-1 to G-122; G-1 to A-121; G-1 to C-120; G-1 to A-119; G-1 to P-118; G-1 to T-117; G-1 to R-116; G-1 to R-115; G-1 to L-114; G-1 to V-113; G-1 to V-112; G-1 to T-111; G-1 to P-110; G-1 to M-109; G-1 to Y-108; G-1 to V-107; G-1 to P-106; G-1 to A-105; G-1 to S-104; G-1 to R-103; G-1 to F-102; G-1 to R-101; G-1 to V-100; G-1 to D-99; G-1 to E-98; G-1 to E-97; G-1 to G-96; G-1 to F-95; G-1 to L-94; G-1 to G-93; G-1 to T-92; G-1 to L-91; G-1 to C-90; G-1 to G-89; G-1 to R-88; G-1 to C-87; G-1 to L-86; G-1 to C-85; G-1 to Y-84; G-1 to A-83; G-1 to E-82; G-1 to P-81; G-1 to L-80; G-1 to Y-79; G-1 to R-78; G-1 to P-77; G-1 to Y-76; G-1 to R-75; G-1 to A-74; G-1 to P-73; G-1 to D-72; G-1 to Y-71; G-1 to S-70; G-1 to I-69; G-1 to R-68; G-1 to Y-67; G-1 to A-66; G-1 to W-65; G-1 to P-64; G-1 to S-63; G-1 to V-62; G-1 to S-61; G-1 to R-60; G-1 to L-59; G-1 to N-58; G-1 to T-57; G-1 to P-56; G-1 to P-55; G-1 to R-54; G-1 to F-53; G-1 to R-52; G-1 to R-51; G-1 to D-50; G-1 to A-49; G-1 to P-48; G-1 to R-47; G-1 to G-46; G-1 to G-45; G-1 to A-44; G-1 to P-43; G-1 to C-42; G-1 to S-41; G-1 to A-40; G-1 to N-39; G-1 to R-38; G-1 to A-37; G-1 to Q-36; G-1 to E-35; G-1 to R-34; G-1 to P-33; G-1 to G-32; G-1 to L-31; G-1 to Q-30; G-1 to L-29; G-1 to T-28; G-1 to H-27; G-1 toH-26; G-1 to F-25; G-1 to A-24; G-1 to S-23; G-1 to L-22; G-1 to V-21; G-1 to G-20; G-1 to A-19; G-1 to A-18; G-1 to L-17; G-1 to R-16; G-1 to G-15; G-1 to Y-14; G-1 to L-13; G-1 to Q-12; G-1 to E-11; G-1 to L-10; G-1 to L-9; G-1 to E-8; G-1 to E-7; and G-1 to P-6 of SEQ ID NO:32. Polypeptides encoded by these polynucleotides are also provided. The present application is also directed to nucleic acid molecules comprising, or alternatively, consisting of, a polynucleotide sequence at least 90%, 95%, 96%, 97%, 98% or 99% identical to the polynucleotide sequence encoding the IL-22 polypeptides described above. The present invention also encompasses the above polynucleotide sequences fused to a heterologous polynucleotide sequence.

The invention also provides polypeptides having one or more amino acids deleted from both the amino and the carboxyl termini of IL-22, which may be described generally as having residues n⁶-m⁶ of SEQ ID NO:32, where n⁶ and m⁶ are integers as described above. Polynucleotides encoding these polypeptides are alos provided.

Moreover, any polypeptide having one or more amino acids deleted from both the amino and the carboxyl termini of IL-22, described specifically as having residues n⁶-m⁶ of SEQ ID NO:32 (where n⁶ and m⁶ are integers as described above) may be excluded from the invention, as may polynucleotides encoding such polypeptides.

The invention further includes IL-21 and IL-22 polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided by Bowie and colleagues (*Science* **247:**1306-1310 (1990)), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used (Cunningham and Wells, *Science* **244:**1081-1085 (1989)). The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of an aliphatic or hydrophobic amino acid with another aliphatic or hydrophobic amino acid such as Ala, Val, Leu or Ile; replacement of a hydroxyl residue with another hydroxyl residue such as Ser or Thr; replacement of an acidic residue with another acidic residue such as Asp or Glu; replacement of an amide residue with another amide residue such as Asn or Gln, replacement of a basic residue with another basic residue such as Lys, Arg, or His; replacement of an aromatic residue with another aromatic residue such as Phe, Tyr, or Trp, and replacement of a small-sized amino acid with another small-sized residue such as Ala, Ser, Thr, Met, or Gly.

Besides conservative amino acid substitution, variants of IL-21 and IL-22 include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretory sequence, or a sequence facilitating purification. Such variant polypeptides are deemed to be within the scope of those skilled in the art from the teachings herein.

For example, IL-21 and IL-22 polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity (Pinckard, *et al., Clin. Exp. Immunol*. **2:**331-340 (1967); Robbins, *et al., Diabetes* **36:**838-845 (1987); Cleland, *et al., Crit. Rev. Ther*. *Drug Carrier Systems* **10:**307-377 (1993)).

### Polynucleotide and Polypeptide Fragments

The invention provides nucleic acid molecules having nucleotide sequences related to extensive portions of SEQ ID NO:3 and SEQ ID NO:31 which have been determined from the following related cDNA clones: HE2CD08R (SEQ ID NO:24); HAGBX04R (SEQ ID NO:25); HCEBA24FB (SEQ ID NO:26); and HCELE59R (SEQ ID NO:27). Furthermore, the invention provides nucleic acid molecules having nucleotide sequences related to extensive portions of SEQ ID NO:28 which has been determined from a related cDNA clone designated HTGED19RB (SEQ ID NO:30). Such polynucleotides (i.e., SEQ ID NOs:24, 25, 26, and 30) may preferably be excluded from the present invention.

In the present invention, a "polynucleotide fragment" refers to a short polynucleotide having a nucleic acid sequence contained in the deposited clones or shown in SEQ ID NO:1. SEQ ID NO:3, SEQ ID NO:28 or SEQ ID NO:31. The short nucleotide fragments are preferably at least about 15 nt, and more preferably at least about 20 nt, still more preferably at least about 30 nt, and even more preferably, at least about 40 nt in length. A fragment "at least 20 nt in length," for example, is intended to include 20 or more contiguous bases from the cDNA sequence contained in the deposited clones or the nucleotide sequences shown in SEQ ID NO:1, SEQ ID NO:3, SEQ ID NO:28 or SEQ ID NO:31. These nucleotide fragments are useful as diagnostic probes and primers as discussed herein. Of course, larger fragments (e.g., 50, 150, 500, 600, 2000 nucleotides) are preferred.

Moreover, representative examples of IL-21 polynucleotide fragments include, for example, fragments having a sequence from about nucleotide number 1-50, 51-100, 101-150, 151-200, 201-250, 251-300, 301-350, 351-400, 401-450, 451-500, 501-550, 551-600, 651-700, or 701 to the end of SEQ ID NO:1 or the cDNA contained in the deposited clone. In addition, representative examples of IL-22 polynucleotide fragments include, for example, fragments having a sequence from about nucleotide number 1-50, 51-100, 101-150, 151-200, 201-250, 251-300, 301-350, 351-400, 401-450, 451-500, 501-550, 551-600, 601-650, 651-700, 701-750, 751-800, 801-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1551-1600, or 1601 to the end of SEQ ID NO:3 or the cDNA contained in the deposited clone. Moreover, representative examples of the full-length IL-21 polynucleotide fragments include, for example, fragments having a sequence from about nucleotide number 1-1025, 50-1025, 100-1025, 150-1025, 200-1025, 250-1025, 300-1025, 350-1025, 400-1025, 450-1025, 500-1025, 550-1025, 600-1025, 650-1025, 700-1025, 750-1025, 800-1025, 850-1025, 900-1025, 950-1025, 1000-1025, 1-1000, 50-1000, 100-1000, 150-1000, 200-1000, 250-1000, 300-1000, 350-1000, 400-1000, 450-1000, 500-1000, 550-1000, 600-1000, 650-1000, 700-1000, 750-1000, 800-1000, 850-1000, 900-1000, 950-1000, 1-950, 50-950, 100-950, 150-950, 200-950, 250-950, 300-950, 350-950, 400-950, 450-950, 500-950, 550-950, 600-950, 650-950, 700-950, 750-950, 800-950, 850-950, 900-950, 1-900, 50-900, 100-900, 150-900, 200-900, 250-900, 300-900, 350-900, 400-900, 450-900, 500-900, 550-900, 600-900, 650-900, 700-900, 750-900, 800-900, 850-900, 1-850, 50-850, 100-850, 150-850, 200-850, 250-850, 300-850, 350-850, 400-850, 450-850, 500-850, 550-850, 600-850, 650-850, 700-850, 750-850, 800-850, 1-800, 50-800, 100-800, 150-800, 200-800, 250-800, 300-800, 350-800, 400-800, 450-800, 500-800, 550-800, 600-800, 650-800, 700-800, 750-800, 1-750, 50-750, 100-750, 150-750, 200-750, 250-750, 300-750, 350-750, 400-750, 450-750, 500-750, 550-750, 600-750, 650-750, 700-750, 1-700, 50-700, 100-700, 150-700, 200-700, 250-700, 300-700, 350-700, 400-700, 450-700, 500-700, 550-700, 600-700, 650-700, 1-650, 50-650, 100-650, 150-650, 200-650, 250-650, 300-650, 350-650, 400-650, 450-650, 500-650, 550-650, 600-650, 1-600, 50-600, 100-600, 150-600, 200-600, 250-600, 300-600, 350-600, 400-600, 450-600, 500-600, 550-600, 1-550, 50-550, 100-550, 150-550, 200-550, 250-550, 300-550, 350-550, 400-550, 450-550, 500-550, 1-500, 50-500, 100-500, 150-500, 200-500, 250-500, 300-500, 350-500, 400-500, 450-500, 1-450, 50-450, 100-450, 150-450, 200-450, 250-450, 300-450, 350-450, 400-450, 1-400, 50-400, 100-400, 150-400, 200-400, 250-400, 300-400, 350-400, 1-350, 50-350, 100-350, 150-350, 200-350, 250-350, 300-350, 1-300, 50-300, 100-300, 150-300, 200-300, 250-300, 1-250, 50-250, 100-250, 150-250, 200-250, 1-200, 50-200, 100-200, 150-200, 1-150, 50-150, 100-150, 1-100, 50-100, and 1-50 of SEQ ID NO:28. In this context "about" includes the particularly recited ranges, larger or smaller by several (5, 4, 3, 2, or 1) nucleotides, at either terminus or at both termini. Preferably, these fragments encode a polypeptide which has biological activity.

Further, the invention includes a polynucleotide comprising any portion of at least about 30 nucleotides, preferably at least about 50 nucleotides, of SEQ ID NO:1 from residue 1 to 650, 25 to 650, 50 to 650, 75 to 650, 100 to 650, 125 to 650, 150 to 650, 175 to 650, 200 to 650, 225 to 650, 250 to 650, 275 to 650, 300 to 650, 325 to 650, 350 to 650, 375 to 650, 400 to 650, 425 to 650, 500 to 650, 525 to 650, 550 to 650, 575 to 650, 600 to 650, 625 to 650, 1 to 600, 25 to 600, 50 to 600, 75 to 600, 100 to 600, 125 to 600, 150 to 600, 175 to 600, 200 to 600, 225 to 600, 250 to 600, 275 to 600, 300 to 600, 325 to 600, 350 to 600, 375 to 600, 400 to 600, 425 to 600, 500 to 600, 525 to 600, 550 to 600, 575 to 600, 1 to 550, 25 to 550, 50 to 550, 75 to 550, 100 to 550, 125 to 550, 150 to 550, 175 to 550, 200 to 550, 225 to 550, 250 to 550, 275 to 550, 300 to 550, 325 to 550, 350 to 550, 375 to 550, 400 to 550, 425 to 550, 500 to 550, 525 to 550, 1 to 500, 25 to 500, 50 to 500, 75 to 500, 100 to 500, 125 to 500, 150 to 500, 175 to 500, 200 to 500, 225 to 500, 250 to 500, 275 to 500, 300 to 500, 325 to 500, 350 to 500, 375 to 500, 400 to 500, 425 to 500, 450 to 500, 475 to 500, 1 to 450, 25 to 450, 50 to 450, 75 to 450, 100 to 450, 125 to 450, 150 to 450, 175 to 450, 200 to 450, 225 to 450, 250 to 450, 275 to 450, 300 to 450, 325 to 450, 350 to 450, 375 to 450, 400 to 450, 425 to 450, 1 to 400, 25 to 400, 50 to 400, 75 to 400, 100 to 400, 125 to 400, 150 to 400, 175 to 400, 200 to 400, 225 to 400, 250 to 400, 275 to 400, 300 to 400, 325 to 400, 350 to 400, 375 to 400, 1 to 350, 25 to 350, 50 to 350, 75 to 350, 100 to 350, 125 to 350, 150 to 350, 175 to 350, 200 to 350, 225 to 350, 250 to 350, 275 to 350, 300 to 350, 325 to 350, 1 to 300, 25 to 300, 50 to 300, 75 to 300, 100 to 300, 125 to 300, 150 to 300, 175 to 300, 200 to 300, 225 to 300, 250 to 300, 275 to 300, 1 to 250, 25 to 250, 50 to 250, 75 to 250, 100 to 250, 125 to 250, 150 to 250, 175 to 250, 200 to 250, 225 to 250, 1 to 200, 25 to 200, 50 to 200, 75 to 200, 100 to 200, 125 to 200, 150 to 200, 175 to 200, 1 to 150,25 to 150, 50 to 150, 75 to 150, 100 to 150, 125 to 150, 1 to 100, 25 to 100, 50 to 100, 75 to 100, 1 to 50, and 25 to 50.

Moreover, the invention includes a polynucleotide comprising any portion of at least about 30 nucleotides, preferably at least about 50 nucleotides, of SEQ ID NO:3 from residue 300 to 850. More preferably, the invention includes a polynucleotide comprising nucleotide residues 50 to 850, 75 to 850, 100 to 850, 125 to 850, 150 to 850, 175 to 850, 200 to 850, 225 to 850, 250 to 850, 275 to 850, 300 to 850, 325 to 850, 350 to 850, 375 to 850, 400 to 850, 425 to 850, 450 to 850, 475 to 850, 500 to 850, 525 to 850, 550 to 850, 575 to 850, 600 to 850, 625 to 850, 650 to 850, 675 to 850, 700 to 850, 750 to 850, 775 to 850, 800 to 850, 50 to 800, 75 to 800, 100 to 800, 125 to 800, 150 to 800, 175 to 800, 200 to 800, 225 to 800, 250 to 800, 275 to 800, 300 to 800, 325 to 800, 350 to 800, 375 to 800, 400 to 800, 425 to 800, 450 to 800, 475 to 800, 500 to 800, 525 to 800, 550 to 800, 575 to 800, 600 to 800, 625 to 800, 650 to 800, 675 to 800, 700 to 800, 750 to 800, 50 to 750, 75 to 750, 100 to 750, 125 to 750, 150 to 750, 175 to 750, 200 to 750, 225 to 750, 250 to 750, 275 to 750, 300 to 750, 325 to 750, 350 to 750, 375 to 750, 400 to 750, 425 to 750, 450 to 750, 475 to 750, 500 to 750, 525 to 750, 550 to 750, 575 to 750, 600 to 750, 625 to 750, 650 to 750, 675 to 750, 700 to 750, 50 to 700, 75 to 700, 100 to 700, 125 to 700, 150 to 700, 175 to 700, 200 to 700, 225 to 700, 250 to 700, 275 to 700, 300 to 700, 325 to 700, 350 to 700, 375 to 700, 400 to 700, 425 to 700, 450 to 700, 475 to 700, 500 to 700, 525 to 700, 550 to 700, 575 to 700, 600 to 700, 625 to 700, 650 to 700, 50 to 650, 75 to 650, 100 to 650, 125 to 650, 150 to 650, 175 to 650, 200 to 650, 225 to 650, 250 to 650, 275 to 650, 300 to 650, 325 to 650, 350 to 650, 375 to 650, 400 to 650, 425 to 650, 450 to 650, 475 to 650, 500 to 650, 525 to 650, 550 to 650, 575 to 650, 600 to 650, 50 to 600, 75 to 600, 100 to 600, 125 to 600, 150 to 600, 175 to 600, 200 to 600, 225 to 600, 250 to 600, 275 to 600, 300 to 600, 325 to 600, 350 to 600, 375 to 600, 400 to 600, 425 to 600, 450 to 600, 475 to 600, 500 to 600, 525 to 600, 550 to 600, 50 to 550, 75 to 550, 100 to 550, 125 to 550, 150 to 550, 175 to 550, 200 to 550, 225 to 550, 250 to 550, 275 to 550, 300 to 550, 325 to 550, 350 to 550, 375 to 550, 400 to 550, 425 to 550, 450 to 550, 475 to 550, 500 to 550, 50 to 500, 75 to 500, 100 to 500, 125 to 500, 150 to 500, 175 to 500, 200 to 500, 225 to 500, 250 to 500, 275 to 500, 300 to 500, 325 to 500, 350 to 500, 375 to 500, 400 to 500, 425 to 500, 450 to 500, 50 to 450, 75 to 450, 100 to 450, 125 to 450, 150 to 450, 175 to 450, 200 to 450, 225 to 450, 250 to 450, 275 to 450, 300 to 450, 325 to 450, 350 to 450, 375 to 450, 400 to 450, 50 to 400, 75 to 400, 100 to 400, 125 to 400, 150 to 400, 175 to 400, 200 to 400, 225 to 400, 250 to 400, 275 to 400, 300 to 400, 325 to 400, 350 to 400, 50 to 350, 75 to 350, 100 to 350, 125 to 350, 150 to 350, 175 to 350, 200 to 350, 225 to 350, 250 to 350, 275 to 350, 300 to 350, 50 to 300, 75 to 300, 100 to 300, 125 to 300, 150 to 300, 175 to 300, 200 to 300, 225 to 300, and 250 to 300.

In the present invention, a "polypeptide fragment" refers to a short amino acid sequence contained in SEQ ID NO:2, SEQ ID NO:4, SEQ ID NO:29, SEQ ID NO:32 or encoded by the cDNAs contained in the deposited clones. Protein fragments may be "free-standing," or comprised within a larger polypeptide of which the fragment forms a part or region, most preferably as a single continuous region. Representative examples of polypeptide fragments of the partial IL-21 invention, include, for example, fragments from about amino acid number 1-20, 21-40, 41-60, 61-83 or to the end of the coding region. Moreover, polypeptide fragments of IL-21 can be about 10, 20, 30, 40, 50, 60, 70, or 80 amino acids in length. Representative examples of polypeptide fragments of the IL-22 invention, include, for example, fragments from about amino acid number 1-20, 21-40, 41-60, 61-80, 81-100, 100-120, 120-140, 140-160, or to the end of the coding region. Moreover, polypeptide fragments of IL-22 can be about 10, 20, 30, 40, 50, 60, 70, 80, 100, 120, 140, or 150 amino acids in length. Representative examples of polypeptide fragments of the full-length IL-21 of the invention, include, for example, fragments from about amino acid number 1-20, 21-40, 41-60, 61-80, 81-100, 100-120, 120-140, 140-160, 160-180, 180-200 or 180-to the end of the coding region. Moreover, polypeptide fragments of the full-length IL-21 can be about 10, 20, 30, 40, 50, 60, 70, 80, 100, 120, 140, 150, 160, 170, 180 or 190 amino acids in length. In this context "about" includes the particularly recited ranges, larger or smaller by several (5, 4, 3, 2, or 1) amino acids, at either extreme or at both extremes.

A further embodiment of the invention relates to a peptide or polypeptide which comprises the amino acid sequence of an IL-21 or IL-22 polypeptide having an amino acid sequence which contains at least one conservative amino acid substitution, but not more than 50 conservative amino acid substitutions, even more preferably, not more than 40 conservative amino acid substitutions, still more preferably, not more than 30 conservative amino acid substitutions, and still even more preferably, not more than 20 conservative amino acid substitutions. Of course, in order of ever-increasing preference, it is highly preferable for a peptide or polypeptide to have an amino acid sequence which comprises the amino acid sequence of an IL-21 or IL-22 polypeptide, which contains at least one, but not more than 10, 9, 8, 7, 6, 5, 4, 3, 2 or 1 conservative amino acid substitutions.

Preferred polypeptide fragments include the secreted IL-21 and IL-22 proteins as well as the mature forms. Further preferred polypeptide fragments include the secreted IL-21 and IL-22 proteins or the mature forms having a continuous series of deleted residues from the amino or the carboxy terminus, or both. For example, any number of amino acids, ranging from 1-60, can be deleted from the amino terminus of either the secreted or the mature form of the IL-21 and IL-22 polypeptides. Similarly, any number of amino acids, ranging from 1-30, can be deleted from the carboxy terminus of the secreted or the mature form of the IL-21 and IL-22 polypeptides. Furthermore, any combination of the above amino and carboxy terminus deletions are preferred. Similarly, polynucleotide fragments encoding these IL-21 and IL-22 polypeptide fragments are also preferred.

Also preferred are IL-21 and IL-22 polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:2, SEQ ID NO:4, SEQ ID NO:29 or SEQ ID NO:32 falling within conserved domains are specifically contemplated by the present invention (Figures 4, 5, 7, and 9). Moreover, polynucleotide fragments encoding these domains are also contemplated.

In additional embodiments, the polynucleotides of the invention encode functional attributes of IL-21 or IL-22. Preferred embodiments of the invention in this regard include fragments that comprise alpha-helix and alpha-helix forming regions ("alpha-regions"), beta-sheet and beta-sheet forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of IL-21 or IL-22.

The data representing the structural or functional attributes of IL-21 set forth in Figure 7 and/or Table I, as described above, was generated using the various modules and algorithms of the DNA*STAR set on default parameters. The data representing the structural or functional attributes of IL-22 set forth in Figure 5 and/or Table II, in Figure 9 and/or Table III, as described above, was generated using the various modules and algorithms of the DNA*STAR set on default parameters. In a preferred embodiment, the data presented in columns VIII, IX, XIII, and XIV of Table I can be used to determine regions of IL-21 which exhibit a high degree of potential for antigenicity. In an additional preferred embodiment, the data presented in columns VIII, IX, XIII, and XIV of Tables II and/or III can be used to determine regions of IL-22 which exhibit a high degree of potential for antigenicity. Regions of high antigenicity are determined from the data presented in columns VIII, IX, XIII, and/or TV by choosing values which represent regions of the polypeptide which are likely to be exposed on the surface of the polypeptide in an environment in which antigen recognition may occur in the process of initiation of an immune response.

Certain preferred regions in these regards are set out in Figure 7, but may, as shown in Tables I, be represented or identified by using tabular representations of the data presented in Figure 7. The DNA*STAR computer algorithm used to generate Figure 7 (set on the original default parameters) was used to present the data in Figure 7 in a tabular format (*See* Table I). The tabular format of the data in Figure 7 may be used to easily determine specific boundaries of a preferred region.

Certain preferred regions in these regards are set out in Figures 5 and 8, but may, as shown in Tables II and III, respectively, be represented or identified by using tabular representations of the data presented in Figures 5 and 8, respectively. The DNA*STAR computer algorithm used to generate Figures 5 and 8 (set on the original default parameters) was used to present the data in Figures 5 and 8 in a tabular format (See Tables II and III, respectively). The tabular format of the data in Figures 5 and 8 may be used to easily determine specific boundaries of a preferred region.

The above-mentioned preferred regions set out in Figures 5, 7, and 9, and in Tables II, I, and III, respectively, include, but are not limited to, regions of the aforementioned types identified by analysis of the amino acid sequence set out in Figures 2A-B, 6A-B, and 8, respectively. As set out in Figure 7 and in Table I, and in Figure 5 and Table II, and in Figure 8 and Table III, such preferred regions include Gamier-Robson alpha-regions, beta-regions, turn-regions, and coil-regions, Chou-Fasman alpha-regions, beta-regions, and coil-regions, Kyte-Doolittle hydrophilic regions and hydrophobic regions, Eisenberg alpha- and beta-amphipathic regions, Karplus-Schulz flexible regions, Emini surface-forming regions and Jameson-Wolf regions of high antigenic index.

**Table I**

| Res | Position | I | II | III | IV | V | VI | VII | VIII | IX | X | XI | XII | XIII | XIV |
|---|---|---|---|---|---|---|---|---|---|---|---|---|---|---|---|
| Met | 1 | A | . | . | . | . | . | . | -0.80 | 0.76 | . | . | . | -0.40 | 0.36 |
| Thr | 2 | . | . | B | . | . | . | . | -0.76 | 0.76 | . | . | . | -0.40 | 0.44 |
| Leu | 3 | A | . | . | . | . | . | . | -1.18 | 0.76 | . | . | . | -0.40 | 0.34 |
| Leu | 4 | A | . | . | . | . | T | . | -1.60 | 1.01 | . | . | . | -0.20 | 0.28 |
| Pro | 5 | A | . | . | . | . | T | . | -1.91 | 1.09 | . | . | F | -0.05 | 0.16 |
| Gly | 6 | A | . | . | . | . | T | . | -2.12 | 1.39 | . | . | . | -0.20 | 0.17 |
| Leu | 7 | A | . | . | . | . | T | . | -2.12 | 1.39 | . | . | . | -0.20 | 0.17 |
| Leu | 8 | A | A | . | . | . | . | . | -1.60 | 1.19 | . | . | . | -0.60 | 0.16 |
| Phe | 9 | A | A | . | . | . | . | . | -1.60 | 1.67 | . | . | . | -0.60 | 0.17 |
| Leu | 10 | A | A | . | . | . | . | . | -1.42 | 1.93 | . | . | . | -0.60 | 0.17 |
| Thr | 11 | A | A | . | . | . | . | . | -1.39 | 1.74 | . | . | . | -0.60 | 0.28 |
| Trp | 12 | A | A | . | . | . | . | . | -1.24 | 1.54 | . | . | . | -0.60 | 0.46 |
| Leu | 13 | A | A | . | . | . | . | . | -1.24 | 1.33 | . | . | . | -0.60 | 0.30 |
| His | 14 | A | A | . | . | . | . | . | -1.13 | 1.33 | * | . | . | -0.60 | 0.17 |
| Thr | 15 | A | A | . | . | . | . | . | -0.36 | 1.34 | . | . | . | -0.60 | 0.17 |
| Cys | 16 | A | A | . | . | . | . | . | -0.08 0 | 0.93 | . | . | . | -0.60 | 0.27 |
| Leu | 17 | A | A | . | . | . | . | . | 0.21 | 0.74 | . | . | . | -0.60 | 0.27 |
| Ala | 18 | . | A | . | . | T | . | . | 0.81 | 0.24 | . | . | . | 0.10 | 0.32 |
| His | 19 | . | A | . | . | T | . | . | 0.54 | 0.19 | . | . | . | 0.44 | 0.91 |
| His | 20 | . | A | . | . | . | . | C | 0.04 | -0.00 | . | * | . | 1.33 | 1.48 |
| Asp | 21 | . | . | . | . | . | T | C | 0.82 | -0.00 | . | * | F | 2.22 | 1.21 |
| Pro | 22 | . | . | . | . | T | T | . | 1.29 | -0.50 | . | * | F | 2.76 | 1.74 |
| Ser | 23 | . | . | . | . | T | T | . | 1.84 | -0.57 | . | * | F | 3.40 | 1.27 |
| Leu | 24 | . | . | . | . | T | T | . | 1.67 | -0.57 | . | * | F | 3.06 | 1.03 |
| Arg | 25 | . | . | . | . | T | . | . | 1.67 | -0.14 | * | * | F | 2.35 | 1.03 |
| Gly | 26 | . | . | . | . | T | . | . | 1.37 | -0.07 | * | * | F | 2.14 | 1.05 |
| His | 27 | . | . | . | . | . | T | C | 1.54 | -0.07 | . | * | . | 1.78 | 1.70 |
| Pro | 28 | . | . | . | . | . | T | C | 1.50 | -0.26 | . | * | . | 1.57 | 1.18 |
| His | 29 | . | . | . | . | T | T | . | 2.00 | 0.17 | . | * | . | 1.30 | 1.18 |
| Ser | 30 | . | . | . | . | T | T | . | 1.68 | 0.23 | . | * | . | 1.17 | 1.26 |
| His | 31 | . | . | . | . | T | . | . | 1.99 | 0.16 | . | . | . | 0.84 | 1.26 |
| Gly | 32 | . | . | . | . | T | . | . | 1.36 | 0.23 | . | . | F | 0.86 | 1.26 |
| Thr | 33 | . | . | . | . | . | T | C | 1.32 | 0.30 | . | . | F | 0.58 | 0.50 |
| Pro | 34 | . | . | . | . | . | T | C | 1.06 | 0.67 | . | . | F | 0.15 | 0.58 |
| His | 35 | . | . | . | . | T | T | . | 0.77 | 0.56 | . | . | . | 0.20 | 0.78 |
| Cys | 36 | . | . | . | . | T | T | . | 0.80 | 0.63 | . | . | | 0.20 | 0.55 |
| Tyr | 37 | . | A | . | . | T | . | C | 1.14 | 0.14 | . | . | . | 0.10 | 0.61 |
| Ser | 38 | . | A | . | . | . | . | C | 0.64 | -0.29 | . | . | . | 0.50 | 0.78 |
| Ala | 39 | A | A | . | . | . | . | . | 0.64 | -0.10 | . | . | . | 0.45 | 1.20 |
| Glu | 40 | A | A | . | . | . | . | . | -0.13 | -0.24 | . | . | F | 0.60 | 1.19 |
| Glu | 41 | A | A | . | . | . | . | . | 0.19 | -0.31 | . | . | . | 0.30 | 0.73 |
| Leu | 42 | A | . | . | | . | T | . | 0.43 | -0.27 | . | . | . | 0.70 | 0.72 |
| Pro | 43 | A | . | . | . | . | T | . | 0.14 | -0.37 | . | . | . | 0.70 | 0.72 |
| Leu | 44 | . | . | . | . | T | T | . | 0.52 | 0.13 | . | . | . | 0.50 | 0.42 |
| Gly | 45 | . | . | . | . | T | T | . | 0.31 | 0.56 | . | . | F | 0.35 | 0.78 |
| Gln | 46 | A | . | . | . | . | | . | 0.28 | 0.30 | . | . | F | 0.05 | 0.78 |
| Ala | 47 | . | . | . | . | . | . | C | 0.28 | 0.37 | * | . | F | 0.40 | 1.29 |
| Pro | 48 | . | . | . | . | . | T | C | -0.32 | 0.37 | * | . | F | 0.60 | 1.08 |
| Pro | 49 | A | . | . | . | . | T | . | -0.10 | 0.63 | * | * | F | -0.05 | 0.51 |
| His | 50 | A | . | . | . | . | T | . | 0.36 | 0.73 | * | * | . | -0.20 | 0.51 |
| Leu | 51 | A | . | . | . | . | T | . | 0.01 | 0.23 | * | * | . | 0.10 | 0.65 |
| Leu | 52 | A | A | . | . | . | . | . | 0.01 | 0.23 | * | * | . | -0.30 | 0.42 |
| Ala | 53 | A | A | . | . | . | . | . | 0.27 | 0.30 | * | . | . | -0.30 | 0.31 |
| Arg | 54 | A | A | . | . | . | . | . | 0.19 | -0.20 | * | . | . | 0.30 | 0.75 |
| Gly | 55 | A | A | . | . | . | . | . | -0.12 | 0.03 | * | . | F | -0.15 | 0.95 |
| Ala | 56 | A | A | . | . | . | . | . | 0.69 | -0.23 | * | . | F | 0.45 | 0.93 |
| Lys | 57 | . | A | . | . | T | . | . | 0.91 | -0.33 | * | . | F | 0.85 | 0.83 |
| Trp | 58 | . | A | . | . | T | . | . | 0.69 | 0.17 | * | . | F | 0.25 | 0.84 |
| Gly | 59 | . | A | . | . | . | . | C | 0.37 | 0.43 | * | . | F | -0.25 | 0.69 |
| Gln | 60 | A | A | . | . | . | . | . | -0.14 | 0.36 | * | . | . | -0.30 | 0.53 |
| Ala | 61 | . | A | . | . | . | . | C | -0.14 | 1.00 | * | . | . | -0.40 | 0.38 |
| Leu | 62 | . | A | B | . | . | . | . | -1.00 | 0.59 | * | . | . | -0.60 | 0.38 |
| Pro | 63 | . | A | B | . | . | . | . | -1.57 | 0.84 | . | . | . | -0.60 | 0.18 |
| Val | 64 | A | A | . | . | . | . | . | -1.52 | 1.09 | . | . | . | -0.60 | 0.13 |
| Ala | 65 | A | A | . | . | . | . | . | -1.82 | 0.97 | . | . | . | -0.60 | 0.22 |
| Leu | 66 | A | A | . | . | . | . | . | -2.04 | 0.67 | . | . | . | -0.60 | 0.19 |
| Val | 67 | A | A | . | . | . | . | . | -1.23 | 0.93 | . | . | . | -0.60 | 0.21 |
| Ser | 68 | A | A | . | . | . | . | . | -1.61 | 0.29 | . | . | . | -0.30 | 0.36 |
| Ser | 69 | A | A | . | . | . | . | . | -1.34 | 0.29 | . | . | . | -0.30 | 0.44 |
| Leu | 70 | A | A | . | . | . | . | . | -1.06 | 0.10 | * | . | . | -0.30 | 0.60 |
| Glu | 71 | A | A | . | . | . | . | . | -0.28 | -0.16 | * | . | . | 0.30 | 0.60 |
| Ala | 72 | A | A | . | . | . | . | . | 0.69 | -0.04 | * | . | . | 0.30 | 0.61 |
| Ala | 73 | A | A | . | . | . | . | . | 0.64 | -0.43 | * | * | . | 0.79 | 1.45 |
| Ser | 74 | A | . | . | . | . | . | . | 1.06 | -0.69 | * | * | . | 1.48 | 0.83 |
| His | 75 | A | . | . | . | . | T | . | 1.83 | -0.69 | * | * | . | 2.17 | 1.60 |
| Arg | 76 | A | . | . | . | . | T | . | 1.83 | -0.69 | . | * | F | 2.66 | 2.16 |
| Gly | 77 | . | . | . | . | T | T | . | 2.53 | -1.19 | . | * | F | 3.40 | 2.79 |
| Arg | 78 | . | . | . | . | T | T | . | 2.91 | -1.57 | . | * | F | 3.06 | 4.02 |
| His | 79 | . | . | . | . | . | . | C | 2.91 | -1.64 | . | * | F | 2.66 | 3.17 |
| Glu | 80 | . | . | . | . | . | . | C | 2.36 | -1.26 | . | * | F | 2.66 | 4.29 |
| Arg | 81 | . | . | . | . | . | T | C | 1.93 | -1.19 | . | * | F | 2.86 | 2.21 |
| Pro | 82 | . | . | . | . | T | T | . | 1.97 | -0.70 | . | . | F | 3.06 | 2.35 |
| Ser | 83 | . | . | . | . | T | T | . | 1.86 | -0.71 | . | * | F | 3.40 | 1.96 |
| Ala | 84 | . | . | . | . | T | T | . | 1.22 | -0.31 | . | * | F | 2.76 | 1.73 |
| Thr | 85 | . | . | . | B | T | . | . | 1.01 | 0.26 | . | * | F | 1.27 | 0.60 |
| Thr | 86 | . | . | . | B | T | . | . | 0.04 | 0.26 | . | * | F | 0.93 | 0.69 |
| Gln | 87 | . | . | . | B | T | . | . | -0.56 | 0.51 | . | . | F | 0.29 | 0.51 |
| Cys | 88 | . | . | B | B | . | . | . | -0.14 | 0.70 | * | . | . | -0.60 | 0.29 |
| Pro | 89 | . | . | B | B | . | . | . | 0.23 | 0.21 | . | * | . | -0.30 | 0.39 |
| Val | 90 | . | . | . | B | . | . | C | 0.54 | 0.16 | . | . | . | -0.10 | 0.35 |
| Leu | 91 | . | A | . | . | . | . | C | 0.86 | -0.24 | . | . | . | 0.65 | 1.14 |
| Arg | 92 | . | A | . | . | . | . | C | 0.00 | -0.81 | * | . | F | 1.10 | 1.27 |
| Pro | 93 | A | A | . | . | . | . | . | -0.14 | -0.60 | * | * | F | 0.90 | 1.27 |
| Glu | 94 | A | A | . | . | . | . | . | 0.07 | -0.56 | * | * | F | 0.90 | 1.27 |
| Glu | 95 | A | A | . | . | . | . | . | 0.33 | -1.24 | * | * | F | 0.90 | 1.13 |
| Val | 96 | A | A | . | . | . | . | . | 1.14 | -0.74 | . | * | . | 0.60 | 0.74 |
| Leu | 97 | A | A | . | . | . | . | . | 0.72 | -1.17 | * | * | . | 0.60 | 0.71 |
| Glu | 98 | A | A | . | . | . | . | . | 0.90 | -0.69 | . | . | . | 0.60 | 0.59 |
| Ala | 99 | A | A | . | . | . | . | . | 0.90 | -0.19 | . | * | F | 0.60 | 1.08 |
| Asp | 100 | A | . | . | . | . | T | . | 1.01 | -0.43 | . | * | F | 1.00 | 2.28 |
| Thr | 101 | A | . | . | . | . | T | . | 1.57 | -1.11 | * | * | F | 1.30 | 2.58 |
| His | 102 | A | . | . | . | . | T | . | 1.49 | -0.73 | * | * | F | 1.30 | 3.42 |
| Gln | 103 | . | . | . | . | T | T | . | 1.19 | -0.54 | * | . | F | 1.91 | 1.43 |
| Arg | 104 | . | . | . | B | T | . | . | 1.57 | -0.16 | * | . | F | 1.42 | 1.33 |
| Ser | 105 | . | . | . | B | T | . | . | 1.28 | -0.21 | * | * | F | 1.63 | 1.51 |
| Ile | 106 | . | . | . | B | . | . | C | 1.70 | 0.20 | * | * | F | 0.89 | 0.92 |
| Ser | 107 | . | . | . | . | . | T | C | 1.49 | -0.20 | * | * | F | 2.10 | 0.92 |
| Pro | 108 | . | . | . | . | T | T | . | 1.60 | 0.56 | * | * | F | 1.34 | 1.07 |
| Trp | 109 | . | . | . | . | T | T | . | 0.63 | 0.17 | * | * | . | 1.28 | 3.00 |
| Arg | 110 | . | . | . | . | . | T | C | 0.93 | 0.13 | . | * | . | 0.87 | 1.66 |
| Tyr | 111 | . | . | . | B | T | . | . | 1.51 | -0.26 | . | * | . | 1.40 | 1.80 |
| Arg | 112 | . | . | . | B | T | . | . | 1.81 | -0.20 | . | * | . | 1.53 | 2.46 |
| Val | 113 | . | . | . | B | . | . | C | 2.02 | -1.11 | . | * | . | 1.97 | 2.10 |
| Asp | 114 | . | . | . | . | T | T | . | 2.31 | -1.11 | . | * | F | 3.06 | 2.32 |
| Thr | 115 | . | . | . | . | T | T | . | 2.31 | -1.87 | . | * | F | 3.40 | 1.98 |
| Asp | 116 | . | . | . | . | T | T | . | 2.31 | -1.87 | * | * | F | 3.06 | 5.23 |
| Glu | 117 | . | . | . | . | T | T | . | 1.99 | -1.76 | * | * | F | 2.72 | 4.90 |
| Asp | 118 | . | . | . | . | T | T | . | 2.84 | -1.33 | * | . | F | 2.38 | 5.25 |
| Arg | 119 | A | . | . | . | . | T | . | 2.89 | -1.41 | * | * | F | 1.64 | 5.45 |
| Tyr | 120 | A | . | . | . | . | T | . | 2.39 | -1.41 | * | . | F | 1.30 | 6.29 |
| Pro | 121 | A | . | . | . | . | T | . | 1.80 | -0.73 | * | * | F | 1.30 | 3.11 |
| Gln | 122 | A | A | . | . | . | . | | 1.10 | -0.23 | | * | F | 0.60 | 1.60 |
| Lys | 123 | A | A | . | . | . | . | | 0.51 | 0.56 | * | * | F | -0.45 | 0.89 |
| Leu | 124 | A | A | . | . | . | . | . | 0.40 | 0.30 | * | * | . | -0.30 | 0.58 |
| Ala | 125 | A | A | . | . | . | . | . | -0.02 | -0.13 | . | . | . | 0.30 | 0.58 |
| Phe | 126 | A | A | . | . | . | . | . | -0.62 | 0.04 | . | . | . | -0.30 | 0.16 |
| Ala | 127 | A | A | . | . | . | . | . | -1.29 | 0.73 | * | . | . | -0.60 | 0.16 |
| Glu | 128 | A | A | . | . | . | . | . | -1.22 | 0.61 | * | * | . | -0.60 | 0.08 |
| Cys | 129 | A | A | . | . | . | . | . | -0.76 | 0.11 | * | . | . | -0.30 | 0.19 |
| Leu | 130 | A | A | . | . | . | . | . | -0.83 | -0.24 | * | * | . | 0.30 | 0.18 |
| Cys | 131 | . | . | . | . | T | T | . | -1.02 | -0.17 | * | * | . | 1.10 | 0.06 |
| Arg | 132 | . | . | . | . | T | T | . | -0.43 | 0.51 | * | * | . | 0.20 | 0.07 |
| Gly | 133 | . | . | . | . | T | T | . | -1.02 | -0.06 | * | * | . | 1.10 | 0.15 |
| Cys | 134 | . | . | . | . | T | T | . | -0.24 | -0.24 | * | * | . | 1.40 | 0.28 |
| Ile | 135 | A | . | . | . | . | . | . | 0.26 | -0.81 | * | * | . | 1.40 | 0.28 |
| Asp | 136 | . | . | . | . | T | . | . | 0.58 | -0.33 | . | * | . | 1.80 | 0.41 |
| Ala | 137 | . | . | . | . | T | . | . | 0.58 | -0.33 | . | * | . | 2.10 | 0.76 |
| Arg | 138 | . | . | . | . | . | T | C | 0.92 | -0.90 | * | * | F | 3.00 | 2.12 |
| Thr | 139 | . | . | . | . | . | T | C | 1.28 | -1.59 | * | * | F | 2.70 | 2.20 |
| Gly | 140 | . | . | . | . | . | T | C | 1.58 | -1.10 | * | * | F | 2.40 | 3.14 |
| Arg | 141 | A | . | . | . | . | T | . | 0.99 | -1.10 | * | . | F | 1.90 | 1.62 |
| Glu | 142 | A | A | . | . | . | . | . | 0.77 | -0.60 | * | * | F | 1.20 | 1.13 |
| Thr | 143 | A | A | . | . | . | . | . | 0.66 | -0.40 | * | . | F | 0.45 | 0.94 |
| Ala | 144 | A | A | . | . | . | . | . | 0.67 | -0.43 | . | * | . | 0.30 | 0.78 |
| Ala | 145 | A | A | . | . | . | . | . | 0.16 | -0.04 | . | * | . | 0.30 | 0.60 |
| Leu | 146 | A | . | . | B | . | . | . | 0.16 | 0.60 | . | * | . | -0.60 | 0.31 |
| Asn | 147 | A | . | . | B | . | . | . | -0.66 | 0.11 | * | . | . | -0.30 | 0.60 |
| Ser | 148 | A | . | . | B | . | . | . | -1.16 | 0.30 | * | . | . | -0.30 | 0.49 |
| Val | 149 | A | . | . | B | . | . | . | -0.57 | 0.49 | * | . | . | -0.60 | 0.49 |
| Arg | 150 | A | . | . | B | . | . | . | -0.28 | 0.20 | * | . | . | -0.30 | 0.53 |
| Leu | 151 | A | . | . | B | . | . | . | -0.28 | 0.19 | * | . | . | -0.30 | 0.53 |
| Leu | 152 | A | . | . | B | . | . | . | -1.09 | 0.49 | * | * | . | -0.60 | 0.58 |
| Gln | 153 | A | . | . | B | . | . | . | -1.64 | 0.53 | * | * | . | -0.60 | 0.25 |
| Ser | 154 | A | . | . | B | . | . | . | -1.60 | 1.17 | . | * | . | -0.60 | 0.22 |
| Leu | 155 | . | . | B | B | . | . | . | -1.60 | 1.17 | * | . | . | -0.60 | 0.22 |
| Leu | 156 | . | . | B | B | . | . | . | -0.68 | 0.49 | * | * | . | -0.60 | 0.25 |
| Val | 157 | . | . | B | B | . | . | . | 0.24 | 0.09 | * | * | . | -0.30 | 0.37 |
| Leu | 158 | . | . | B | B | . | . | . | 0.03 | -0.30 | * | . | . | 0.30 | 0.87 |
| Arg | 159 | . | . | . | B | T | . | . | -0.33 | -0.56 | . | . | F | 1.30 | 1.63 |
| Arg | 160 | . | . | . | B | T | . | . | 0.18 | -0.67 | . | * | F | 1.30 | 1.18 |
| Arg | 161 | . | . | . | B | . | . | C | 1.10 | -0.93 | . | * | F | 1.10 | 1.91 |
| Pro | 162 | . | . | . | . | T | . | . | 1.96 | -1.61 | . | * | F | 1.84 | 1.91 |
| Cys | 163 | . | . | . | . | T | . | . | 2.42 | -1.61 | . | * | F | 2.18 | 1.63 |
| Ser | 164 | . | . | . | . | T | T | . | 2.01 | -1.19 | . | * | F | 2.57 | 0.82 |
| Arg | 165 | . | . | . | . | T | T | . | 1.56 | -0.80 | * | . | F | 2.91 | 0.71 |
| Asp | 166 | . | . | . | . | T | T | . | 0.63 | -0.80 | * | * | F | 3.40 | 1.32 |
| Gly | 167 | . | . | . | . | T | T | . | 0.63 | -0.69 | * | . | F | 2.91 | 0.81 |
| Ser | 168 | . | . | . | . | T | . | . | 0.99 | -0.64 | * | . | F | 2.37 | 0.64 |
| Gly | 169 | . | . | . | . | . | . | C | 1.08 | -0.16 | * | . | F | 1.53 | 0.55 |
| Leu | 170 | . | . | . | . | . | . | C | 0.62 | 0.27 | * | . | F | 0.59 | 0.87 |
| Pro | 171 | . | . | . | . | . | . | C | 0.03 | 0.27 | . | . | F | 0.25 | 0.64 |
| Thr | 172 | . | . | . | . | . | T | C | -0.32 | 0.39 | . | . | F | 0.45 | 0.65 |
| Pro | 173 | . | . | . | . | . | T | C | -0.61 | 0.74 | . | . | F | 0.15 | 0.68 |
| Gly | 174 | . | . | . | . | . | T | C | -0.97 | 0.56 | . | . | F | 0.15 | 0.45 |
| Ala | 175 | A | . | . | . | . | T | . | -0.19 | 0.91 | . | . | . | -0.20 | 0.27 |
| Phe | 176 | A | A | . | . | . | . | . | -0.29 | 0.93 | . | . | . | -0.60 | 0.24 |
| Ala | 177 | A | A | . | . | . | . | . | 0.02 | 0.99 | . | * | . | -0.60 | 0.34 |
| Phe | 178 | A | A | . | . | . | . | . | -0.47 | 0.56 | . | . | . | -0.60 | 0.59 |
| His | 179 | A | A | . | . | . | . | . | -1.01 | 0.84 | . | . | . | -0.60 | 0.59 |
| Thr | 180 | A | . | . | B | . | . | . | -0.46 | 0.74 | . | . | . | -0.60 | 0.41 |
| Glu | 181 | A | . | . | B | . | . | . | -0.61 | 0.74 | . | . | . | -0.60 | 0.64 |
| Phe | 182 | A | . | . | B | . | . | . | -0.23 | 0.60 | . | . | . | -0.60 | 0.35 |
| Ile | 183 | . | . | . | B | T | . | . | -0.39 | 0.53 | . | . | . | -0.20 | 0.38 |
| His | 184 | . | . | . | B | T | . | . | -0.70 | 0.69 | . | . | . | -0.20 | 0.16 |
| Val | 185 | . | . | . | B | . | . | C | -1.06 | 1.11 | . | . | . | -0.40 | 0.18 |
| Pro | 186 | . | . | . | . | T | T | . | -1.37 | 0.90 | . | . | . | 0.20 | 0.14 |
| Val | 187 | . | . | . | . | T | T | . | -1.33 | 0.70 | . | . | . | 0.20 | 0.15 |
| Gly | 188 | . | . | . | . | T | T | . | -1.30 | 0.77 | . | * | . | 0.20 | 0.11 |
| Cys | 189 | . | . | . | . | T | T | . | -2.08 | 0.77 | . | . | . | 0.20 | 0.05 |
| Thr | 190 | . | . | B | B | . | . | . | -1.43 | 1.03 | . | * | . | -0.60 | 0.06 |
| Cys | 191 | . | . | B | B | . | . | . | -1.11 | 0.81 | . | . | . | -0.60 | 0.09 |
| Val | 192 | . | . | B | B | . | . | . | -0.56 | 0.39 | * | . | . | -0.30 | 0.33 |
| Leu | 193 | . | . | B | . | . | T | . | -1.07 | 0.20 | * | . | . | 0.28 | 0.31 |
| Pro | 194 | . | . | B | . | . | T | . | -0.79 | 0.36 | * | . | F | 0.61 | 0.42 |
| Arg | 195 | . | . | . | . | T | T | . | -0.87 | 0.21 | * | . | . | 1.04 | 0.73 |
| Ser | 196 | . | . | . | . | T | T | . | -0.59 | -0.00 | * | . | . | 1.97 | 1.13 |
| Val | 197 | . | . | . | . | T | . | . | -0.12 | -0.26 | * | . | . | 1.80 | 0.93 |

**Table II**

| Res | Position | I | II | III | IV | V | VI | VII | VIII | IX | X | XI | XII | XIII |
|---|---|---|---|---|---|---|---|---|---|---|---|---|---|---|
| Asn | 1 | . | . | . | . | . | . | C | 0.58 | . | * | . | 0.85 | 1.60 |
| Ser | 2 | . | A | . | . | . | . | C | 1.08 | . | * | . | 0.65 | 1.26 |
| Ala | 3 | . | A | B | . | . | . | . | 0.88 | . | * | . | 0.75 | 1.93 |
| Arg | 4 | . | A | B | . | . | . | . | 0.41 | . | * | . | 0.75 | 1.22 |
| Ala | 5 | . | A | B | . | . | . | . | -0.01 | . | * | . | 0.30 | 0.67 |
| Arg | 6 | . | A | B | . | . | . | . | -0.31 | . | * | . | 0.30 | 0.55 |
| Ala | 7 | . | A | B | . | . | . | . | -0.60 | . | * | . | 0.30 | 0.38 |
| Val | 8 | . | A | B | . | . | . | . | -0.71 | . | * | . | -0.30 | 0.38 |
| Leu | 9 | . | A | B | . | . | . | . | -0.86 | * | * | . | -0.60 | 0.17 |
| Ser | 10 | . | A | B | . | . | . | . | -0.30 | * | * | . | -0.60 | 0.22 |
| Ala | 11 | . | A | B | . | . | . | . | -0.72 | * | . | . | -0.60 | 0.41 |
| Phe | 12 | . | A | B | . | . | . | . | -0.94 | * | . | . | -0.60 | 0.72 |
| His | 13 | . | A | B | . | . | . | . | -0.09 | * | . | . | -0.60 | 0.44 |
| His | 14 | . | A | B | . | . | . | . | -0.09 | * | . | . | -0.60 | 0.76 |
| Thr | 15 | . | A | B | . | . | . | . | -0.13 | * | . | . | -0.60 | 0.72 |
| Leu | 16 | . | A | . | . | . | . | C | 0.24 | * | * | . | -0.10 | 0.52 |
| Gln | 17 | . | A | . | . | T | . | . | 1.06 | * | * | . | 0.40 | 0.60 |
| Leu | 18 | . | A | . | . | . | . | C | 1.09 | . | * | . | 0.80 | 0.81 |
| Gly | 19 | . | . | . | . | . | T | C | 1.12 | . | * | F | 2.40 | 1.70 |
| Pro | 20 | . | . | . | . | . | T | C | 0.84 | * | * | F | 3.00 | 1.70 |
| Arg | 21 | . | . | . | . | . | T | C | 1.77 | * | * | F | 2.70 | 2.08 |
| Glu | 22 | . | . | B | . | . | T | . | 1.77 | * | * | F | 2.20 | 4.12 |
| Gln | 23 | . | . | B | . | . | . | . | 1.99 | * | * | F | 1.70 | 4.28 |
| Ala | 24 | . | . | . | . | T | . | . | 2.03 | * | * | F | 1.80 | 2.21 |
| Arg | 25 | . | . | . | . | T | . | . | 1.58 | * | * | F | 1.50 | 1.71 |
| Asn | 26 | . | . | . | . | T | . | . | 1.26 | * | * | F | 1.05 | 0.53 |
| Ala | 27 | . | . | . | . | T | . | . | 0.67 | * | . | . | 0.90 | 0.81 |
| Ser | 28 | . | . | B | . | . | . | . | 0.32 | . | . | . | 0.78 | 0.42 |
| Cys | 29 | . | . | B | . | . | T | . | 0.57 | . | * | . | 0.66 | 0.26 |
| Pro | 30 | . | . | . | . | T | T | . | 0.57 | . | * | . | 1.34 | 0.25 |
| Ala | 31 | . | . | . | . | T | T | . | 0.36 | . | * | F | 2.37 | 0.37 |
| Gly | 32 | . | . | . | . | T | T | . | 0.36 | . | . | F | 2.80 | 1.06 |
| Gly | 33 | . | . | . | . | . | . | C | 0.66 | * | * | F | 1.97 | 0.69 |
| Arg | 34 | . | . | B | . | . | . | . | 1.43 | * | . | F | 1.94 | 1.15 |
| Pro | 35 | . | . | B | . | . | T | . | 1.76 | * | . | F | 1.86 | 2.27 |
| Ala | 36 | . | . | B | . | . | T | . | 1.64 | * | * | F | 1.58 | 4.49 |
| Asp | 37 | . | . | B | . | . | T | . | 2.10 | * | * | F | 1.30 | 1.99 |
| Arg | 38 | . | . | B | . | . | T | . | 2.23 | * | * | F | 1.30 | 2.52 |
| Arg | 39 | . | . | B | . | . | . | . | 1.91 | * | * | F | 1.10 | 3.85 |
| Phe | 40 | . | . | B | . | . | . | . | 1.81 | * | * | F | 1.44 | 3.57 |
| Arg | 41 | . | . | B | . | . | . | . | 2.40 | * | * | F | 1.78 | 2.63 |
| Pro | 42 | . | . | . | . | . | T | C | 1.59 | . | * | F | 2.22 | 2.16 |
| Pro | 43 | . | . | . | . | T | T | . | 1.59 | . | * | F | 2.16 | 2.05 |
| Thr | 44 | . | . | . | . | T | T | . | 1.18 | . | * | F | 3.40 | 2.05 |
| Asn | 45 | . | . | . | . | . | T | C | 1.02 | * | * | F | 2.56 | 1.78 |
| Leu | 46 | . | . | B | B | . | . | . | 0.61 | * | * | F | 0.87 | 0.85 |
| Arg | 47 | . | . | B | B | . | . | . | 0.61 | * | . | F | 1.13 | 0.79 |
| Ser | 48 | . | . | B | B | . | . | . | 0.53 | * | . | F | 0.79 | 0.76 |
| Val | 49 | . | . | B | B | . | . | . | 0.26 | * | . | F | -0.45 | 0.97 |
| Ser | 50 | . | . | B | . | . | T | . | 0.01 | * | * | F | 0.25 | 0.50 |
| Pro | 51 | . | . | B | . | . | T | . | 0.93 | * | * | . | -0.20 | 0.59 |
| Trp | 52 | . | . | B | . | . | T | . | -0.07 | * | * | . | -0.05 | 1.55 |
| Ala | 53 | . | . | B | . | . | T | . | -0.07 | * | * | . | -0.20 | 0.81 |
| Tyr | 54 | . | . | B | B | . | . | . | 0.54 | * | * | . | -0.60 | 0.70 |
| Arg | 55 | . | . | B | B | . | . | . | 0.84 | . | * | . | -0.45 | 1.05 |
| Ile | 56 | . | . | B | B | . | . | . | 0.84 | * | * | . | 0.13 | 1.73 |
| Ser | 57 | . | . | B | . | . | . | . | 0.54 | * | * | . | 0.61 | 1.71 |
| Tyr | 58 | . | . | . | . | T | . | . | 1.24 | * | * | . | 1.74 | 0.88 |
| Asp | 59 | . | . | . | . | . | T | C | 1.24 | * | * | F | 2.32 | 2.46 |
| Pro | 60 | . | . | . | . | T | T | . | 0.92 | * | . | F | 2.80 | 2.88 |
| Ala | 61 | . | . | . | . | T | T | . | 1.92 | * | . | F | 2.52 | 2.84 |
| Arg | 62 | . | . | B | . | . | T | . | 1.98 | * | . | F | 2.14 | 3.33 |
| Tyr | 63 | . | . | B | . | . | T | . | 1.41 | * | . | . | 1.41 | 3.37 |
| Pro | 64 | . | . | B | . | . | T | . | 1.20 | * | . | . | 0.53 | 2.75 |
| Arg | 65 | . | . | . | . | T | T | . | 1.41 | * | . | . | 0.65 | 2.17 |
| Tyr | 66 | . | . | B | . | . | T | . | 1.41 | * | . | F | 0.40 | 2.40 |
| Leu | 67 | . | . | B | . | . | . | . | 1.06 | * | . | F | 0.80 | 1.57 |
| Pro | 68 | . | . | B | . | . | . | . | 0.63 | * | . | . | 0.05 | 1.26 |
| Glu | 69 | . | . | . | . | T | . | . | 0.03 | * | . | . | 0.00 | 0.43 |
| Ala | 70 | . | . | B | B | . | . | . | -0.74 | * | . | . | -0.60 | 0.43 |
| Tyr | 71 | . | . | B | B | . | . | . | -0.39 | * | . | . | -0.60 | 0.15 |
| Cys | 72 | . | . | B | B | . | . | . | 0.08 | * | . | . | -0.30 | 0.17 |
| Leu | 73 | . | . | B | B | . | . | . | -0.38 | . | * | . | -0.60 | 0.16 |
| Cys | 74 | . | . | B | . | . | T | . | -1.19 | . | * | . | -0.20 | 0.06 |
| Arg | 75 | . | . | B | . | . | T | . | -0.91 | * | * | . | -0.20 | 0.09 |
| Gly | 76 | . | . | B | . | . | T | . | -1.01 | * | . | . | -0.20 | 0.15 |
| Cys | 77 | . | . | B | . | . | T | . | -1.16 | . | * | . | 0.10 | 0.28 |
| Leu | 78 | . | . | B | B | . | . | . | -1.04 | . | . | . | -0.30 | 0.12 |
| Thr | 79 | . | . | B | B | . | . | . | -0.72 | . | * | . | -0.60 | 0.10 |
| Gly | 80 | . | . | . | B | . | . | C | -0.83 | . | * | . | -0.40 | 0.19 |
| Leu | 81 | . | . | . | B | . | . | C | -0.49 | . | . | . | -0.40 | 0.40 |
| Phe | 82 | . | . | B | B | . | . | . | 0.18 | . | . | F | 0.45 | 0.48 |
| Gly | 83 | . | . | . | B | . | . | C | 0.13 | . | * | F | 0.95 | 0.81 |
| Glu | 84 | . | A | B | . | . | . | . | 0.56 | . | * | F | 0.45 | 0.73 |
| Glu | 85 | . | A | B | . | . | . | . | 0.20 | . | * | F | 0.90 | 1.65 |
| Asp | 86 | . | A | B | B | . | . | . | 1.12 | . | * | F | 0.90 | 1.45 |
| Val | 87 | . | A | B | B | . | . | . | 1.52 | . | * | F | 0.90 | 1.63 |
| Arg | 88 | . | A | . | B | T | . | . | 1.28 | . | * | . | 1.15 | 1.26 |
| Phe | 89 | . | A | . | B | T | . | . | 1.07 | . | * | . | 1.00 | 0.77 |
| Arg | 90 | . | A | . | B | T | . | . | 0.21 | . | * | . | 0.85 | 1.59 |
| Ser | 91 | . | A | . | B | . | . | C | -0.03 | . | * | . | 0.50 | 0.60 |
| Ala | 92 | . | . | . | B | . | . | C | 0.22 | . | * | . | 0.25 | 1.09 |
| Pro | 93 | . | . | . | B | . | . | C | -0.10 | . | * | . | -0.10 | 0.55 |
| Val | 94 | . | . | . | B | T | . | . | 0.29 | * | . | . | -0.20 | 0.64 |
| Tyr | 95 | . | . | B | B | . | . | . | -0.68 | * | . | . | -0.60 | 0.91 |
| Met | 96 | . | . | B | B | . | . | . | -1.23 | . | . | . | -0.60 | 0.44 |
| Pro | 97 | . | . | B | B | . | . | . | -1.46 | . | * | . | -0.60 | 0.44 |
| Thr | 98 | . | . | B | B | . | . | . | -1.13 | * | . | . | -0.60 | 0.23 |
| Val | 99 | . | . | B | B | . | . | . | -0.17 | * | . | . | -0.60 | 0.46 |
| Val | 100 | . | . | B | B | . | . | . | -0.23 | . | . | . | 0.30 | 0.58 |
| Leu | 101 | . | . | B | B | . | . | . | 0.16 | . | . | . | 0.30 | 0.58 |
| Arg | 102 | . | . | B | B | . | . | . | -0.22 | . | . | F | 0.60 | 1.20 |
| Arg | 103 | . | . | B | B | . | . | . | -0.58 | . | . | F | 0.60 | 1.63 |
| Thr | 104 | . | . | B | B | . | . | . | -0.31 | . | . | F | 0.60 | 1.06 |
| Pro | 105 | . | . | B | B | . | . | . | 0.20 | * | . | F | 1.00 | 0.55 |
| Ala | 106 | . | . | B | . | . | . | . | 0.67 | . | * | . | 1.00 | 0.28 |
| Cys | 107 | . | . | B | . | . | T | . | 0.67 | . | . | . | 0.85 | 0.19 |
| Ala | 108 | . | . | . | . | T | T | . | 0.26 | * | * | . | 2.10 | 0.24 |
| Gly | 109 | . | . | . | . | T | T | . | -0.29 | * | . | F | 2.50 | 0.32 |
| Gly | 110 | . | . | . | . | T | T | . | -0.32 | * | . | F | 2.25 | 0.44 |
| Arg | 111 | . | . | B | B | . | . | . | -0.04 | * | . | F | 0.60 | 0.69 |
| Ser | 112 | . | . | B | B | . | . | . | 0.62 | * | . | F | 0.35 | 1.00 |
| Val | 113 | . | . | B | B | . | . | . | 0.62 | * | . | . | 0.70 | 1.75 |
| Tyr | 114 | . | . | B | . | . | . | . | 0.72 | . | . | . | 0.50 | 0.90 |
| Thr | 115 | . | . | B | . | . | . | . | 0.21 | . | . | . | -0.25 | 1.05 |
| Glu | 116 | . | . | B | B | . | . | . | -0.21 | . | * | . | -0.45 | 1.05 |
| Ala | 117 | . | . | B | B | . | . | . | -0.80 | . | * | . | -0.60 | 0.97 |
| Tyr | 118 | . | . | B | B | . | . | . | -0.16 | . | * | . | -0.60 | 0.47 |
| Val | 119 | . | . | B | B | . | . | . | -0.77 | . | * | . | -0.60 | 0.42 |
| Thr | 120 | . | . | B | B | . | . | . | -0.80 | . | * | . | -0.60 | 0.31 |
| Ile | 121 | . | . | B | B | . | . | . | -1.47 | . | * | . | -0.60 | 0.20 |
| Pro | 122 | . | . | B | . | . | T | . | -1.19 | . | * | . | -0.20 | 0.14 |
| Val | 123 | . | . | . | . | T | T | . | -1.61 | . | . | . | 0.20 | 0.14 |
| Gly | 124 | . | . | . | . | T | T | . | -1.61 | . | . | . | 0.20 | 0.11 |
| Cys | 125 | . | . | B | . | . | T | . | -1.51 | . | . | . | -0.20 | 0.05 |
| Thr | 126 | . | . | B | . | . | . | . | -0.62 | . | . | . | -0.40 | 0.11 |
| Cys | 127 | . | . | B | . | . | . | . | -0.62 | . | . | . | -0.10 | 0.19 |
| Val | 128 | . | . | B | . | . | T | . | 0.23 | . | . | . | 0.40 | 0.55 |
| Pro | 129 | . | . | B | . | . | T . | . | 0.62 | . | . | F | 1.45 | 0.65 |
| Glu | 130 | . | . | B | . | . | T | . | 1.29 | * | . | F | 2.20 | 2.44 |
| Pro | 131 | . | . | B | . | . | T | . | 1.01 | * | . | F | 2.50 | 5.49 |
| Glu | 132 | . | . | . | . | T | . | . | 1.68 | * | . | F | 3.00 | 3.59 |
| Lys | 133 | A | . | . | . | . | . | . | 2.23 | * | . | F | 2.30 | 3.46 |
| Asp | 134 | A | . | . | . | . | T | . | 1.56 | * | . | F | 2.20 | 3.00 |
| Ala | 135 | A | . | . | . | . | T | . | 1.56 | * | . | F | 1.90 | 1.21 |
| Asp | 136 | A | . | . | . | . | T | . | 1.47 | * | . | F | 1.45 | 0.98 |
| Ser | 137 | . | . | B | . | . | T | . | 1.17 | * | . | F | 1.15 | 0.78 |
| Ile | 138 | . | . | B | . | . | . | . | 0.23 | * | . | F | 0.80 | 1.04 |
| Asn | 139 | . | . | B | . | . | T | . | 0.23 | * | . | F | 0.85 | 0.44 |
| Ser | 140 | . | . | B | . | . | T . | . | 0.87 | * | . | F | 1.16 | 0.54 |
| Ser | 141 | . | . | B | . | . | T | . | 0.87 | * | . | F | 1.62 | 1.55 |
| Ile | 142 | . | . | B | . | . | T | . | 0.82 | . | * | F | 2.23 | 1.67 |
| Asp | 143 | . | . | B | . | . | T | . | 1.12 | * | * | F | 2.54 | 1.23 |
| Lys | 144 | . | . | . | . | T | T | . | 1.17 | * | . | F | 3.10 | 0.93 |
| Gln | 145 | . | . | B | . | . | T | . | 0.66 | * | . | F | 2.54 | 2.65 |
| Gly | 146 | . | . | B | . | . | T | . | 0.14 | * | . | F | 2.23 | 1.31 |
| Ala | 147 | . | A | B | . | . | . | . | 0.22 | * | . | F | 1.07 | 0.54 |
| Lys | 148 | . | A | B | . | . | . | . | -0.12 | . | . | F | 0.16 | 0.26 |
| Leu | 149 | . | A | B | . | . | . | . | -0.38 | * | . | . | -0.60 | 0.26 |
| Leu | 150 | . | A | B | . | . | . | . | -0.38 | . | . | . | -0.60 | 0.39 |
| Leu | 151 | . | A | B | . | . | . | . | -0.03 | . | . | . | -0.06 | 0.32 |
| Gly | 152 | . | . | B | . | . | T | . | -0.03 | . | . | F | 0.73 | 0.64 |
| Pro | 153 | . | . | . | | . | T | C | -0.29 | . | . | F | 1.17 | 0.78 |
| Asn | 154 | . | . | . | | T | T | . | -0.07 | . | . | F | 2.36 | 1.47 |
| Asp | 155 | . | . | . | | . | T | C | 0.40 | . | . | F | 2.40 | 1.50 |
| Ala | 156 | . | . | . | | . | . | C | 1.00 | . | . | F | 1.81 | 0.96 |
| Pro | 157 | . | . | . | | . | T | C | 0.96 | . | . | F | 1.77 | 0.92 |
| Ala | 158 | . | . | . | | . | T | C | 0.78 | . | . | . | 1.38 | 0.71 |
| Gly | 159 | . | . | . | | . | T | C | 0.39 | . | . | . | 0.54 | 0.90 |
| Pro | 160 | . | . | B | | . | T | . | 0.00 | . | . | . | 0.10 | 0.74 |

**Table III**

| Res | Position | I | II | III | IV | V | VI | VII | VIII | IX | X | XI | XII | XII |
|---|---|---|---|---|---|---|---|---|---|---|---|---|---|---|
| Gly | 1 | . | A | . | . | T | . | . | 0.46 | -0.21 . | . | . | 0.70 | 0.34 |
| Cys | 2 | . | A | . | . | T | . | . | 0.63 | -0.64 . | . | . | 1.00 | 0.51 |
| Ala | 3 | . | A | . | . | . | . | C | 1.02 | -0.64 . | . | . | 0.80 | 0.62 |
| Asp | 4 | . | A | . | . | . | . | C | 1.41 | -1.07 . | . | . | 0.95 | 1.09 |
| Arg | 5 | A | A | . | . | . | . | . | 0.99 | -1.50 * | . | F | 0.90 | 3.51 |
| Pro | 6 | A | A | . | . | . | . | . | 0.52 | -1.39 * | . | F | 0.90 | 2.87 |
| Glu | 7 | A | A | . | . | . | . | . | 1.19 | -1.20 * | . | F | 0.90 | 1.42 |
| Glu | 8 | A | A | . | . | . | . | . | 1.78 | -1.20 * | . | F | 0.90 | 1.25 |
| Leu | 9 | A | A | . | . | . | . | . | 0.97 | -0.80 * | . | F | 0.90 | 1.40 |
| Leu | 10 | A | A | . | . | . | . | . | 0.61 | -0.54 * | . | F | 0.75 | 0.67 |
| Glu | 11 | A | A | . | . | . | . | . | 0.48 | 0.21 * | * | . | -0.30 | 0.60 |
| Gln | 12 | A | A | . | B | . | . | . | 0.59 | 0.64 * | * | . | -0.60 | 0.73 |
| Leu | 13 | A | A | . | B | . | . | . | -0.22 | -0.04 * | * | . | 0.45 | 1.72 |
| Tyr | 14 | A | A | . | B | . | . | . | -0.00 | -0.04 * | * | . | 0.30 | 0.82 |
| Gly | 15 | A | A | . | . | . | . | . | 0.22 | 0.46 * | * | . | -0.60 | 0.48 |
| Arg | 16 | A | A | . | . | . | . | . | -0.12 | 0.56 * | * | . | -0.60 | 0.59 |
| Leu | 17 | A | A | . | . | . | . | . | -0.98 | 0.30 * | * | . | -0.30 | 0.37 |
| Ala | 18 | A | A | . | B | . | . | . | -0.98 | 0.19 * | * | . | -0.30 | 0.28 |
| Ala | 19 | A | A | . | B | . | . | . | -1.03 | 0.44 * | * | . | -0.60 | 0.12 |
| Gly | 20 | A | A | . | B | . | . | . | -1.28 | 0.83 * | * | . | -0.60 | 0.19 |
| Val | 21 | A | A | . | B | . | . | . | -2.09 | 0.64 * | . | . | -0.60 | 0.19 |
| Leu | 22 | A | A | . | B | . | . | . | -1.31 | 0.93 . | . | . | -0.60 | 0.16 |
| Ser | 23 | A | A | . | . | . | . | . | -0.76 | 0.93 . | . | . | -0.60 | 0.22 |
| Ala | 24 | A | A | . | . | . | . | . | -0.48 | 1.00 . | . | . | -0.60 | 0.41 |
| Phe | 25 | A | A | . | B | . | . | . | -0.94 | 0.84 * | . | . | -0.60 | 0.72 |
| His | 26 | A | A | . | B | . | . | . | -0.09 | 0.84 * | * | . | -0.60 | 0.44 |
| His | 27 | . | A | B | B | . | . | . | -0.09 | 0.86 * | . | . | -0.60 | 0.76 |
| Thr | 28 | . | A | B | B | . | . | . | -0.13 | 1.04 . | . | . | -0.60 | 0.72 |
| Leu | 29 | . | A | . | B | . | . | C | 0.24 | 0.69 * | * | . | -0.10 | 0.52 |
| Gln | 30 | . | A | . | B | T | . | . | 1.06 | 0.61 * | * | . | 0.40 | 0.60 |
| Leu | 31 | . | A | . | B | . | . | C | 1.09 | 0.11 . | * | . | 0.80 | 0.81 |
| Gly | 32 | . | . | . | . | . | T | C | 1.12 | -0.37. | * | F | 2.40 | 1.70 |
| Pro | 33 | . | . | . | . | . | T | C | 0.84 | -0.66 * | * | F | 3.00 | 1.70 |
| Arg | 34 | . | . | . | . | . | T | C | 1.77 | -0.56 * | * | F | 2.70 | 2.08 |
| Glu | 35 | A | . | . | . | . | T | . | 1.77 | -1.24 * | * | F | 2.20 | 4.12 |
| Gln | 36 | A | . | . | . | . | . | . | 1.99 | -1.27 * | * | F | 1.70 | 4.28 |
| Ala | 37 | . | . | . | . | T | . | . | 2.03 | -1.20 * | * | F | 1.80 | 2.21 |
| Arg | 38 | . | . | . | . | T | . | . | 1.58 | -0.81 * | * | F | 1.50 | 1.71 |
| Asn | 39 | . | . | . | . | T | . | . | 1.26 | -0.24 * | * | F | 1.05 | 0.53 |
| Ala | 40 | . | . | . | . | T | . | . | 0.67 | -0.21 * | . | . | 0.90 | 0.81 |
| Ser | 41 | . | . | B | . | . | . | . | 0.32 | -0.21 . | . | . | 0.78 | 0.42 |
| Cys | 42 | . | . | B | . | . | T | . | 0.57 | 0.21 . | * | . | 0.66 | 0.26 |
| Pro | 43 | . | . | . | . | T | T | . | 0.57 | 0.24 . | * | . | 1.34 | 0.25 |
| Ala | 44 | . | . | . | . | T | T | . | 0.36 | -0.26 . | * | F | 2.37 | 0.37 |
| Gly | 45 | . | . | . | . | T | T | . | 0.36 | -0.21 . | . | F | 2.80 | 1.06 |
| Gly | 46 | . | . | . | . | . | . | C | 0.66 | -0.29 * | * | F | 1.97 | 0.69 |
| Arg | 47 | . | . | B | . | . | . | . | 1.43 | -0.71 * | . | F | 1.94 | 1.15 |
| Pro | 48 | . | . | B | . | . | T | . | 1.76 | -1.21 * | . | F | 1.86 | 2.27 |
| Ala | 49 | . | . | B | . | . | T | . | 1.64 | -1.64 * | * | F | 1.58 | 4.49 |
| Asp | 50 | . | . | B | . | . | T | . | 2.10 | -1.29 * | * | F | 1.30 | 1.99 |
| Arg | 51 | . | . | B | . | . | T | . | 2.23 | -1.29 * | * | F | 1.30 | 2.52 |
| Arg | 52 | . | . | B | . | . | | . | 1.91 | -1.29 * | * | F | 1.10 | 3.85 |
| Phe | 53 | . | . | B | . | . | . | . | 1.81 | -1.36 * | * | F | 1.44 | 3.57 |
| Arg | 54 | . | . | B | . | . | . | . | 2.40 | -0.87 * | * | F | 1.78 | 2.63 |
| Pro | 55 | . | . | . | . | . | T | C | 1.59 | -0.47 . | * | F | 2.22 | 2.16 |
| Pro | 56 | . | . | . | . | T | T | . | 1.59 | 0.21 . | * | F | 2.16 | 2.05 |
| Thr | 57 | . | . | . | . | T | T | . | 1.18 | -0.57 . | * | F | 3.40 | 2.05 |
| Asn | 58 | . | . | . | . | . | T | C | 1.02 | -0.19 * | * | F | 2.56 | 1.78 |
| Leu | 59 | . | . | B | B | . | . | . | 0.61 | 0.03 * | * | F | 0.87 | 0.85 |
| Arg | 60 | . | . | B | B | . | . | . | 0.61 | -0.01 * | * | F | 1.13 | 0.79 |
| Ser | 61 | . | . | B | B | . | . | . | 0.53 | -0.07 * | . | F | 0.79 | 0.76 |
| Val | 62 | . | . | B | B | . | . | . | 0.26 | 0.44 * | . | F | -0.45 | 0.97 |
| Ser | 63 | . | . | B | . | . | T | . | 0.01 | 0.26 * | * | F | 0.25 | 0.50 |
| Pro | 64 | . | . | B | . | . | T | . | 0.93 | 1.01 * | * | . | -0.20 | 0.59 |
| Trp | 65 | . | . | B | . | . | T | . . | -0.07 | 0.63 * | * | . | -0.05 | 1.55 |
| Ala | 66 | . | . | B | . | . | T | . | -0.07 | 0.67 * | * | . | -0.20 | 0.81 |
| Tyr | 67 | . | . | B | B | . | . | . | 0.54 | 0.67 * | * | . | -0.60 | 0.70 |
| Arg | 68 | . | . | B | B | . | . | . | 0.84 | 1.00 . | * | . | -0.45 | 1.05 |
| Ile | 69 | . | . | B | B | . | . | . | 0.84 | 0.09 * | * | . | 0.13 | 1.73 |
| Ser | 70 | . | . | B | . | . | . | . | 0.54 | 0.01 * | * | . | 0.61 | 1.71 |
| Tyr | 71 | . | . | . | . | T | . | . | 1.24 | -0.24 * | * | . | 1.74 | 0.88 |
| Asp | 72 | . | . | . | . | . | T | C | 1.24 | -0.24 * | * | F | 2.32 | 2.46 |
| Pro | 73 | . | . | . | . | T | T | . | 0.92 | -0.17 * | * | F | 2.80 | 2.88 |
| Ala | 74 | . | . | . | . | T | T | . | 1.92 | -0.13 * | . | F | 2.52 | 2.84 |
| Arg | 75 | . | . | B | . | . | T | . | 1.98 | -0.89 * | . | F | 2.14 | 3.33 |
| Tyr | 76 | . | . | B | . | . | T | . | 1.41 | -0.13 * | . | . | 1.41 | 3.37 |
| Pro | 77 | . | . | B | . | . | T | . | 1.20 | 0.13 * | . | . | 0.53 | 2.75 |
| Arg | 78 | . | . | . | . | T | T | . | 1.41 | 0.06 * | . | . | 0.65 | 2.17 |
| Tyr | 79 | .< | . | B | . | . | T | . | 1.41 | 0.06 * | . | F | 0.40 | 2.40 |
| Leu | 80 | . | . | B | . | . | . | . | 1.06 | -0.20 * | . | F | 0.80 | 1.57 |
| Pro | 81 | . | . | B | . | . | . | . | 0.63 | 0.13 * | . | . | 0.05 | 1.26 |
| Glu | 82 | . | . | . | . | T | . | . | 0.03 | 0.70 * | . | . | 0.00 | 0.43 |
| Ala | 83 | . | . | B | B | . | . | . | -0.74 | 0.63 * | . | . | -0.60 | 0.43 |
| Tyr | 84 | . | . | B | B | . | . | . | -0.39 | 0.51 . | . | . | -0.60 | 0.15 |
| Cys | 85 | . | . | B | B | . | . | . | 0.08 | 0.09 * | . | . | -0.30 | 0.17 |
| Leu | 86 | . | . | B | B | . | . | . | -0.38 | 0.51 . | * | . | -0.60 | 0.16 |
| Cys | 87 | . | . | B | . | . | T | . | -1.19 | 0.59 . | * | . | -0.20 | 0.06 |
| Arg | 88 | . | . | B | . | . | T | . | -0.91 | 0.51 * | * | . | -0.20 | 0.09 |
| Gly | 89 | . | . | B | . | . | T | . | -1.01 | 0.43 * | . | . | -0.20 | 0.15 |
| Cys | 90 | . | . | B | . | . | T | . | -1.16 | 0.17 . | * | . | 0.10 | 0.28 |
| Leu | 91 | . | . | B | B | . | . | . | -1.04 | 0.29 . | . | . | -0.30 | 0.12 |
| Thr | 92 | . | . | B | B | . | . | . | -0.72 | 1.07 . | * | . | -0.60 | 0.10 |
| Gly | 93 | . | . | . | B | . | . | C | -0.83 | 1.07 . | * | . | -0.40 | 0.19 |
| Leu | 94 | . | . | . | B | . | . | C | -0.49 | 0.50 . | . | . | -0.40 | 0.40 |
| Phe | 95 | . | . | B | B | . | . | . | 0.18 | -0.19 . | . | F | 0.45 | 0.48 |
| Gly | 96 | A | . | . | B | . | . | . | 0.13 | -0.67 . | * | F | 0.75 | 0.81 |
| Glu | 97 | A | A | . | . | . | . | . | 0.56 | -0.46 . | * | F | 0.45 | 0.73 |
| Glu | 98 | A | A | . | . | . | . | . | 0.20 | -1.14 . | * | F | 0.90 | 1.65 |
| Asp | 99 | A | A | . | B | . | . | . | 1.12 | -1.14 . | * | F | 0.90 | 1.45 |
| Val | 100 | A | A | . | B | . | . | . | 1.52 | -1.57 . | * | F | 0.90 | 1.63 |
| Arg | 101 | A | A | . | B | . | . | . | 1.28 | -1.19 . | * | . | 0.75 | 1.26 |
| Phe | 102 | A | A | . | B | . | . | . | 1.07 | -0.69 . | * | . | 0.60 | 0.77 |
| Arg | 103 | A | A | . | B | . | . | . | 0.21 | -0.26 . | * | . | 0.45 | 1.59 |
| Ser | 104 | . | A | . | B | . | . | C | -0.03 | -0.26 . | * | . | 0.50 | 0.60 |
| Ala | 105 | . | . | . | B | . | . | C | 0.22 | 0.50 . | * | . | -0.25 | 1.09 |
| Pro | 106 | . | . | . | B | . | . | C | -0.10 | 0.33 . | * | . | -0.10 | 0.55 |
| Val | 107 | . | . | . | B | T | . | . | 0.29 | 0.76 * | . | . | -0.20 | 0.64 |
| Tyr | 108 | . | . | B | B | . | . | . | -0.68 | 0.86 * | . | . | -0.60 | 0.91 |
| Met | 109 | . | . | B | B | . | . | . | -1.23 | 1.00 . | . | . | -0.60 | 0.44 |
| Pro | 110 | . | . | B | B | . | . | . | -1.46 | 1.21 . | * | . | -0.60 | 0.44 |
| Thr | 111 | . | . | B | B | . | . | . | -1.13 | 1.26 * | . | . | -0.60 | 0.23 |
| Val | 112 | . | . | B | B | . | . | . | -0.17 | 0.50 * | . | . | -0.60 | 0.46 |
| Val | 113 | . | . | B | B | . | . | . | -0.23 | -0.11 . | . | . | 0.30 | 0.58 |
| Leu | 114 | . | . | B | B | . | . | . | 0.16 | -0.06 . | . | . | 0.30 | 0.58 |
| Arg | 115 | . | . | B | B | . | . | . | -0.22 | -0.11 . | . | F | 0.60 | 1.20 |
| Arg | 116 | . | . | B | B | . | . | . | -0.58 | -0.26 . | . | F | 0.60 | 1.63 |
| Thr | 117 | . | . | B | B | . | . | . | -0.31 | -0.33 . | . | F | 0.60 | 1.06 |
| Pro | 118 | . | . | B | B | . | . | . | 0.20 | -0.51 * | . | F | 1.00 | 0.55 |
| Ala | 119 | . | . | B | . | . | . | . | 0.67 | -0.09 . | * | . | 1.00 | 0.28 |
| Cys | 120 | . | . | B | . | . | T | . | 0.67 | 0.34 . | * | . | 0.85 | 0.19 |
| Ala | 121 | . | . | . | . | T | T | . | 0.26 | -0.14 * | * | . | 2.10 | 0.24 |
| Gly | 122 | . | . | . | . | T | T | . | -0.29 | -0.19 * | . | F | 2.50 | 0.32 |
| Gly | 123 | . | . | . | . | T | T | . | -0.32 | -0.04 * | . | F | 2.25 | 0.44 |
| Arg | 124 | . | . | B | B | . | . | . | -0.04 | 0.14 . | . | F | 0.60 | 0.69 |
| Ser | 125 | . | . | B | B | . | . | . | 0.62 | 0.13 . | . | F | 0.35 | 1.00 |
| Val | 126 | . | . | B | B | . | . | . | 0.62 | -0.30 . | . | . | 0.70 | 1.75 |
| Tyr | 127 | . | . | B | . | . | . | . | 0.72 | -0.23 . | . | . | 0.50 | 0.90 |
| Thr | 128 | . | . | B | . | . | . | . | 0.21 | 0.53 . | . | . | -0.25 | 1.05 |
| Glu | 129 | . | . | B | B | . | . | . | -0.21 | 0.79 . | * | . | -0.45 | 1.05 |
| Ala | 130 | . | . | B | B | . | . | . | -0.80 | 0.63 . | * | . | -0.60 | 0.97 |
| Tyr | 131 | . | . | B | B | . | . | . | -0.16 | 0.56 . | * | . | -0.60 | 0.47 |
| Val | 132 | . | . | B | B | . | . | . | -0.77 | 0.50 . | * | . | -0.60 | 0.42 |
| Thr | 133 | . | . | B | B | . | . | . | -0.80 | 1.14 . | * | . | -0.60 | 0.31 |
| Ile | 134 | . | . | B | B | . | . | . | -1.47 | 1.07 . | * | . | -0.60 | 0.20 |
| Pro | 135 | . | . | B | . | . | T | . | -1.19 | 0.89 . | * | . | -0.20 | 0.14 |
| Val | 136 | . | . | . | . | T | T | . | -1.61 | 0.73 . | . | . | 0.20 | 0.14 |
| Gly | 137 | . | . | . | . | T | T | . | -1.61 | 0.81 . | . | . | 0.20 | 0.11 |
| Cys | 138 | . | . | B | . | . | T | . | -1.51 | 0.77 . | . | . | -0.20 | 0.05 |
| Thr | 139 | . | . | B | . | . | . | . | -0.62 | 0.77 . | . | . | -0.40 | 0.11 |
| Cys | 140 | . | . | B | . | . | . | . | -0.62 | 0.13 . | . | . | -0.10 | 0.19 |
| Val | 141 | . | . | B | . | . | T | . | 0.23 | 0.13 . | . | . | 0.10 | 0.55 |
| Pro | 142 | . | . | B | . | . | T | . | 0.62 | -0.44 . | . | F | 0.85 | 0.65 |
| Glu | 143 | . | . | B | . | . | T | . | 1.29 | -0.93 . | . | F | 1.30 | 2.44 |
| Pro | 144 | A | . | . | . | . | T | . | 1.01 | -1.50 * | . | F | 1.30 | 5.49 |
| Glu | 145 | A | . | . | . | . | . | . | 1.68 | -1.64 * | . | F | 1.10 | 3.59 |
| Lys | 146 | A | . | . | . | . | . | . | 2.23 | -2.07 * | . | F | 1.10 | 3.46 |
| Asp | 147 | A | . | . | . | . | T | . | 1.56 | -1.69 . | . | F | 1.30 | 3.00 |
| Ala | 148 | A | . | . | . | . | T | . | 1.56 | -1.43 * | . | F | 1.30 | 1.21 |
| Asp | 149 | A | . | . | . | . | T | . | 1.47 | -1.03 * | . | F | 1.15 | 0.98 |
| Ser | 150 | A | . | . | . | . | T | . | 1.17 | -0.64 * | . | F | 1.15 | 0.78 |
| Ile | 151 | A | . | . | . | . | . | . | 0.23 | -0.26 * | * | F | 0.80 | 1.04 |
| Asn | 152 | . | . | B | . | . | T | . | 0.23 | -0.07 * | . | F | 0.85 | 0.44 |
| Ser | 153 | . | . | B | . | . | T | . | 0.87 | -0.07 * | . | F | 0.85 | 0.54 |
| Ser | 154 | . | . | B | . | . | T | . | 0.87 | -0.46 * | * | F | 1.00 | 1.55 |
| Ile | 155 | A | . | . | . | . | T | . | 0.82 | -0.74 . | * | F | 1.30 | 1.67 |
| Asp | 156 | A | . | . | . | . | T | . | 1.12 | -0.71 * | * | F | 1.30 | 1.23 |
| Lys | 157 | A | . | . | . | . | T | . | 1.17 | -0.60 * | . | F | 1.15 | 0.93 |
| Gln | 158 | A | . | . | . | . | T | . | 0.66 | -0.99 * | . | F | 1.30 | 2.65 |
| Gly | 159 | . | . | B | . | . | T | . | 0.14 | -0.99 * | . | F | 1.30 | 1.31 |
| Ala | 160 | . | A | B | . | . | . | . | 0.22 | -0.30 * | . | F | 0.45 | 0.54 |
| Lys | 161 | . | A | B | . | . | . | . | -0.12 | 0.39 . | . | F | -0.15 | 0.26 |
| Leu | 162 | . | A | B | . | . | . | . | -0.38 | 0.41 . | . | . | -0.60 | 0.26 |
| Leu | 163 | . | A | B | . | . | . | . | -0.38 | 0.41 . | . | . | -0.60 | 0.39 |
| Leu | 164 | . | A | B | . | . | . | . | -0.03 | 0.31 . | . | . | -0.06 | 0.32 |
| Gly | 165 | . | . | B | . | . | T | . | -0.03 | 0.31 . | . | F | 0.73 | 0.64 |
| Pro | 166 | . | . | . | . | . | T | C | -0.29 | 0.13 . | . | F | 1.17 | 0.78 |
| Asn | 167 | . | . | . | . | T | T | . | -0.07 | -0.13 . | . | F | 2.36 | 1.47 |
| Asp | 168 | . | . | . | . | . | T | C | 0.40 | -0.31 . | . | F | 2.40 | 1.50 |
| Ala | 169 | . | . | . | . | . | . | C | 1.00 | -0.31 . | . | F | 1.81 | 0.96 |
| Pro | 170 | . | . | . | . | . | T | C | 0.96 | -0.31 . | . | F | 1.77 | 0.92 |
| Ala | 171 | . | . | . | . | . | T | C | 0.78 | -0.29 . | . | . | 1.38 | 0.71 |
| Gly | 172 | . | . | . | . | . | T | C | 0.39 | 0.14 . | . | . | 0.54 | 0.90 |
| Pro | 173 | . | . | B | . | . | T | . | 0.00 | 0.07 . | . | . | 0.10 | 0.74 |

Among highly preferred fragments in this regard are those that comprise reigons of IL-21 or IL-22 that combine several structural features, such as several of the features set out above.

Other preferred fragments are biologically active IL-21 and IL-22 fragments. Biologically active fragments are those exhibiting activity similar, but not necessarily identical, to an activity of the IL-21 and IL-22 polypeptides. The biological activity of the fragments may include an improved desired activity, or a decreased undesirable activity.

### Transgenics and "knock-outs"

The polypeptides of the invention can also be expressed in transgenic animals. Animals of any species, including, but not limited to, mice, rats, rabbits, hamsters, guinea pigs, pigs, micro-pigs, goats, sheep, cows and non-human primates, e.g., baboons, monkeys, and chimpanzees may be used to generate transgenic animals. In a specific embodiment, techniques described herein or otherwise known in the art, are used to express polypeptides of the invention in humans, as part of a gene therapy protocol.

Any technique known in the art may be used to introduce the transgene (i.e., polynucleotides of the invention) into animals to produce the founder lines of transgenic animals. Such techniques include, but are not limited to, pronuclear microinjection (Paterson et al., Appl. Microbiol. Biotechnol. 40:691-698 (1994); Carver et al., Biotechnology (NY) 11:1263-1270 (1993); Wright et al., Biotechnology (NY) 9:830-834 (1991); and Hoppe et al., U.S. Pat. No. 4,873,191 (1989)); retrovirus mediated gene transfer into germ lines (Van der Putten et al., Proc. Natl. Acad. Sci., USA 82:6148-6152 (1985)), blastocysts or embryos; gene targeting in embryonic stem cells (Thompson et al., Cell 56:313-321 (1989)); electroporation of cells or embryos (Lo, 1983, Mol Cell. Biol. 3:1803-1814 ( 1983)); introduction of the polynucleotides of the invention using a gene gun (see, e.g., Ulmer et al., Science 259:1745 (1993); introducing nucleic acid constructs into embryonic pleuripotent stem cells and transferring the stem cells back into the blastocyst; and sperm-mediated gene transfer (Lavitrano et al., Cell 57:717-723 (1989); etc. For a review of such techniques, see Gordon, "Transgenic Animals," Intl. Rev. Cytol. 115:171-229 (1989), which is incorporated by reference herein in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell et al., Nature 380:64-66 (1996); Wilmut et al., Nature 385:810-813 (1997)).

The present invention provides for transgenic animals that carry the transgene in all their cells, as well as animals which carry the transgene in some, but not all their cells, i.e., mosaic animals or chimeric. The transgene may be integrated as a single transgene or as multiple copies such as in concatamers, e.g., head-to-head tandems or head-to-tail tandems. The transgene may also be selectively introduced into and activated in a particular cell type by following, for example, the teaching of Lasko et al. (Lasko et al., Proc. Natl. Acad. Sci. USA 89:6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu et al. (Gu et al., Science 265:103-106 (1994)). The regulatory sequences required for such a cell-type specific inactivation will depend upon the particular cell type of interest, and will be apparent to those of skill in the art.

In specific preferred embodiments, IL-21 or IL-22 polynucleotides of the invention may be expressed under the direction of a murine transferrin receptor promoter construct thereby restricting expression to the liver of transgenic animals. In other specific preferred embodiments, IL-21 or IL-22 polynucleotides of the invention are expressed under the direction of a murine beta-actin promoter construct thereby effecting ubiquitous expression of the IL-21 or IL-22 polynucleotide.

Once transgenic animals have been generated, the expression of the recombinant gene may be assayed utilizing standard techniques. Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of tissue samples obtained from the animal, *in situ* hybridization analysis, and reverse transcriptase-PCR (rt-PCR) and "TaqMAN" real time PCR. Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic and "knock-out" animals of the invention have uses which include, but are not limited to, animal model systems useful in elaborating the biological function of IL-21 and/or IL-22 polypeptides, studying conditions and/or disorders associated with aberrant IL-21 and/or IL-22 expression, and in screening for compounds effective in ameliorating such conditions and/or disorders.

In further embodiments of the invention, cells that are genetically engineered to express the polypeptides of the invention, or alternatively, that are genetically engineered not to express the polypeptides of the invention (e.g., knockouts) are administered to a patient *in vivo.* Such cells may be obtained from the patient (i.e., animal, including human) or an MHC compatible donor and can include, but are not limited to fibroblasts, bone marrow cells, blood cells (e.g., lymphocytes), adipocytes, muscle cells, endothelial cells etc. The cells are genetically engineered *in vitro* using recombinant DNA techniques to introduce the coding sequence of polypeptides of the invention into the cells, or alternatively, to disrupt the coding sequence and/or endogenous regulatory sequence associated with the polypeptides of the invention, e.g., by transduction (using viral vectors, and preferably vectors that integrate the transgene into the cell genome) or transfection procedures, including, but not limited to, the use of plasmids, cosmids, YACs, naked DNA, electroporation, liposomes, etc. The coding sequence of the polypeptides of the invention can be placed under the control of a strong constitutive or inducible promoter or promoter/enhancer to achieve expression, and preferably secretion, of the polypeptides of the invention. The engineered cells which express and preferably secrete the polypeptides of the invention can be introduced into the patient systemically, e.g., in the circulation, or intraperitoneally.

Alternatively, the cells can be incorporated into a matrix and implanted in the body, e.g., genetically engineered fibroblasts can be implanted as part of a skin graft; genetically engineered endothelial cells can be implanted as part of a lymphatic or vascular graft. (*See, for example*, Anderson, *et al*. U.S. Patent No. 5,399,349; and Mulligan & Wilson, U.S. Patent No. 5,460,959 each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

### Epitopes & Antibodies

In the present invention, "epitopes" refer to IL-21 and IL-22 polypeptide fragments having antigenic or immunogenic activity in an animal, especially in a human. A preferred embodiment of the present invention relates to an IL-21 or IL-22 polypeptide fragment comprising an epitope, as well as the polynucleotide encoding this fragment. A region of a protein molecule to which an antibody can bind is defined as an "antigenic epitope". In contrast, an "immunogenic epitope" is defined as a part of a protein that elicits an antibody response (see, for instance, *Geysen, et al., Proc. Natl. Acad. Sci. USA* **81:**3998- 4002 (1983)).

Fragments which function as epitopes may be produced by any conventional means (see, e.g., Houghten, R. A., *Proc. Natl. Acad. Sci*. *USA* **82:**5131-5135 (1985); further described in U.S. Patent No. 4,631,211).

In the present invention, antigenic epitopes preferably contain a sequence of at least seven, more preferably at least nine, and most preferably between about 15 to about 30 amino acids. Antigenic epitopes are useful to raise antibodies, including monoclonal antibodies, that specifically bind the epitope (see, for instance, Wilson, *et al*., *Cell* **37:**767-778 (1984); Sutcliffe, J. G. *et al*., *Science* **219:**660-666 (1983)).

Similarly, immunogenic epitopes can be used to induce antibodies according to methods well known in the art (see, for instance, Sutcliffe, *et al*., *supra*; Wilson, *et al*., *supra*; Chow, M., *et al*., *Proc. Natl. Acad. Sci. USA* **82:**910-914; and Bittle, F. J., *et al*., *J. Gen. Virol.* **66:**2347-2354 (1985)). A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting).

Using DNAstar analysis, SEQ ID NO:2 was found to be immunogenic at amino acids: from about Arg-2 to about Pro-11, from about Cys-24 to about Glu-32, and from about Arg-51 to about Gly-59. Thus, these regions can be used as epitopes to produce antibodies against the protein encoded by HTGED19. Again using DNAstar analysis, SEQ ID NO:4 was found to be immunogenic at amino acids: from about Gly-19 to about Ala-27, from about Pro-30 to about Arg-38, from about Phe-40 to about Ser-48, from about Tyr-58 to about Leu-67, from about Pro-105 to about Val-113, from about Pro-129 to about Ser-137, from about Asn-139 to about Ala-147, and from about Leu-151 to about Gly-159. Thus, these regions can be used as epitopes to produce antibodies against the protein encoded by HFPBX96.

As used herein, the term "antibody" (Ab) or "monoclonal antibody" (Mab) is meant to include intact molecules as well as antibody fragments (such as, for example, Fab and F(ab')2 fragments) which are capable of specifically binding to protien. Fab and F(ab')2 fragments lack the Fc fragment of intact-antibody, clear more rapidly from the circulation, and may have less non-specific tissue binding than an intact antibody (Wahl, *et al., J. Nucl. Med*. **24**:316-325 (1983)). Thus, these fragments are preferred, as well as the products of a FAB or other immunoglobulin expression library. Moreover, antibodies of the present invention include chimeric, single chain, and humanized antibodies.

The present invention further relates to antibodies and T-cell antigen receptors (TCR) which specifically bind the polypeptides of the present invention. The antibodies of the present invention include IgG (including IgG1, IgG2, IgG3, and IgG4), IgA (including IgA1 and IgA2), IgD, IgE, or IgM, and IgY. As used herein, the term "antibody" (Ab) is meant to include whole antibodies, including single-chain whole antibodies, and antigen-binding fragments thereof. Most preferably the antibodies are human antigen binding antibody fragments of the present invention include, but are not limited to, Fab, Fab' and F(ab')2, Fd, single-chain Fvs (scFv), single-chain antibodies, disulfide-linked Fvs (sdFv) and fragments comprising either a V_{L} or V_{H} domain. The antibodies may be from any animal origin including birds and mammals. Preferably, the antibodies are human, murine, rabbit, goat, guinea pig, camel, horse, or chicken.

Antigen-binding antibody fragments, including single-chain antibodies, may comprise the variable region(s) alone or in combination with the entire or partial of the following: hinge region, CH1, CH2, and CH3 domains. Also included in the invention are any combinations of variable region(s) and hinge region, CH1, CH2, and CH3 domains. The present invention further includes chimeric, humanized, and human monoclonal and polyclonal antibodies which specifically bind the polypeptides of the present invention. The present invention further includes antibodies which are anti-idiotypic to the antibodies of the present invention.

The antibodies of the present invention may be monospecific, bispecific, trispecific or of greater multispecificity. Multispecific antibodies may be specific for different epitopes of a polypeptide of the present invention or may be specific for both a polypeptide of the present invention as well as for heterologous compositions, such as a heterologous polypeptide or solid support material. See, e.g., WO 93/17715; WO 92/08802; WO 91/00360; WO 92/05793; Tutt, A. et al. (1991) J. Immunol. 147:60-69; US Patents 5,573,920, 4,474,893, 5,601,819, 4,714,681, 4,925,648; Kostelny, S.A. et al. ( 1992) J. Immunol. 148:1547-1553.

Antibodies of the present invention may be described or specified in terms of the epitope(s) or portion(s) of a polypeptide of the present invention which are recognized or specifically bound by the antibody. The epitope(s) or polypeptide portion(s) may be specified as described herein, e.g., by N-terminal and C-terminal positions, by size in contiguous amino acid residues, or listed in the Tables and Figures. Antibodies which specifically bind any epitope or polypeptide of the present invention may also be excluded. Therefore, the present invention includes antibodies that specifically bind polypeptides of the present invention, and allows for the exclusion of the same.

Antibodies of the present invention may also be described or specified in terms of their cross-reactivity. Antibodies that do not bind any other analog, ortholog, or homolog of the polypeptides of the present invention are included. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. Further included in the present invention are antibodies which only bind polypeptides encoded by polynucleotides which hybridize to a polynucleotide of the present invention under stringent hybridization conditions (as described herein). Antibodies of the present invention may also be described or specified in terms of their binding affinity. Preferred binding affinities include those with a dissociation constant or Kd less than 5X10⁻⁶M, 10⁻⁶M, 5X10⁻⁷M, 10⁻⁷M, 5X10⁻⁸M, 10⁻⁸M, 5X10⁻⁹M, 10⁻⁹M, 5X10⁻¹⁰M, 10⁻¹⁰M, 5X10⁻¹¹M, 10⁻¹¹M, 5X10⁻¹²M, 10⁻¹²M, 5X10⁻¹³M, 10⁻¹³M, 5X10⁻ ¹⁴M, 10⁻¹⁴M, 5X10⁻¹⁵M, and 10⁻¹⁵M.

Antibodies of the present invention have uses that include, but are not limited to, methods known in the art to purify, detect, and target the polypeptides of the present invention including *both in vitro* and *in vivo* diagnostic and therapeutic methods. For example, the antibodies have use in immunoassays for qualitatively and quantitatively measuring levels of the polypeptides of the present invention in biological samples. See, e.g., Harlow et al., *ANTIBODIES: A LABORATORY MANUAL,* (Cold Spring Harbor Laboratory Press, 2nd ed. 1988) (incorporated by reference in the entirety).

The antibodies of the present invention may be used either alone or in combination with other compositions. The antibodies may further be recombinantly fused to a heterologous polypeptide at the N- or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs, or toxins. See, e.g., WO 92/08495; WO 91/14438; WO 89/12624; US Patent 5,314,995; and EP 0 396 387.

The antibodies of the present invention may be prepared by any suitable method known in the art. For example, a polypeptide of the present invention or an antigenic fragment thereof can be administered to an animal in order to induce the production of sera containing polyclonal antibodies. Monoclonal antibodies can be prepared using a wide of techniques known in the art including the use of hybridoma and recombinant technology. See, e.g., Harlow et al., ANTIBODIES: A LABORATORY MANUAL, (Cold Spring Harbor Laboratory Press, 2nd ed. 1988); Hammerling, et al., in: MONOCLONAL ANTIBODIES AND T-CELL HYBRIDOMAS 563-681 (Elsevier, N.Y., 1981) (said references incorporated by reference in their entireties).

The antibodies of the present invention may be prepared by any of a variety of standard methods. For example, cells expressing the IL-21 and/or IL-22 polypeptide or an antigenic fragment thereof can be administered to an animal in order to induce the production of sera containing polyclonal antibodies. In a preferred method, a preparation of IL-21 and/or IL-22 polypeptide is prepared and purified to render it substantially free of natural contaminants. Such a preparation is then introduced into an animal in order to produce polyclonal antisera of greater specific activity.

In the most preferred method, the antibodies of the present invention are monoclonal antibodies (or IL-21 and/or IL-22 polypeptide binding fragments thereof). Such monoclonal antibodies can be prepared using hybridoma technology (Kohler *et al*., *Nature 256*:495 (1975); Köhler *et al*., *Eur. J. Immunol*. *6*:511 (1976); Kohler et al., Eur. J. Immunol. 6:292 (1976); Hammerling et al., in: *Monoclonal Antibodies and T-Cell Hybridomas*, Elsevier, N.Y., (1981) pp. 563-681 ). In general, such procedures involve immunizing an animal (preferably a mouse) with an IL-21 and/or IL-22 polypeptide antigen or, more preferably, with an IL-21 and/or IL-22 polypeptide-expressing cell. Suitable cells can be recognized by their capacity to bind anti-IL-21 and/or anti-IL-22 polypeptide antibody. Such cells may be cultured in any suitable tissue culture medium; however, it is preferable to culture cells in Earle's modified Eagle's medium supplemented with 10% fetal bovine serum (inactivated at about 56° C), and supplemented with about 10 g/l of nonessential amino acids, about 1,000 U/ml of penicillin, and about 100 µg/ml of streptomycin. The splenocytes of such mice are extracted and fused with a suitable myeloma cell line. Any suitable myeloma cell line may be employed in accordance with the present invention; however, it is preferable to employ the parent myeloma cell line (SP2O), available from the ATCC, Manassas, Virginia. After fusion, the resulting hybridoma cells are selectively maintained in HAT medium, and then cloned by limiting dilution as described by Wands, *et al*. (*Gastroenterology* 80:225-232 (1981)). The hybridoma cells obtained through such a selection are then assayed to identify clones which secrete antibodies capable of binding the IL-21 and/or IL-22 antigen.

Alternatively, additional antibodies capable of binding to the IL-21 and/or IL-22 polypeptide antigen may be produced in a two-step procedure through the use of anti-idiotypic antibodies. Such a method makes use of the fact that antibodies are themselves antigens, and that, therefore, it is possible to obtain an antibody which binds to a second antibody. In accordance with this method, IL-21 and/or IL-22 polypeptide-specific antibodies are used to immunize an animal, preferably a mouse. The splenocytes of such an animal are then used to produce hybridoma cells, and the hybridoma cells are screened to identify clones which produce an antibody whose ability to bind to the IL-21 and/or IL-22 polypeptide-specific antibody can be blocked by the IL-21 and/or IL-22 antigen. Such antibodies comprise anti-idiotypic antibodies to the IL-21 and/or IL-22 polypeptide-specific antibody and can be used to immunize an animal to induce formation of further IL-21 and/or IL-22 polypeptide-specific antibodies.

Fab and F(ab')2 fragments may be produced by proteolytic cleavage, using enzymes such as papain (to produce Fab fragments) or pepsin (to produce F(ab')2 fragments).

Alternatively, antibodies of the present invention can be produced through the application of recombinant DNA technology or through synthetic chemistry using methods known in the art. For example, the antibodies of the present invention can be prepared using various phage display methods known in the art. In phage display methods, functional antibody domains are displayed on the surface of a phage particle which carries polynucleotide sequences encoding them. Phage with a desired binding property are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman U. et al. (1995) J. Immunol. Methods 182:41-50; Ames, R.S. et al. (1995) J. Immunol. Methods 184:177-186; Kettleborough, C.A. et al. (1994) Eur. J. Immunol. 24:952-958; Persic, L. et al. (1997) Gene 187 9-18; Burton, D.R. et al. (1994) Advances in Immunology 57:191-280; PCT/GB91/01134; WO 90/02809; WO 91/10737; WO 92/01047; WO 92/18619; WO 93/11236; WO 95/15982; WO 95/20401; and US Patents 5,698,426, 5,223,409, 5,403,484, 5,580,717, 5,427,908, 5,750,753, 5,821,047, 5,571,698, 5,427,908, 5,516,637, 5,780,225, 5,658,727 and 5,733,743 (said references incorporated by reference in their entireties).

As described in the above references, after phage selection, the antibody coding regions from the phage can be isolated and used to generate whole antibodies, including human antibodies, or any other desired antigen binding fragment, and expressed in any desired host including mammalian cells, insect cells, plant cells, yeast, and bacteria. For example, techniques to recombinantly produce Fab, Fab' and F(ab')2 fragments can also be employed using methods known in the art such as those disclosed in WO 92/22324; Mullinax, R.L. et al. BioTechniques 12(6):864-869 (1992); and Sawai, H. et al. AJRI 34:26-34 (1995); and Better, M. et al. Science 240:1041-1043 (1988) (said references incorporated by reference in their entireties).

Examples of techniques which can be used to produce single-chain Fvs (scFvs) and antibodies include those described in U.S. Patents 4,946,778 and 5,258,498; Huston et al. Methods in Enzymology 203:46-88 (1991); Shu, L. et al. PNAS 90:7995-7999 (1993); and Skerra, A. et al. Science 240:1038-1040 (1988). For some uses, including *in vivo* use of antibodies in humans and *in vitro* detection assays, it may be preferable to use chimeric, humanized, or human antibodies. Methods for producing chimeric antibodies are known in the art. See e.g., Morrison, Science 229:1202 (1985); Oi et al., BioTechniques 4:214 (1986); Gillies, S.D. et al., J. Immunol. Methods 125:191-202 (1989); and US Patent 5,807,715. Antibodies can be humanized using a variety of techniques including CDR-grafting (EP 0 239 400; WO 91/09967; US Patent 5,530,101; and 5,585,089), veneering or resurfacing (EP 0 592 106; EP 0 519 596; Padlan, E.A., Molecular Immunology 28(4/5):489-498 (1991); Studnicka G.M. et al., Protein Engineering 7(6):805-814 (1994); Roguska M.A. et al., PNAS 91:969-973) (1994), and chain shuffling (US Patent 5,565,332). Human antibodies can be made by a variety of methods known in the art including phage display methods described above. See also, US Patents 4,444,887, 4,716,111, 5,545,806, and 5,814,318; and WO 98/46645 (said references incorporated by reference in their entireties).

Further included in the present invention are antibodies recombinantly fused or chemically conjugated (including both covalently and non-covalently conjugations) to a polypeptide of the present invention. The antibodies may be specific for antigens other than polypeptides of the present invention. For example, antibodies may be used to target the polypeptides of the present invention to particular cell types, either *in vitro* or *in vivo,* by fusing or conjugating the polypeptides of the present invention to antibodies specific for particular cell surface receptors. Antibodies fused or conjugated to the polypeptides of the present invention may also be used in *in vitro* immunoassays and purification methods using methods known in the art. See e.g., Harbor et al. *supra* and WO 93/21232; EP 0 439 095; Naramura, M. et al., Immunol. Lett. 39:91-99 (1994); US Patent 5,474,981; Gillies, S.O. et al. PNAS 89:1428-1432 ( 1992); Fell, H.P. et al., J. Immunol. 146:2446-2452 (1991) (said references incorporated by reference in their entireties).

The present invention further includes compositions comprising the polypeptides of the present invention fused or conjugated to antibody domains other than the variable regions. For example, the polypeptides of the present invention may be fused or conjugated to an antibody Fc region, or portion thereof. The antibody portion fused to a polypeptide of the present invention may comprise the hinge region, CH1 domain, CH2 domain, and CH3 domain or any combination of whole domains or portions thereof. The polypeptides of the present invention may be fused or conjugated to the above antibody portions to increase the *in vivo* half life of the polypeptides or for use in immunoassays using methods known in the art. The polypeptides may also be fused or conjugated to the above antibody portions to form multimers. For example, Fc portions fused to the polypeptides of the present invention can form dimers through disulfide bonding between the Fc portions. Higher multimeric forms can be made by fusing the polypeptides to portions of IgA and IgM. Methods for fusing or conjugating the polypeptides of the present invention to antibody portions are known in the art. See e.g., US Patents 5,336,603, 5,622,929, 5,359,046, 5,349,053, 5,447,851, 5,112,946; EP 0 307 434, EP 0 367 166; WO 96/04388, WO 91/06570; Ashkenazi, A. et al., PNAS 88:10535-10539 (1991); Zheng, X.X. et al., J. Immunol. 154:5590-5600 (1995); and Vil, H. et al., PNAS 89:11337-11341 (1992) (said references incorporated by reference in their entireties).

The invention further relates to antibodies which act as agonists or antagonists of the polypeptides of the present invention. For example, the present invention includes antibodies which disrupt the receptor/ligand interactions with the polypeptides of the invention either partially or fully. Included are both receptor-specific antibodies and ligand-specific antibodies. Included are receptor-specific antibodies which do not prevent ligand binding but prevent receptor activation. Receptor activation (i.e., signaling) may be determined by techniques described herein or otherwise known in the art. Also included are receptor-specific antibodies which both prevent ligand binding and receptor activation. Likewise, included are neutralizing antibodies which bind the ligand and prevent binding of the ligand to the receptor, as well as antibodies which bind the ligand, thereby preventing receptor activation, but do not prevent the ligand from binding the receptor. Further included are antibodies which activate the receptor. These antibodies may act as agonists for either all or less than all of the biological activities affected by ligand-mediated receptor activation. The antibodies may be specified as agonists or antagonists for biological activities comprising specific activities disclosed herein. The above antibody agonists can be made using methods known in the art. See e.g., WO 96/40281; US Patent 5,811,097; Deng, B. et al., Blood 92(6):1981-1988 (1998); Chen, Z. et al., Cancer Res. 58(16):3668-3678 (1998); Harrop, J.A. et al., J. Immunol. 161(4):1786-1794 (1998); Zhu, Z. et al., Cancer Res. 58(15):3209-3214 (1998); Yoon, D.Y. et al., J. Immunol. 160(7):3170-3179 (1998); Prat, M. et al., J. Cell. Sci. 111(Pt2):237-247 (1998); Pitard, V. et al., J. Immunol. Methods 205(2):177-190 (1997); Liautard, J. et al., Cytokinde 9(4):233-241 (1997); Carlson, N.G. et al., J. Biol. Chem. 272(17):11295-11301 (1997); Taryman, R.E. et al., Neuron 14(4):755-762 (1995); Muller, Y.A. et al., Structure 6(9):1153-1167 (1998); Bartunek, P. et al., Cytokine 8(1):14-20 (1996) (said references incorporated by reference in their entireties).

As discussed above, antibodies to the IL-21 and/or IL-22 polypeptides of the invention can, in turn, be utilized to generate anti-idiotype antibodies that "mimic" the IL-21 and/or IL-22, using techniques well known to those skilled in the art. (See, e.g., Greenspan & Bona, *FASEB J.* 7(5):437-444 (1989), and Nissinoff, *J. Immunol*. 147(8):2429-2438 (1991)). For example, antibodies which bind to IL-21 and/or IL-22 and competitively inhibit the IL-21 and/or IL-22 binding to receptor can be used to generate anti-idiotypes that "mimic" the IL-21 and/or IL-22 binding domain and, as a consequence, bind to and neutralize IL-21 and/or IL-22 and/or its receptor. Such neutralizing anti-idiotypes or Fab fragments of such anti-idiotypes can be used in therapeutic regimens to neutralize IL-21 and/or IL-22 ligands.

### Fusion Proteins

Any IL-21 or IL-22 polypeptide can be used to generate fusion proteins. For example, the IL-21 or IL-22 polypeptides, when fused to a second protein, can be used as an antigenic tag. Antibodies raised against the IL-21 or IL-22 polypeptides can be used to indirectly detect a second protein by binding to IL-21 or IL-22, respectively. Moreover, because secreted proteins target cellular locations based on trafficking signals, the IL-21 and IL-22 polypeptides can be used as targeting molecules once fused to other proteins.

Examples of domains that can be fused to the IL-21 and IL-22 polypeptides include not only heterologous signal sequences, but also other heterologous functional regions. The fusion does not necessarily need to be direct, but may occur through linker sequences.

Moreover, fusion proteins may also be engineered to improve characteristics of the IL-21 and IL-22 polypeptides. For instance, a region of additional amino acids, particularly charged amino acids, may be added to the N-terminus of the IL-21 and IL-22 polypeptides to improve stability and persistence during purification from the host cell or during subsequent handling and storage. Also, peptide moieties may be added to the IL-21 and IL-22 polypeptides to facilitate purification. Such regions may be removed prior to final preparation of the IL-21 and IL-22 polypeptides. The addition of peptide moieties to facilitate handling of polypeptides are familiar and routine techniques in the art.

Moreover, IL-21 and IL-22 polypeptides, including fragments, and specifically epitopes, can be combined with parts of the constant domain of immunoglobulins (IgG), resulting in chimeric polypeptides. These fusion proteins facilitate purification and show an increased half-life *in vivo.* One reported example describes chimeric proteins consisting of the first two domains of the human CD4-polypeptide and various domains of the constant regions of the heavy or light chains of mammalian immunoglobulins (EP A 394,827; Traunecker, *et al*., *Nature* **331:**84-86 (1988)). Fusion proteins having disulfide-linked dimeric structures (due to the IgG) can also be more efficient in binding and neutralizing other molecules, than the monomeric secreted protein or protein fragment alone (Fountoulakis, *et al*., *J. Biochem.* **270:**3958-3964 (1995)).

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties (EP-A 0232 262). Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5 (see, Bennett, D., *et al., J. Mol. Recog.* **8:**52-58 (1995); Johanson, K., *et al., J. Biol. Chem*. **270:**9459-9471 (1995)).

Moreover, the IL-21 and IL-22 polypeptides can be fused to marker sequences, such as a peptide which facilitates purification of IL-21 and IL-22, respectively. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described by Gentz and coworkers (*Proc. Natl. Acad. Sci. USA* **86:**821-824 (1989)), for instance, hexa-histidine provides for convenient purification of the fusion protein. Another peptide tag useful for purification, the "HA" tag, corresponds to an epitope derived from the influenza hemagglutinin protein (Wilson, *et al., Cell* **37:**767 (1984)).

In further preferred embodiments, IL-21 or IL-22 polynucleotides of the invention are fused to a polynucleotide encoding a "FLAG" polypeptide. Thus, an IL-21-FLAG or an IL-22-FLAG fusion protein is encompassed by the present invention. The FLAG antigenic polypeptide may be fused to an IL-21 or an IL-22 polypeptide of the invention at either or both the amino or the carboxy terminus. In preferred embodiments, an IL-21-FLAG or an IL-22-FLAG fusion protein is expressed from a pFLAG-CMV-5a or a pFLAG-CMV-1 expression vector (available from Sigma, St. Louis, MO, USA). *See*, Andersson, S., *et al., J. Biol. Chem*. 264:8222-29 (1989); Thomsen, D. R., *et al., Proc. Natl. Acad. Sci. USA*, 81:659-63 (1984); and Kozak, M., *Nature* 308:241 (1984) (each of which is hereby incorporated by reference). In further preferred embodiments, an IL-21-FLAG or an IL-22-FLAG fusion protein is detectable by anti-FLAG monoclonal antibodies (also available from Sigma).

Thus, any of the above fusion proteins can be engineered using IL-21 and/or IL-22 polynucleotides or the polypeptides of the invention.

### Vectors, Host Cells, and Protein Production

The present invention also relates to vectors containing the IL-21 and IL-22 polynucleotides, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

IL-21 and IL-22 polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The IL-21 and IL-22 polynucleotide inserts should be operatively linked to an appropriate promoter, such as the phage lambda PL promoter, the *E. coli* lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli, Streptomyces*, and *Salmonella typhiniurium* cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pHE4-5 and other pHE-like vectors; pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals (for example, Davis, *et al., Basic Methods In Molecular Biology* (1986)). It is specifically contemplated that IL-21 and IL-22 polypeptides may, in fact, be expressed by a host cell lacking a recombinant vector.

IL-21 and IL-22 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

IL-21 and IL-22 polypeptides, and preferably the secreted forms thereof, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the IL-21 and IL-22 polypeptides may be glycosylated or may be non-glycosylated. In addition, IL-21 and IL-22 polypeptides may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

### Uses of the IL-21 and IL-22 Polynucleotides

The IL-21 and IL-22 polynucleotides identified herein can be used in numerous ways as reagents. The following description should be considered exemplary and utilizes known techniques.

There exists an ongoing need to identify new chromosome markers, since few chromosome marking reagents, based on actual sequence data (repeat polymorphisms), are presently available. Clone HTGED 19 and clone HFPBX96 can each be mapped to a specific chromosome. Thus, IL-21 and IL-22 polynucleotides can then be used in linkage analysis as a marker for those specific chromosome.

Briefly, sequences can be mapped to chromosomes by preparing PCR primers (preferably 15-25 bp) from the sequences shown in SEQ ID NO:1, SEQ ID NO:3, SEQ ID NO:28, and SEQ ID NO:31. Primers can be selected using computer analysis so that primers do not span more than one predicted exon in the genomic DNA. These primers are then used for PCR screening of somatic cell hybrids containing individual human chromosomes. Only those hybrids containing the human IL-21 or IL-22 genes corresponding to SEQ ID NO:1, SEQ ID NO:3, SEQ ID NO:28 or SEQ ID NO:31, respectively, will yield an amplified fragment.

Similarly, somatic hybrids provide a rapid method of PCR mapping the polynucleotides to particular chromosomes. Three or more clones can be assigned per day using a single thermal cycler. Moreover, sublocalization of the IL-21 and IL-22 polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the IL-21 and IL-22 polynucleotides can also be achieved using fluorescence *in situ* hybridization (FISH) of a metaphase chromosomal spread. This technique uses polynucleotides as short as 500 or 600 bases; however, polynucleotides 2,000-4,000 bp are preferred (For review, see Verma, *et al*., "Human Chromosomes: a Manual of Basic Techniques," Pergamon Press, New York (1988)).

For chromosome mapping, the IL-21 and IL-22 polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely conserved within gene families, thus increasing the chance of cross hybridization during chromosomal mapping.

In a preferred embodiment, the gene encoding IL-22 of the present invention has been mapped using FISH technology to a location on human chromosome 13 at position 13q11. In addition, the gene encoding IL-21 of the present invention has mapped to a location on human chromosome 7. *See also*, Example 4 *infra*.

Once a polynucleotide has been mapped to a precise chromosomal location, the physical position of the polynucleotide can be used in linkage analysis. Linkage analysis establishes coinheritance between a chromosomal location and presentation of a particular disease (disease mapping data are found, for example, in McKusick, V., Mendelian Inheritance in Man (available on line through Johns Hopkins University Welch Medical Library)). Assuming 1 megabase mapping resolution and one gene per 20 kb, a cDNA precisely localized to a chromosomal region associated with the disease could be one of 50-500 potential causative genes.

Thus, once coinheritance is established, differences in the IL-21 and IL-22 polynucleotides and the corresponding genes between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the IL-21 and IL-22 polypeptides and the corresponding genes from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using IL-21 and IL-22 polynucleotides. Any of these alterations (altered expression, chromosomal rearrangement, or mutation) can be used as a diagnostic or prognostic marker.

In addition to the foregoing, an IL-21 or IL-22 polynucleotide can be used to control gene expression through triple helix formation or antisense DNA or RNA. Both methods rely on binding of the polynucleotide to DNA or RNA. For these techniques, preferred polynucleotides are usually 20 to 40 bases in length and complementary to either the region of the gene involved in transcription (triple helix - see Lee, *et al., Nucl. Acids Res*. 6:3073 (1979); Cooney, *et al., Science* 241:456 (1988); and Dervan, *et al., Science* 251:1360 (1991)) or to the mRNA itself (antisense - Okano, J. *Neurochem*. 56:560 (1991); Oligodeoxy-nucleotides as Antisense Inhibitors of Gene Expression, CRC Press, Boca Raton, FL (1988)). Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

IL-21 and IL-22 polynucleotides are also useful in gene therapy. One goal of gene therapy is to insert a normal gene into an organism having a defective gene, in an effort to correct the genetic defect. IL-21 and IL-22 offer means of targeting such genetic defects in a highly accurate manner. Another goal is to insert a new gene that was not present in the host genome, thereby producing a new trait in the host cell.

The IL-21 and IL-22 polynucleotides are also useful for identifying individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The IL-21 and IL-22 polynucleotides can be used as additional DNA markers for RFLP.

The IL-21 and IL-22 polynucleotides can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, etc., can be amplified using PCR. In one prior art technique, gene sequences amplified from polymorphic loci, such as DQa class II HLA gene, are used in forensic biology to identify individuals (Erlich, *H., PCR Technology*, Freeman and Co. (1992)). Once these specific polymorphic loci are amplified, they are digested with one or more restriction enzymes, yielding an identifying set of bands on a Southern blot probed with DNA corresponding to the DQa class II HLA gene. Similarly, IL-21 and IL-22 polynucleotides can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from IL-21 and IL-22 sequences. Panels of such reagents can identify tissue by species and/or by organ type. In a similar fashion, these reagents can be used to screen tissue cultures for contamination.

Because IL-21 is found expressed almost exclusively in apoptotic T-cells, IL-21 polynucleotides are useful as hybridization probes for differential identification of the tissue(s) or cell type(s) present in a biological sample. Similarly, polypeptides and antibodies directed to IL-21 polypeptides are useful to provide immunological probes for differential identification of the tissue(s) or cell type(s). In addition, for a number of disorders of the above tissues or cells, particularly of the Immune system, significantly higher or lower levels of IL-21 gene expression may be detected in certain tissues (e.g., cancerous and wounded tissues) or bodily fluids (e.g., serum, plasma; urine, synovial fluid or spinal fluid) taken from an individual having such a disorder, relative to a "standard" IL-21 gene expression level, i.e., the IL-21 expression level in healthy tissue from an individual not having the Immune system disorder.

Likewise, since IL-22 is found expressed in bone marrow, skeletal muscle, and brain, IL-22 polynucleotides are useful as hybridization probes for differential identification of the tissue(s) or cell type(s) present in a biological sample. Similarly, polypeptides and antibodies directed to IL-22 polypeptides are useful to provide immunological probes for differential identification of the tissue(s) or cell type(s). In addition, for a number of disorders of the above tissues or cells, particularly of the Immune system, significantly higher or lower levels of IL-22 gene expression may be detected in certain tissues (e.g., cancerous and wounded tissues) or bodily fluids (e.g., serum, plasma, urine, synovial fluid or spinal fluid) taken from an individual having such a disorder, relative to a "standard" IL-22 gene expression level, i.e., the IL-22 expression level in healthy tissue from an individual not having the Immune system disorder.

Thus, the invention provides a diagnostic method of a disorder, which involves: (a) assaying IL-21 or IL-22 gene expression level in cells or body fluid of an individual; (b) comparing the IL-21 or IL-22 gene expression level with a standard IL-21 or IL-22 gene expression level, respectively, whereby an increase or decrease in the assayed IL-21 or IL-22 gene expression level compared to the standard expression level is indicative of disorder in the Immune system.

In the very least, the IL-21 and IL-22 polynucleotides can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

### Uses of IL-21 and IL-22 Polypeptides

IL-21 and IL-22 polypeptides can be used in numerous ways. The following description should be considered exemplary and utilizes known techniques.

IL-21 and IL-22 polypeptides can be used to assay protein levels in a biological sample using antibody-based techniques. For example, protein expression in tissues can be studied with classical immunohistological methods (Jalkanen, *M., et al*., *J. Cell. Biol.* **101:**976-985 (1985); Jalkanen, M., *et al*., *J. Cell. Biol*. **105:**3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, ¹³¹I, ¹¹²In, ^{99m}Tc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ^{99m}Tc. The labeled antibody or antibody fragment will then preferentially accumulate at the location of cells which contain the specific protein. *In vivo* tumor imaging is described by Burchiel and colleagues ("Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982)).

Thus, the invention provides a diagnostic method of a disorder, which involves (a) assaying the expression of IL-21 or IL-22 polypeptides in cells or body fluid of an individual; (b) comparing the level of IL-21 or IL-22 gene expression with a standard gene expression level, whereby an increase or decrease in the assayed IL-21 or IL-22 polypeptide gene expression level compared to the standard expression level is indicative of a disorder.

Moreover, IL-21 and IL-22 polypeptides can be used to treat disease. For example, patients can be administered IL-21 and IL-22 polypeptides in an effort to replace absent or decreased levels of the IL-21 and IL-22 polypeptides, respectively, (e.g., insulin), to supplement absent or decreased levels of a different polypeptide (e.g., hemoglobin S for hemoglobin B), to inhibit the activity of a polypeptide (e.g., an oncogene), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth).

Similarly, antibodies directed to IL-21 and IL-22 polypeptides can also be used to treat disease. For example, administration of an antibody directed to an IL-21 or IL-22 polypeptide can bind and reduce overproduction of the polypeptide. Similarly, administration of an antibody can activate the polypeptide, such as by binding to a polypeptide bound to a membrane (receptor).

At the very least, the IL-21 and IL-22 polypeptides can be used as molecular weight markers on SDS-PAGE gels or on molecular sieve gel filtration columns using methods well known to those of skill in the art, IL-21 and IL-22 polypeptides can also be used to raise antibodies, which, in turn, are used to measure protein expression from a recombinant cell, as a way of assessing transformation of the host cell. Moreover, IL-21 and IL-22 polypeptides can be used to test the following biological activities.

### Biological Activities of IL-21 and IL-22

IL-21 and IL-22 polynucleotides and polypeptides can be used in assays to test for one or more biological activities. If IL-21 and IL-22 polynucleotides and polypeptides do exhibit activity in a particular assay, it is likely that IL-21 and IL-22 may be involved in the diseases associated with the biological activity. Therefore, IL-21 and IL-22 could be used to treat the associated disease.

The IL-21 and IL-22 proteins of the present invention modulate IL-6 secretion from NIH-3T3 cells. An *in vitro* ELISA assay which quantitates the amount of IL-6 secreted from cells in response to treatment with cytokines or the soluble extracellular domains of cytokine receptors has been described (Yao, Z., *et al*., *Immunity* **3:**811-821 (1995)). Briefly, the assay involves plating the target cells at a density of approximately 5 x 10⁶ cells/mL in a volume of 500 µL in the wells of a 24 well flat-bottomed culture plate (Costar). The cultures are then treated with various concentrations of the cytokine or the soluble extracellular domain of cytokine receptor in question. The cells are then cultured for 24 hours at 37°C. At this time, 50 µL of supernatant is removed and assayed for the quantity of IL-6 essentially as described by the manufacturer (Genzyme, Boston, MA). IL-6 levels are then calculated by reference to a standard curve constructed with recombinant IL-17 cytokine. Such activity is useful for determining the level of IL-21- or IL-22-mediated IL-6 secretion.

IL-21 and IL-22 protein modulates immune system cell proliferation and differentiation in a dose-dependent manner in the above-described assay. Thus, "a polypeptide having IL-21 or IL-22 protein activity" includes polypeptides that also exhibit any of the same stimulatory activities in the above-described assays in a dose-dependent manner. Although the degree of dose-dependent activity need not be identical to that of the IL-21 or IL-22 proteins, preferably, "a polypeptide having IL-21 or IL-22 protein activity" will exhibit substantially similar dose-dependence in a given activity as compared to the IL-21 or IL-22 protein (i.e., the candidate polypeptide will exhibit greater activity or not more than about 25-fold less and, preferably, not more than about tenfold less activity relative to the reference IL-21 or IL-22 protein).

Lymphocyte proliferation is another *in vitro* assay which may be performed to determine the activity of IL-21 and IL-22. For example, Yao and colleagues (*Immunity* **3:**811-821 (1995)) have recently described an *in vitro* assay for determining the effects of various cytokines and soluble cytokine receptors on the proliferation of murine leukocytes. Briefly, lymphoid organs are harvested aseptically, lymphocytes are isolated from the harvested organs, and the resulting collection of lymphoid cells are suspended in standard culture medium as described by Fanslow and coworkers (*J. Immunol.* **147:**535-5540 (1991)). The lymphoid cell suspensions may then be divided into several different subclasses of lymphoid cells including splenic T-cells, lymph node B-cells, CD4⁺ and CD8⁺ T-cells, and mature adult thymocytes. For splenic T-cells, spleen cell suspensions (200 x 10⁶ cells) are incubated with CD11b mAb and class II MHC mAb for 30 min at 4°C, loaded on a T-cell purification column (Pierce, Rockford, IL), and the T-cells eluted according to the manufacturer's instructions. Using this method, purity of the resulting T-cell populations should be >95% CD3⁺ and <1% sIgM⁺. For purification of lymph node subsets, B-cells are removed from by adherence to tissue culture dishes previously coated with goat anti-mouse IgG (10µg/mL). Remaining cells were then incubated with anti-CD4 or anti-CD8 for 30 min at 4°C then washed and placed on tissue culture dishes previously coated with goat anti-rat IgG (20 µg/mL). After 45 min, nonadherent cells are removed and tested for purity by flow cytometry. CD4 and surface Ig-depleted cells should be >90% TCR-ab, CD8⁺, whereas CD8 and surface Ig-depleted cells should be >95% TCR-ab, CD4⁺. Finally, to enrich for mature adult thymocytes, cells are suspended at 10⁸/mL in 10% anti-HSA and 10% low tox rabbit complement (Cedarlane, Ontario, Canada), incubated for 45 min at 37°C, and remaining viable cells isolated over Ficoll-Hypaque (Pharmacia, Piscataway, NJ). This procedure should yield between 90 and 95% CD3^{hi} cells that are either CD4⁺8⁻ or CD4⁻8⁺.

### Immune Activity

IL-21 and IL-22 polypeptides or polynucleotides may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious. Moreover, IL-21 and IL-22 polynucleotides or polypeptides can be used as a marker or detector of a particular immune system disease or disorder.

IL-21 and IL-22 polynucleotides or polypeptides may be useful in treating or detecting deficiencies or disorders of hematopoietic cells. IL-21 and IL-22 polypeptides or polynucleotides could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia), ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, IL-21 and IL-22 polypeptides or polynucleotides can also be used to modulate hemostatic (the stopping of bleeding) or thrombolytic activity (clot formation). For example, by increasing hemostatic or thrombolytic activity, IL-21 and IL-22 polynucleotides or polypeptides could be used to treat blood coagulation disorders (e.g., afibrinogenemia, factor deficiencies), blood platelet disorders (e.g. thrombocytopenia), or wounds resulting from trauma, surgery, or other causes. Alternatively, IL-21 and IL-22 polynucleotides or polypeptides that can decrease hemostatic or thrombolytic activity could be used to inhibit or dissolve clotting, important in the treatment of heart attacks (infarction), strokes, or scarring.

IL-21 and IL-22 polynucleotides or polypeptides may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of IL-21 and IL-22 polypeptides or polynucleotides that can inhibit an immune response, particularly the proliferation, differentiation, or chemotaxis of T-cells, may be an effective therapy in preventing autoimmune disorders.

Examples of autoimmune disorders that can be treated or detected by IL-21 and IL-22 include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by IL-21 and IL-22 polypeptides or polynucleotides. Moreover, IL-21 and IL-22 can be used to treat anaphylaxis, hypersensitivity to an antigenic molecule, or blood group incompatibility.

IL-21 and IL-22 polynucleotides or polypeptides may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of IL-21 and IL-22 polypeptides or polynucleotides that inhibits an immune response, particularly the proliferation, differentiation, or chemotaxis of T-cells, may be an effective therapy in preventing organ rejection or GVHD.

Similarly, IL-21 and IL-22 polypeptides or polynucleotides may also be used to modulate inflammation. For example, IL-21 and IL-22 polypeptides or polynucleotides may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

### Hyperproliferative Disorders

IL-21 and IL-22 polypeptides or polynucleotides can be used to treat or detect hyperproliferative disorders, including neoplasms. IL-21 and IL-22 polypeptides or polynucleotides may inhibit the proliferation of the disorder through direct or indirect interactions. Alternatively, IL-21 and IL-22 polypeptides or polynucleotides may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by IL-21 and IL-22 polynucleotides or polypeptides include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by IL-21 and IL-22 polynucleotides or polypeptides. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

### Infectious Disease

IL-21 and IL-22 polypeptides or polynucleotides can be used to treat or detect infectious agents. For example, by increasing the immune response, particularly increasing the proliferation and differentiation of B and/or T cells, infectious diseases may be treated. The immune response may be increased by either enhancing an existing immune response, or by initiating a new immune response. Alternatively, IL-21 and IL-22 polypeptides or polynucleotides may also directly inhibit the infectious agent, without necessarily eliciting an immune response.

Viruses are one example of an infectious agent that can cause disease or symptoms that can be treated or detected by IL-21 and IL-22 polynucleotides or polypeptides. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g., Influenza), Papovaviridae, Parvoviridae, Picornaviridae, Poxviridae (such as Smallpox or Vaccinia), Reoviridae (e.g., Rotavirus), Retroviridae (HTLV-1, HTLV-II, Lentivirus), and Togaviridae (e.g., Rubivirus). Viruses falling within these families can cause a variety of diseases or symptoms, including, but not limited to: arthritis, bronchiollitis, encephalitis, eye infections (e.g., conjunctivitis, keratitis), chronic fatigue syndrome, hepatitis (A, B, C, E, Chronic Active, Delta), meningitis, opportunistic infections (e.g., AIDS), pneumonia, Burkitt's Lymphoma, chickenpox , hemorrhagic fever, Measles, Mumps, Parainfluenza, Rabies, the common cold, Polio, leukemia, Rubella, sexually transmitted diseases, skin diseases (e.g., Kaposi's, warts), and viremia. IL-21 and IL-22 polypeptides or polynucleotides can be used to treat or detect any of these symptoms or diseases.

Similarly, bacterial or fungal agents that can cause disease or symptoms and that can be treated or detected by IL-21 and IL-22 polynucleotides or polypeptides include, but not limited to, the following Gram-Negative and Gram-positive bacterial families and fungi: Actinomycetales (e.g., Corynebacterium, Mycobacterium, Norcardia), Aspergillosis, Bacillaceae (e.g., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (e.g., Acinetobacter, Gonorrhea, Menigococcal), Pasteurellacea Infections (e.g., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. IL-21 and IL-22 polypeptides or polynucleotides can be used to treat or detect any of these symptoms or diseases.

Moreover, parasitic agents causing disease or symptoms that can be treated or detected by IL-21 polynucleotides or polypeptides include, but not limited to, the following families: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas. These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), Malaria, pregnancy complications, and toxoplasmosis. IL-21 and IL-22 polypeptides or polynucleotides can be used to treat or detect any of these symptoms or diseases.

Preferably, treatment using IL-21 and IL-22 polypeptides or polynucleotides could either be by administering an effective amount of IL-21 or IL-22 polypeptide to the patient, or by removing cells from the patient, supplying the cells with IL-21 and IL-22 polynucleotide, and returning the engineered cells to the patient *(ex vivo* therapy). Moreover, the IL-21 and IL-22 polypeptide or polynucleotide can be used as an antigen in a vaccine to raise an immune response against infectious disease.

### Regeneration

IL-21 and IL-22 polynucleotides or polypeptides can be used to differentiate, proliferate, and attract cells, leading to the regeneration of tissues (see, *Science* **276**:59-87 (1997)). The regeneration of tissues could be used to repair, replace, or protect tissue damaged by congenital defects, trauma (wounds, bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vascular (including vascular endothelium), nervous, hematopoietic, and skeletal (bone, cartilage, tendon, and ligament) tissue. Preferably, regeneration occurs without or decreased scarring. Regeneration also may include angiogenesis.

Moreover, IL-21 and IL-22 polynucleotides or polypeptides may increase regeneration of tissues difficult to heal. For example, increased tendon/ligament regeneration would quicken recovery time after damage. IL-21 and IL-22 polynucleotides or polypeptides of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using IL-21 and IL-22 polynucleotides or polypeptides to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the IL-21 and IL-22 polynucleotides or polypeptides.

### Chemotaxis

IL-21 and IL-22 polynucleotides or polypeptides may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

IL-21 and IL-22 polynucleotides or polypeptides may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. As a chemotactic molecule, IL-21 and IL-22 could also attract fibroblasts, which can be used to treat wounds.

It is also contemplated that IL-21 and IL-22 polynucleotides or polypeptides may inhibit chemotactic activity. These molecules could also be used to treat disorders. Thus, IL-21 and IL-22 polynucleotides or polypeptides could be used as an inhibitor of chemotaxis.

### Binding Activity

IL-21 and IL-22 polypeptides may be used to screen for molecules that bind to IL-21 or IL-22 or for molecules to which IL-21 or IL-22 bind. The binding of IL-21 and IL-22 and the molecule may activate (agonist), increase, inhibit (antagonist), or decrease activity of the IL-21 and IL-22 or the molecule bound. Examples of such molecules include antibodies, oligonucleotides, proteins (e.g., receptors),or small molecules.

Preferably, the molecule is closely related to the natural ligand of IL-21 or IL-22, e.g., a fragment of the ligand, or a natural substrate, a ligand, a structural or functional mimetic (see, Coligan, *et al*., Current Protocols in Immunology 1(2):Chapter 5 (1991)). Similarly, the molecule can be closely related to the natural receptor to which IL-21 and IL-22 bind, or at least, a fragment of the receptor capable of being bound by IL-21 or IL-22 (e.g., active site). In either case, the molecule can be rationally designed using known techniques.

Preferably, the screening for these molecules involves producing appropriate cells which express IL-21 and IL-22, either as a secreted protein or on the cell membrane. Preferred cells include cells from mammals, yeast, Drosophila, or *E*. *coli*. Cells expressing IL-21 and IL-22 (or cell membrane containing the expressed polypeptide) are then preferably contacted with a test compound potentially containing the molecule to observe binding, stimulation, or inhibition of activity of either IL-21 and IL-22 or the molecule.

The assay may simply test binding of a candidate compound to IL-21 or IL-22, wherein binding is detected by a label, or in an assay involving competition with a labeled competitor. Further, the assay may test whether the candidate compound results in a signal generated by binding to IL-21 or IL-22.

Alternatively, the assay can be carried out using cell-free preparations, polypeptide/molecule affixed to a solid support, chemical libraries, or natural product mixtures. The assay may also simply comprise the steps of mixing a candidate compound with a solution containing IL-21 or IL-22, measuring IL-21/molecule or IL-22/molecule activity or binding, respectively, and comparing the IL-21/molecule or IL-22/molecule activity or binding to a standard.

Preferably, an ELISA assay can measure IL-21 and IL-22 levels or activities in a sample (e.g., biological sample) using a monoclonal or polyclonal antibody. The antibody can measure IL-21 and IL-22 levels or activities by either binding, directly or indirectly, to IL-21 or IL-22 or by competing with IL-21 or IL-22 for a substrate.

All of these above assays can be used as diagnostic or prognostic markers. The molecules discovered using these assays can be used to treat disease or to bring about a particular result in a patient (e.g., blood vessel growth) by activating or inhibiting IL-21 or IL-22. Moreover, the assays can discover agents which may inhibit or enhance the production of IL-21 and IL-22 from suitably manipulated cells or tissues.

Therefore, the invention includes a method of identifying compounds which bind to IL-21 and IL-22 comprising the steps of: (a) incubating a candidate binding compound with IL-21 or IL-22; and (b) determining if binding has occurred. Moreover, the invention includes a method of identifying agonists/antagonists comprising the steps of: (a) incubating a candidate compound with IL-21 or LL-22, (b) assaying a biological activity , and (b) determining if a biological activity of IL-21 or IL-22, respectively, has been altered.

### Other Activities

IL-21 and IL-22 polypeptides or polynucleotides may also increase or decrease the differentiation or proliferation of embryonic stem cells, besides, as discussed above, hematopoietic lineage.

IL-21 and IL-22 polypeptides or polynucleotides may also be used to modulate mammalian characteristics, such as body height, weight, hair color, eye color, skin, percentage of adipose tissue, pigmentation, size, and shape (e.g., cosmetic surgery). Similarly, IL-21 and IL-22 polypeptides or polynucleotides may be used to modulate mammalian metabolism affecting catabolism, anabolism, processing, utilization, and storage of energy.

IL-21 and IL-22 polypeptides or polynucleotides may be used to change a mammal's mental state or physical state by influencing biorhythms, caricadic rhythms, circadian rhythms, depression (including depressive disorders), tendency for violence, tolerance for pain (in preferred embodiments, analyzed by a rat hyperalgesic footpad pain assay), reproductive capabilities (preferably by Activin or Inhibin-like activity), hormonal or endocrine levels, appetite, libido, memory, stress, or other cognitive qualities.

IL-21 and IL-22 polypeptides or polynucleotides may also be used as a food additive or preservative, such as to increase or decrease storage capabilities, fat content, lipid, protein, carbohydrate, vitamins, minerals, cofactors or other nutritional components.

Having generally described the invention, the same will be more readily understood by reference to the following examples, which are provided by way of illustration and are not intended as limiting.

### Examples

In the case where the full-length IL-21 and the partial IL-22 are not specifically mentioned, specific details are provided in the following examples only for the partial-length IL-21 molecules of the present invention. However, the examples can also be easily performed for the full-length IL-21 and the full-length or partial-length IL-22 molecules of the present invention by using the details provided for the partial IL-21 and substituting appropriate nucleotides or amino acid residues of the full-length IL-21, the full-length or partial-length IL-22, and/or any deletion mutations or other variants of either IL-21 or IL-22, for example, in the design of suitable PCR primers, and the like. The use or applicability of another IL-21 or IL-22 in place of the IL-21 exemplified below is thus contemplated in each of the following examples. When provided with the nucleotide and amino acid sequences of IL-21 (SEQ ID NO:1, SEQ ID NO:2, SEQ ID NO:28, and SEQ ID NO:29) and IL-22 (SEQ ID NO:3, SEQ ID NO:4, SEQ ID NO:30, and SEQ ID NO:31) of the present invention, one of ordinary skill in the art could easily perform the following examples with the intent of isolating or further characterizing or manipulating another IL-21 or IL-22 in place of the IL-21 shown in the Examples below.

### Example 1: Isolation of the IL-21 and IL-22 cDNA Clones From the Deposited Samples.

The cDNAs encoding the partial IL-21 and IL-22 molecules are each inserted into the *Eco* RI and *Xho* I restriction sites of the multiple cloning site of pBluescript. pBluescript contains an ampicillin resistance gene and may be transformed into *E. coli* strain DH10B, available from Life Technologies (see, for instance, Gruber, C. E., et al., *Focus 15:*59 (1993)).

Two approaches can be used to isolate IL-21 from the deposited sample. First, a specific polynucleotide of SEQ ID NO:1 with 30-40 nucleotides is synthesized using an Applied Biosystems DNA synthesizer according to the sequence reported. The oligonucleotide is labeled, for instance, with ³²P-gamma-ATP using T4 polynucleotide kinase and purified according to routine methods (e.g., Maniatis, *et al*., Molecular Cloning: A Laboratory Manual, Cold Spring Harbor Press, Cold Spring, NY (1982)). The plasmid mixture is transformed into a suitable host (such as XL-1 Blue (Stratagene)) using techniques known to those of skill in the art, such as those provided by the vector supplier or in related publications or patents. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook, *et al*., Molecular Cloning: A Laboratory Manual, 2nd Edit., (1989), Cold Spring Harbor Laboratory Press, pages 1.93 to 1.104), or other techniques known to those of skill in the art.

Alternatively, two primers of 17-20 nucleotides derived from both ends of the SEQ ID NO:1 (i.e., within the region of SEQ ID NO:1 bounded by the 5' and 3' nucleotides of the clone) are synthesized and used to amplify the IL-21 cDNA using the deposited cDNA plasmid as a template. The polymerase chain reaction is carried out under routine conditions, for instance, in 25 microliters of reaction mixture with 0.5 micrograms of the above cDNA template. A convenient reaction mixture is 1.5-5 mM MgCl₂, 0.01% (w/v) gelatin, 20 micromolar each of dATP, dCTP, dGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of the IL-21 gene which may not be present in the deposited clone. These methods include, but are not limited to, filter probing, clone enrichment using specific probes, and protocols similar or identical to 5' and 3' RACE protocols which are well known in the art. For instance, a method similar to 5' RACE is available for generating the missing 5' end of a desired full-length transcript (Fromont-Racine, *et al., Nucl. Acids Res.* **21**(7):1683-1684 (1993)).

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the IL-21 gene of interest is used to PCR amplify the 5' portion of the IL-21 full-length gene. This amplified product may then be sequenced and used to generate the full length gene.

This above method starts with total RNA isolated from the desired source, although poly-A+ RNA can be used. The RNA preparation can then be treated with a phosphatase, if necessary, to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNA. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the IL-21 gene.

### Example 2: Isolation of IL-21 Genomic Clones.

A human genomic P1 library (Genomic Systems, Inc.) is screened by PCR using primers selected for the cDNA sequence corresponding to SEQ ID NO:1., according to the method described in Example 1 (see also, Sambrook, *et al*., *supra*).

### Example 3: Tissue Distribution of IL-21.

Tissue distribution of mRNA expression of IL-21 is determined using protocols for Northern blot analysis, described by, among others, Sambrook and colleagues (*supra*). For example, an IL-21 probe produced by the method described in Example 1 is labeled with ³²P using the rediprime™ DNA labeling system (Amersham Life Science), according to manufacturer's instructions. After labeling, the probe is purified using a CHROMA SPIN-100™ column (Clontech Laboratories, Inc.), according to manufacturer's protocol number PT1200-1. The purified labeled probe is then used to examine various human tissues for mRNA expression.

Multiple Tissue Northern (MTN) blots containing various human tissues (H) or human immune system (IM) tissues (Clontech) are examined with the labeled probe using ExpressHyb™ hybridization solution (Clontech) according to manufacturer's protocol number PT1190-1. Following hybridization and washing, the blots are mounted and exposed to film at -70°C overnight, and the films developed according to standard procedures.

Using essentially the above-prescribed protocol, Northern blot analyses were performed to determine the expression pattern of IL-21 and IL-22. In the case of IL-21, very light signals of 1.8 and 3.0 kb were detected in skeletal muscle, and signals of indeterminate sizes were detected in fetal lung and fetal kidney. In the case of IL-22, a major message of 2.4 kb was detected in conjunction with a minor band in all brain tissues examined, and was also detected to a lesser extent in skeletal muscle, heart, testis, spinal cord, bone marrow, small intestine, kidney, and lung. A minor band of 4.4 kb was also detected in skeletal muscle.

### Example 4: Chromosomal Mapping of IL-21.

An oligonucleotide primer set is designed according to the sequence at the 5' end of SEQ ID NO:1. This primer preferably spans about 100 nucleotides. This primer set is then used in a polymerase chain reaction under the following set of conditions 30 seconds, 95°C; 1 minute, 56°C; 1 minute, 70°C. This cycle is repeated 32 times followed by one 5 minute cycle at 70°C. Human, mouse, and hamster DNA is used as template in addition to a somatic cell hybrid panel containing individual chromosomes or chromosome fragments (Bios, Inc). The reactions are analyzed on either 8% polyacrylamide gels or 3.5 % agarose gels. Chromosome mapping is determined by the presence of an approximately 100 bp PCR fragment in the particular somatic cell hybrid.

### Example 5: Bacterial Expression of IL-21.

An IL-21 polynucleotide encoding an IL-21 polypeptide of the invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as *Bam* HI and *Hin* dIII, at the 5' end of the primers in order to clone the amplified product into the expression vector. For example, *Bam* HI and *Hin* dIII correspond to the restriction enzyme sites on the bacterial expression vector pQE-9 (Qiagen Inc., Chatsworth, CA). This plasmid vector encodes antibiotic resistance (Amp^{R}), a bacterial origin of replication (ori), an IPTG-regulatable promoter/operator (P/O), a ribosome binding site (RBS), a 6-histidine tag (6-His), and restriction enzyme cloning sites.

Specifically, to clone the mature domain of the IL-21 protein in a bacterial vector, the 5' primer has the sequence 5'-GAT CGC GGA TCC GAC ACG GAT GAG GAC CGC TAT CCA CAG AAG CTG-3' (SEQ ID NO:9) containing the underlined *Bam* HI restriction site followed several nucleotides of the amino terminal coding sequence of the mature IL-21 sequence in SEQ ID NO:1. One of ordinary skill in the art would appreciate, of course, that the point in the protein coding sequence where the 5' primer begins may be varied to amplify a DNA segment encoding any desired portion of the complete IL-21 protein shorter or longer than the mature form of the protein. The 3' primer has the sequence 5'-CCC AAG CTT TCA CAC TGA ACG GGG CAG CAC GCA GGT GCA GC-3' (SEQ ID NO:10) containing the underlined Hin dIII restriction site followed by a number nucleotides complementary to the 3' end of the coding sequence of the IL-21 DNA sequence of SEQ ID NO:1.

The pQE-9 vector is digested with *Bam* HI and *Hin* dIII and the amplified fragment is ligated into the pQE-9 vector maintaining the reading frame initiated at the bacterial RBS. The ligation mixture is then used to transform the *E. coli* strain M15/rep4 (Qiagen, Inc.) which contains multiple copies of the plasmid pREP4, which expresses the lacI repressor and also confers kanamycin resistance (Kan^{R}). Transformants are identified by their ability to grow on LB plates and colonies are selected which are resistant to both ampicillin and kanamycin. Plasmid DNA is isolated and confirmed by restriction analysis.

Clones containing the desired constructs are grown overnight (O/N) in liquid culture in LB media supplemented with both Amp (100 µg/ml) and Kan (25 µg/ml). The O/N culture is used to inoculate a large culture at a ratio of 1:100 to 1:250. The cells are grown to an optical density 600 (O.D.₆₀₀) of between 0.4 and 0.6. IPTG (Isopropyl-B-D-thiogalacto pyranoside) is then added to a final concentration of 1 mM. IPTG induces by inactivating the lacI repressor, clearing the promoter/operator leading to increased gene expression.

Cells are grown for an additional 3 to 4 hours. Cells are then harvested by centrifugation (20 mins at 6000 X *g*). The cell pellet is solubilized in the chaotropic agent 6 M Guanidine-HCl by stirring for 3-4 hours at 4°C. The cell debris is removed by centrifugation, and the supernatant containing the polypeptide is loaded onto a nickel-nitrilo-tri-acetic acid ("Ni-NTA") affinity resin column (QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified IL-21 protein is then renatured by dialyzing it against phosphate-buffered saline (PBS) or 50 mM Na-acetate, pH 6 buffer plus 200 mM NaCl. Alternatively, the IL-21 protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified IL-21 protein is stored at 4°C or frozen at -80°C.

In addition to the above expression vector, the present invention further includes an expression vector comprising phage operator and promoter elements operatively linked to an IL-21 polynucleotide, called pHE4a (ATCC Accession Number 209645, deposited February 25, 1998). This vector contains: (1) a neomycin phosphotransferase gene as a selection marker, (2) an *E. coli* origin of replication, (3) a T5 phage promoter sequence, (4) two lac operator sequences, (5) a Shine-Delgarno sequence, and (6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with *Nde* I and *Xba* I, *Bam* HI, *Xho* I, or *Asp* 718, running the restricted product on a gel, and isolating the larger fragment (the stuffer fragment should be about 310 base pairs). The DNA insert is generated according to the PCR protocol described in Example 1, using PCR primers which encode restriction sites for *Nde* I (5' primer) and *Nde* I and *Xba* I, *Bam* HI, *Xho* I, or *Asp* 718 (3' primer). The PCR insert is gel purified and restricted with compatible enzymes. The insert and vector are ligated according to standard protocols.

The engineered vector could easily be substituted in the above protocol to express protein in a bacterial system.

### Example 6: Purification of IL-21 Polypeptide from an Inclusion Body.

The following alternative method can be used to purify IL-21 polypeptide expressed in *E coli* when it is present in the form of inclusion bodies. Unless otherwise specified, all of the following steps are conducted at 4-10°C.

Upon completion of the production phase of the *E. coli* fermentation, the cell culture is cooled to 4-10°C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 x g centrifugation for 15 min., the pellet is discarded and the polypeptide containing supernatant is incubated at 4°C overnight to allow further GuHCl extraction.

Following high speed centrifugation (30,000 x *g*) to remove insoluble particles, the GuHCl solubilized protein is refolded by quickly mixing the GuHCl extract with 20 volumes of buffer containing 50 mM sodium, pH 4.5, 150 mM NaCl, 2 mM EDTA by vigorous stirring. The refolded diluted protein solution is kept at 4°C without mixing for 12 hours prior to further purification steps.

To clarify the refolded polypeptide solution, a previously prepared tangential filtration unit equipped with 0.16 micrometer membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the IL-21 polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant A₂₈₀ monitoring of the effluent. Fractions containing the polypeptide (determined, for instance, by 16% SDS-PAGE) are then pooled.

The resultant IL-21 polypeptide should exhibit greater than 95% purity after the above refolding and purification steps. No major contaminant bands should be observed from Commassie blue stained 16% SDS-PAGE gel when 5 micrograms of purified protein is loaded. The purified IL-21 protein can also be tested for endotoxin/LPS contamination, and typically the LPS content is less than 0.1 ng/ml according to LAL assays.

### Example 7: Cloning and Expression of IL-21 in a Baculovirus Expression System.

In this example, the plasmid shuttle vector pA2 is used to insert IL-21 polynucleotide into a baculovirus to express IL-21. This expression vector contains the strong polyhedrin promoter of the *Autographa californica* nuclear polyhedrosis virus (AcMNPV) followed by convenient restriction sites such as *Bam* HI, *Xba* I and *Asp* 718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned IL-21 polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, by Luckow and colleagues (*Virology* **170:**31-39 (1989)).

Specifically, the IL-21 cDNA sequence contained in the deposited clone, including the AUG initiation codon and any naturally associated leader sequence, is amplified using the PCR protocol described in Example 1. If the naturally occurring signal sequence is used to produce the secreted protein, the pA2 vector does not need a second signal peptide. However, since the predicted naturally occurring signal peptides of IL-21 and IL-22 are not known, the vector can be modified (now designated pA2GP) to include a baculovirus leader sequence, using the standard methods described by Summers and coworkers ("A Manual of Methods for Baculovirus Vectors and Insect Cell Culture Procedures," Texas Agricultural Experimental Station Bulletin No. 1555 (1987)).

More specifically, the cDNA sequence encoding the full-length IL-21 protein in the deposited clone is amplified using PCR oligonucleotide primers corresponding to the 5' and 3' sequences of the gene. The 5' primer has the sequence 5'-CGC CGC GGA TCC *GCC ATC* CGC ACG AGT GGA CAC GG-3' (SEQ ID NO:11) containing the *Bam* HI restriction enzyme site, an efficient signal for initiation of translation in eukaryotic cells (shown in the primer sequence in italics; Kozak, M., *J. Mol. Biol*. **196:**947-950 (1987)), a "C" residue to preserve the reading frame, and 16 nucleotides of the sequence of the complete IL-21 protein shown in Figure 1. The 3' primer has the sequence 5'-CGC GGT ACC CAC TGA ACG GGG CAG CAC GC-3' (SEQ ID NO:12) containing the *Asp* 718 restriction site followed by 20 nucleotides complementary to the 3' noncoding sequence in Figure 1.

The amplified fragment is isolated from a 1 % agarose gel using a commercially available kit ("Geneclean," BIO 101 Inc., La Jolla, CA). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, CA).

The fragment and the dephosphorylated plasmid are ligated together with T4 DNA ligase. *E*. *coli* HB 101 or other suitable *E*. *coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five micrograms of a plasmid containing the polynucleotide is co-transfected with 1.0 µg of a commercially available linearized baculovirus DNA ("BaculoGold™ baculovirus DNA", Pharmingen, San Diego, CA), using the lipofection method described by Felgner and colleagues (*Proc. Natl. Acad. Sci. USA* **84:**7413-7417 (1987)). One µg of BaculoGold™ virus DNA and 5 µg of the plasmid are mixed in a sterile well of a microtiter plate containing 50 µl of serum-free Grace's medium (Life Technologies Inc., Gaithersburg, MD). Afterwards, 10 µl Lipofectin plus 90 µl Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27°C. The transfection solution is then removed from the plate and 1 ml of Grace's insect medium supplemented with 10% fetal calf serum is added. Cultivation is then continued at 27°C for four days.

After four days the supernatant is collected and a plaque assay is performed, as described by Summers and Smith (*supra*). An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 µl of Grace's medium and the suspension containing the recombinant baculovirus is used to infect Sf9 cells seeded in 35 mm dishes. Four days later the supernatants of these culture dishes are harvested and then they are stored at 4°C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 µCi of ³⁵S-methionine and 5 µCi ³⁵S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced IL-21 protein.

### Example 8: Expression of IL-21 in Mammalian Cells.

IL-21 polypeptide can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLV-I, HIV-1 and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, Hela, 293, H9 and Jurkat cells, mouse NIH3T3 and C127 cells, Cos 1, Cos 7 and CV1, quail QC 1-3 cells, mouse L cells and Chinese hamster ovary (CHO) cells.

Alternatively, IL-21 polypeptide can be expressed in stable cell lines containing the IL-21 polynucleotide integrated into a chromosome. The co-transfection with a selectable marker such as *dhfr, gpt*, neomycin or hygromycin allows the identification and isolation of the transfected cells.

The transfected IL-21 gene can also be amplified to express large amounts of the encoded protein. The DHFR (dihydrofolate reductase) marker is useful in developing cell lines that carry several hundred or even several thousand copies of the gene of interes (see, e.g., Alt, F. W., *et al., J. Biol. Chem*. **253:**1357-1370 (1978); Hamlin, J. L. and Ma, *C., Biochem. et Biophys. Acta*, **1097:**107-143 (1990); Page, M. J. and Sydenham, M. *A., Biotechnology* **9:**64-68 (1991)). Another useful selection marker is the enzyme glutamine synthase (GS; Murphy, *et al., Biochem. J.* **227:**277-279 (1991); Bebbington, *et al., Bio*/*Technology* **10:**169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen, *et al*., *Mol. Cell. Biol*., 438-447 (March, 1985)) plus a fragment of the CMV-enhancer (Boshart, *et al., Cell* **41:**521-530 (1985)). Multiple cloning sites, e.g., with the restriction enzyme cleavage sites *Bam* HI, *Xba* I and *Asp* 718, facilitate the cloning of IL-21. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

IL-21 polynucleotide is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence (see, e.g., WO 96/34891).

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five µg of the expression plasmid pC6 is cotransfected with 0.5 µg of the plasmid pSVneo using lipofectin (Felgner, et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (for example, 50 nM, 100 nM, 200 nM, 400 nM, 800 nM). Clones growing at the highest concentrations of methotrexate are then transferred to new 6-well plates containing even higher concentrations of methotrexate (1 µM, 2 µM, 5 µM, 10 mM, 20 mM). The same procedure is repeated until clones are obtained which grow at a concentration of 100-200 µM. Expression of IL-21 is analyzed, for instance, by SDS-PAGE and Western blot or by reverse phase HPLC analysis.

### Example 9: Protein Fusions of IL-21.

IL-21 polypeptides are preferably fused to other proteins. These fusion proteins can be used for a variety of applications. For example, fusion of IL-21 polypeptides to His-tag, HA-tag, protein A, IgG domains, and maltose binding protein facilitates purification (see Example 5; see also EP A 394,827; Traunecker, *et al., Nature* **331:**84-86 (1988).) Similarly, fusion to IgG-1, IgG-3, and albumin increases the halflife time *in vivo*. Nuclear localization signals fused to IL-21 polypeptides can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the *Bam* HI cloning site. Note that the 3' *Bam* HI site should be destroyed. Next, the vector containing the human Fc portion is again restricted with *Bam* HI, linearizing the vector, and IL-21 polynucleotide, isolated by the PCR protocol described in Example 1, is ligated into this *Bam* HI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence (see, e.g., WO 96/34891).

### Human IgG Fc region (SEQ ID NO:13):

GGGATCCGGAGCCCAAATCTTCTGACAAAACTCACACATGCCCACCGTGCCCAGCACCTGAATTCGAGGGTGCACCGTC
AGTCTTCCTCTTCCCCCCAAAACCCAAGGACACCCTCATGATCTCCCGGACTCCTGAGGTCACATGCGTGGTGGTGGACGTAAGCC
ACGAAGACCCTGAGGTCAAGTTCAACTGGTACGTGGACGGCGTGGAGGTGCATAATGCCAAGACAAAGCCGCGGGAGGAGCAGTAC
AACAGCACGTACCGTGTGGTCAGCGTCCTCACCGTCCTGCACCAGGACTGGCTGAATGGCAAGGAGTACAAGTGCAAGGTCTCCAA
CAAAGCCCTCCCAACCCCCATCGAGAAAACCATCTCCAAAGCCAAAGGGCAGCCCCGAGAACCACAGGTGTACACCCTGCCCCCAT
CCCGGGATGAGCTGACCAAGAACCAGGTCAGCCTGACCTGCCTGGTCAAAGGCTTCTATCCAAGCGACATCGCCGTGGAGTGGGAG
AGCAATGGGCAGCCGGAGAACAACTACAAGACCACGCCTCCCGTGCTGGACTCCGACGGCTCCTTCTTCCTCTACAGCAAGCTCAC
CGTGGACAAGAGCAGGTGGCAGCAGGGGAACGTCTTCTCATGCTCCGTGATGCATGAGGCTCTGCACAACCACTACACGCAGAAGA
GCCTCTCCCTGTCTCCGGGTAAATGAGTGCGACGGCCGCGACTCTAGAGGAT

### Example 10: Production of an Antibody.

The antibodies of the present invention can be prepared by a variety of methods (see, Current Protocols, Chapter 2). For example, cells expressing IL-21 is administered to an animal to induce the production of sera containing polyclonal antibodies. In a preferred method, a preparation of IL-21 protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology (Kohler, *et al., Nature* **256:**495 (1975); *Kohler, et al., Eur. J. Immunol*. **6:**511 (1976); Kohler, *et al., Eur. J. Immunol*. **6:**292 (1976); Hammerling, *et al*., in: Monoclonal Antibodies and T-Cell Hybridomas, Elsevier, N.Y., pp. 563-681 (1981)). In general, such procedures involve immunizing an animal (preferably a mouse) with IL-21 polypeptide or, more preferably, with a secreted IL-21 polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands and colleagues (*Gastroenterology* **80:**225-232 (1981)). The hybridoma cells obtained through such a selection are then assayed to identify clones which secrete antibodies capable of binding the IL-21 polypeptide.

Alternatively, additional antibodies capable of binding to IL-21 polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the IL-21 protein-specific antibody can be blocked by IL-21. Such antibodies comprise anti-idiotypic antibodies to the IL-21 protein-specific antibody and can be used to immunize an animal to induce formation of further IL-21 protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted IL-21 protein-binding fragments can be produced through the application of recombinant DNA technology or through synthetic chemistry.

For *in vivo* use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art (see, for review, Morrison, *Science* **229:**1202 (1985); Oi, *et al*., *BioTechniques* **4:**214 (1986); Cabilly, *et al*., U.S. Patent No. 4,816,567; Taniguchi, *et al*., EP 171496; Morrison, *et al*., EP 173494; Neuberger, *et al*., WO 8601533; Robinson, *et al*., WO 8702671; Boulianne, *et al*., *Nature* **312:**643 (1984); Neuberger, *et al*., *Nature* **314:**268 (1985)).

### Example 11: Production Of IL-21 Protein For High-Throughput Screening Assays.

The following protocol produces a supernatant containing IL-21 polypeptide to be tested. This supernatant can then be used in the screening assays described subsequently in Examples 13-20.

First, dilute poly-D-lysine (644 587 Boehringer-Mannheim) stock solution (1mg/ml in PBS) 1:20 in PBS (Phosphate Buffered Saline; w/o calcium or magnesium 17-516F Biowhittaker) for a working solution of 50 µg/ml. Add 200 µl of this solution to each well (24 well plates) and incubate at RT for 20 minutes. Be sure to distribute the solution over each well (note: a 12-channel pipetter may be used with tips on every other channel). Aspirate the poly-D-lysine solution and rinse with 1 ml PBS. The PBS should remain in the well until just prior to plating the cells and plates may be poly-lysine coated in advance for up to two weeks.

Plate 293T cells (do not carry cells past P+20) at 2 x 10⁵ cells/well in 0.5 ml DMEM (Dulbecco's Modified Eagle Medium) supplemented with 4.5 G/L glucose, L-glutamine (12-604F Biowhittaker)), 10% heat inactivated FBS (14-503F Biowhittaker), and 1x Penstrep (17-602E Biowhittaker). Let the cells grow overnight.

Following overnight incubation, mix together in a sterile solution basin: 300 µl Lipofectamine (18324-012 Gibco/BRL) and 5ml Optimem I (31985070 Gibco/BRL) in each well of a 96-well plate. With a small volume multi-channel pipetter, aliquot approximately 2 µg of an expression vector containing a polynucleotide insert, produced by the methods described in Examples 8 or 9, into an appropriately labeled 96-well round bottom plate. With a multi-channel pipetter, add 50 µl of the Lipofectamine/Optimem I mixture to each well. Pipette up and down gently to mix. Incubate at RT for 15-45 minutes. After about 20 minutes, use a multi-channel pipetter to add 150 µl Optimem I to each well. As a control, one plate of vector DNA lacking an insert should be transfected with each set of transfections.

Preferably, the transfection should be performed by simultaneously performing the following tasks in a staggered fashion. Thus, hands-on time is cut in half, and the cells are not excessively incubated in PBS. First, person A aspirates the media from four 24-well plates of cells, and then person B rinses each well with 0.5-1ml PBS. Person A then aspirates the PBS rinse, and person B, using a 12-channel pipetter with tips on every other channel, adds the 200 µl of DNA/Lipofectamine/Optimem I complex to the odd wells first, then to the even wells, to each row on the 24-well plates. Plates are then incubated at 37°C for 6 hours.

While cells are incubating, the appropriate media is prepared: either 1% BSA in DMEM with 1x penstrep, or HGS CHO-5 media (116.6 mg/L of CaCl₂ (anhyd); 0.00130 mg/L CuSO₄-5H₂O; 0.050 mg/L of Fe(NO₃)₃-9H₂O; 0.417 mg/L of FeSO₄-7H₂O; 311.80 mg/L of KCl; 28.64 mg/L of MgCl₂; 48.84 mg/L of MgSO₄; 6995.50 mg/L of NaCl; 2400.0 mg/L of NaHCO₃; 62.50 mg/L of NaH₂PO₄-H₂O; 71.02 mg/L of Na₂HPO₄; .4320 mg/L of ZnSO₄-7H₂O; .002 mg/L of Arachidonic Acid; 1.022 mg/L of Cholesterol; 0.070 mg/L of D-L-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L-Alanine; 147.50 mg/ml of L-Arginine-HCL; 7.50 mg/ml of L-Asparagine-H₂O; 6.65 mg/ml of L-Aspartic Acid; 29.56 mg/ml of L-Cystine-2HCL-H₂O; 31.29 mg/ml of L-Cystine-2HCl; 7.35 mg/ml of L-Glutamic Acid; 365.0 mg/ml of L-Glutamine; 18.75 mg/ml of Glycine; 52.48 mg/ml of L-Histidine-HCL-H₂O; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101.05 mg/ml of L-Threonine; 19.22 mg/ml of L-Tryptophan; 91.79 mg/ml of L-Tryrosine-2Na-2H₂O; and 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridoxal HCL; 0.031 mg/L of Pyridoxine HCL; 0.319 mg/L of Riboflavin; 3.17 mg/L of Thiamine HCL; 0.365 mg/L of Thymidine; 0.680 mg/L of Vitamin B₁₂; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethanolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; 10 mg/L of Methyl-B-Cyclodextrin complexed with Retinal Acetate. Adjust osmolarity to 327 mOsm) with 2mm glutamine and 1x penstrep. (BSA (81-068-3 Bayer) 100gm dissolved in 1L DMEM for a 10% BSA stock solution). Filter the media and collect 50 µl for endotoxin assay in 15ml polystyrene conical.

The transfection reaction is terminated, again, preferably by two people, at the end of the incubation period. Person A aspirates the transfection media, while person B adds 1.5 ml of the appropriate media to each well. Incubate at 37°C for 45 or 72 hours, depending on the media used (1%BSA for 45 hours or CHO-5 for 72 hours).

On day four, using a 300 µl multichannel pipetter, aliquot 600 µl in one 1ml deep well plate and the remaining supernatant into a 2 ml deep well. The supernatants from each well can then be used in the assays described in Examples 13-20.

It is specifically understood that when activity is obtained in any of the assays described below using a supernatant, the activity originates from either the IL-21 polypeptide directly (e.g., as a secreted protein) or by IL-21 inducing expression of other proteins, which are then secreted into the supernatant. Thus, the invention further provides a method of identifying the protein in the supernatant characterized by an activity in a particular assay.

### Example 12: Construction of GAS Reporter Construct.

One signal transduction pathway involved in the differentiation and proliferation of cells is called the Jaks-STATs pathway. Activated proteins in the Jaks-STATs pathway bind to gamma activation site ("GAS") elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stat1 and Stat3 are present in many cell types, as is Stat2 (as response to IFN-alpha is widespread). Stat4 is more restricted and is not in many cell types though it has been found in T-helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below (adapted from review by Schidler and Darnell, *Ann. Rev. Biochem*. **64:**621-51 (1995))). A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-alpha, IFN-gamma, and IL-10. The Class 1 receptors share a conserved cysteine motif (a set of four conserved cysteines and one tryptophan) and a WSXWS motif (a membrane proximal region encoding Trp-Ser-Xxx-Trp-Ser (where "Xxx" represents any amino acid; SEQ ID NO:14)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway (see Table below). Thus, by using GAS elements linked to reporter molecules, activators of the Jaks-STATs pathway can be identified.

| | JAKs | | | | STATS | GAS(elements) or ISRE |
|---|---|---|---|---|---|---|
| Ligand | tyk2 | Jak1 | Jak2 | Jak3 | | |
| IFN family | | | | | | |
| IFN-alpha/beta | + | + | - | - | 1,2,3 | ISRE |
| IFN-gamma | | + | + | - | 1 | GAS (IRF1>Lys6>IFP) |
| II-10 | + | ? | ? | - | 1,3 | |

| gp 130 family | | | | | | |
|---|---|---|---|---|---|---|
| IL-6 (Pleiotrohic) | + | + | + | ? | 1,3 | GAS (IRF1>Lys6>IFP) |
| Il-11(Pleiotrohic) | ? | + | ? | ? | 1,3 | |
| OnM(Pleiotrohic) | ? | + | + | ? | 1,3 | |
| LIF(Pleiotrohic) | ? | + | + | ? | 1,3 | |
| CNTF(Pleiotrohic) | -/+ | + | + | ? | 1,3 | |
| G-CSF(Pleiotrohic) | ? | + | ? | ? | 1,3 | |
| IL-12(Pleiotrohic) | + | - | + | + | 1,3 | |

| g-C family | | | | | | |
|---|---|---|---|---|---|---|
| IL-2 (lymphocytes) | - | + | - | + | 1,3,5 | GAS |
| IL-4 (lymph/myeloid) - | | + | - | + | 6 | GAS (IRF1 = IFP >>Ly6)(IgH) |
| IL-7 (lymphocytes) | - | + | - | + | 5 | GAS |
| IL-9 (lymphocytes) | - | + | - | + | 5 | GAS |
| IL-13 (lymphocyte) | - | + | ? | ? | 6 | GAS |
| IL-15 | ? | + | ? | + | 5 | GAS |

| gp 140 family | | | | | | |
|---|---|---|---|---|---|---|
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |

| Growth hormone family | | | | | | |
|---|---|---|---|---|---|---|
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |

| Receptor Tyrosine Kinases | | | | | | |
|---|---|---|---|---|---|---|
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the biological assays described in Examples 13-14, a PCR based strategy is employed to generate a GAS-SV40 promoter sequence. The 5' primer contains four tandem copies of the GAS-binding site found in the IRF1 promoter and previously demonstrated to bind STATs upon induction with a range of cytokines (Rothman, *et al*., *Immunity* **1:**457-468 (1994)), although other GAS or ISRE elements can be used instead. The 5' primer also contains 18 bp of sequence complementary to the SV40 early promoter sequence and is flanked with an *Xho* I restriction site. The sequence of the 5' primer is: 5'-GCG CCT CGA GAT TTC CCC GAA ATC TAG ATT TCC CCG AAA TGA TTT CCC CGA AAT GAT TTC CCC GAA ATA TCT GCC ATC TCA ATT AG-3' (SEQ ID NO: 15).

The downstream primer is complementary to the SV40 promoter and is flanked with a *Hin* dIII site: 5'-GCG GCA AGC TTT TTG CAA AGC CTA GGC-3' (SEQ ID NO:16).

PCR amplification is performed using the SV40 promoter template present in the B-gal:promoter plasmid obtained from Clontech. The resulting PCR fragment is digested with *Xho* I and *Hin* dIII and subcloned into BLSK2- (Stratagene). Sequencing with forward and reverse primers confirms that the insert contains the following sequence:

CTCGAGATTTCCCCGAAATCTAGATTTCCCCGAAATGATTTCCCCGAAATGATTTCCCCGAAATATCTGCCATCTCAAT
TAGTCAGCAACCATAGTCCCGCCCCTAACTCCGCCCATCCCGCCCCTAACTCCGCCCAGTTCCGCCCATTCTCCGCCCCATGGCTG
ACTAATTTTTTTTATTTATGCAGAGGCCGAGGCCGCCTCGGCCTCTGAGCTATTCCAGAAGTAGTGAGGAGGCTTTTTTGGAGGCC
TAGGCTTTTGCAAAAAGCTT (SEQ ID NO:17).

With this GAS promoter element linked to the SV40 promoter, a GAS:SEAP2 reporter construct is next engineered. Here, the reporter molecule is a secreted alkaline phosphatase, or "SEAP". Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using *Hin* dIII and *Xho* I, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using *Sal* I and *Not* I, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NF-kappaB and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-kappaB/EGR, GAS/NF-kappaB, Il-2/NFAT, or NF-kappaB/GAS). Similarly, other cell lines can be used to test reporter construct activity, such as HeLa (epithelial), HUVEC (endothelial), Reh (B-cell), Saos-2 (osteoblast), HUVAC (aortic), or Cardiomyocyte.

### Example 13: High-Throughput Screening Assay for T-cell Activity.

The following protocol is used to assess T-cell activity of IL-21 by determining whether IL-21 supernatant proliferates and/or differentiates T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th 1 helper cells. In order to generate stable cell lines, approximately 2 million Jurkat cells are transfected with the GAS-SEAP/neo vector using DMRIE-C (Life Technologies: transfection procedure described below). The transfected cells are seeded to a density of approximately 20,000 cells per well and transfectants resistant to 1 mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 µl of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 µg of plasmid DNA in a T25 flask. Add 2.5 ml OPTI-MEM containing 50 µl of DMRIE-C and incubate at room temperature for 15-45 min.

During the incubation period, count cell concentration, spin down the required number of cells (10⁷ per transfection), and resuspend in OPTI-MEM to a final concentration of 10⁷ cells/ml. Then add 1ml of 1 x 10⁷ cells in OPTI-MEM to T25 flask and incubate at 37°C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing IL-21 polypeptides or IL-21 induced polypeptides as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are required.

Transfer the cells to a triangular reservoir boat, in order to dispense the cells into a 96 well dish, using a 12 channel pipette. Using a 12 channel pipette, transfer 200 µl of cells into each well (therefore adding 100, 000 cells per well).

After all the plates have been seeded, 50 µl of the supernatants are transferred directly from the 96 well plate containing the supernatants into each well using a 12 channel pipette. In addition, a dose of exogenous interferon gamma (0.1, 1.0, 10 ng) is added to wells H9, H10, and H11 to serve as additional positive controls for the assay.

The 96 well dishes containing Jurkat cells treated with supernatants are placed in an incubator for 48 hrs (note: this time is variable between 48-72 hrs). 35 µl samples from each well are then transferred to an opaque 96 well plate using a 12 channel pipette. The opaque plates should be covered (using sellophene covers) and stored at -20°C until SEAP assays are performed according to Example 17. The plates containing the remaining treated cells are placed at 4°C and serve as a source of material for repeating the assay on a specific well if desired.

As a positive control, 100 Unit/ml interferon gamma can be used which is known to activate Jurkat T cells. Over 30 fold induction is typically observed in the positive control wells.

### Example 14: High-Throughput Screening Assay Identifying Myeloid Activity.

The following protocol is used to assess myeloid activity of IL-21 by determining whether IL-21 proliferates and/or differentiates myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda, *et. al., Cell Growth & Differentiation*, 5:259-265 (1994)) is used. First, harvest 2x10⁷ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 µg GAS-SEAP2 plasmid DNA, 140 mM NaCl, 5 mM KCl, 375 uM Na₂HPO₄7H₂O, 1 mM MgCl₂, and 675 uM CaCl₂. Incubate at 37°C for 45 min.

Wash the cells with RPMI 1640 medium containing 10% FBS and then resuspend in 10 ml complete medium and incubate at 37°C for 36 hr.

The GAS-SEAP/U937 stable cells are obtained by growing the cells in 400 µg/ml G418. The G418-free medium is used for routine growth but every one to two months, the cells should be re-grown in 400 ug/ml G418 for couple of passages.

These cells are tested by harvesting 1x10⁸ cells (this is enough for ten 96-well plates assay) and wash with PBS. Suspend the cells in 200 ml above described growth medium, with a final density of 5x10⁵ cells/ml. Plate 200 µl cells per well in the 96-well plate (or 1x10⁵ cells/well).

Add 50 µl of the supernatant prepared by the protocol described in Example 11. Incubate at 37°C for 48 to 72 hr. As a positive control, 100 U/ml interferon gamma can be used which is known to activate U937 cells. Over 30-fold induction is typically observed in the positive control wells. SEAP assay the supernatant according to the protocol described in Example 17.

### Example 15: High-Throughput Screening Assay Identifying Neuronal Activity.

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed by IL-21.

Particularly, the following protocol is used to assess neuronal activity in PC 12 cell lines. PC 12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR1 gene expression is activated during this treatment. Thus, by stably transfecting PC12 cells with a construct containing an EGR promoter linked to SEAP reporter, activation of PC 12 cells by IL-21 can be assessed.

The EGR/SEAP reporter construct can be assembled by the following protocol. The EGR-1 promoter sequence (nucleotides -633 to +1: Sakamoto, K., *et al*., *Oncogene* **6:**867-871 (1991)) can be PCR amplified from human genomic DNA using the following primers: (A) 5' Primer: 5'-GCG CTC GAG GGA TGA CAG CGA TAG AAC CCC GG-3' (SEQ ID NO:18) and (B) 3' Primer: 5'-GCG AAG CTT CGC GAC TCC CCG GAT CCG CCT C-3' (SEQ ID NO:19).

Using the GAS:SEAP/Neo vector produced in Example 12, EGR 1 amplified product can then be inserted into this vector. Linearize the GAS:SEAP/Neo vector using restriction enzymes *Xho* I and *Hin* dIII, removing the GAS/SV40 stuffer fragment. Digest the EGR1 amplified product with the same enzymes. Ligate the vector and the EGR1 promoter.

To prepare 96 well-plates for cell culture, 2 ml of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 µg/ml streptomycin on a precoated 10 cm tissue culture dish. A 1:4 split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 µg/ml G418. The G418-free medium is used for routine growth but every one to two months, the cells should be re-grown in 300 µg/ml G418 for several passages.

To assay for neuronal activity, a 10 cm plate with cells around 70 to 80% confluent is screened by removing the old medium. Wash the cells once with PBS. Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as 5x10⁵ cells/ml.

Add 200 µl of the cell suspension to each well of 96-well plate (equivalent to 1x10⁵ cells/well). Add 50 µl supernatant produced by Example 11, 37° C for 48 to 72 hr. As a positive control, a growth factor known to activate PC 12 cells through EGR can be used, such as 50 ng/µl of Neuronal Growth Factor (NGF). Over fifty-fold induction of SEAP is typically seen in the positive control wells. SEAP assay the supernatant according to Example 17.

### Example 16: High-Throughput Screening Assay for T-cell Activity.

NF-kappaB (Nuclear Factor kappaB) is a transcription factor activated by a wide variety of agents including the inflammatory cytokines IL-1 and TNF, CD30 and CD40, lymphotoxin-a and lymphotoxin-b, by exposure to LPS or thrombin, and by expression of certain viral gene products. As a transcription factor, NF-kappaB regulates the expression of genes involved in immune cell activation, control of apoptosis (NF-kappaB appears to shield cells from apoptosis), B- and T-cell development, anti-viral and antimicrobial responses, and multiple stress responses.

In non-stimulated conditions, NF-kappaB is retained in the cytoplasm with I-kappaB (Inhibitor kappaB). However, upon stimulation, I- kappaB is phosphorylated and degraded, causing NF-kappaB to shuttle to the nucleus, thereby activating transcription of target genes. Target genes activated by NF-kappaB include IL-2, IL-6, GM-CSF, ICAM-1 and class 1 MHC.

Due to its central role and ability to respond to a range of stimuli, reporter constructs utilizing the NF-kappaB promoter element are used to screen the supernatants produced in Example 11. Activators or inhibitors of NF-kappaB would be useful in treating diseases. For example, inhibitors of NF-kappaB could be used to treat those diseases related to the acute or chronic activation of NF-kappaB, such as rheumatoid arthritis.

To construct a vector containing the NF-kappaB promoter element, a PCR based strategy is employed. The upstream primer contains four tandem copies of the NF-kappaB binding site (5'-GGG GAC TTT CCC-3'; SEQ ID NO:20), 18 bp of sequence complementary to the 5' end of the SV40 early promoter sequence, and is flanked with an *Xho* I site: 5'-GCG GCC TCG AGG GGA CTT TCC CGG GGA CTT TCC GGG GAC TTT CCG GGA CTT TCC ATC CTG CCA TCT CAA TTA G-3' (SEQ ID NO:21).

The downstream primer is complementary to the 3' end of the SV40 promoter and is flanked with a *Hin* dIII site: 5'-GCG GCA AGC TTT TTG CAA AGC CTA GGC-3' (SEQ ID NO:22).

PCR amplification is performed using the SV40 promoter template present in the pB-gal:promoter plasmid obtained from Clontech. The resulting PCR fragment is digested with *Xho* I and *Hin* dIII and subcloned into BLSK2- (Stratagene). Sequencing with the T7 and T3 primers confirms the insert contains the following sequence:

5'-CTCGAGGGGACTTTCCCGGGGACTTTCCGGGGACTTTCCGGGACTTT
CCATCTGCCATCTCAATTAGTCAGCAACCATAGTCCCGCCCCTAACTCCGCCCA

TCCCGCCCCTAACTCCGCCCAGTTCCGCCCATTCTCCGCCCCATGGCTGACTAA
TTTTTTTTATTTATGCAGAGGCCGAGGCCGCCTCGGCCTCTGAGCTATTCCAGA
AGTAGTGAGGAGGCTTTTTTGGAGGCCTAGGCTTTTGCAAAAAGCTT-3' (SEQ
ID NO:23)

Next, replace the SV40 minimal promoter element present in the pSEAP2-promoter plasmid (Clontech) with this NF-kappaB/SV40 fragment using *Xho* I and *Hin* dIII. However, this vector does not contain a neomycin resistance gene, and therefore, is not preferred for mammalian expression systems.

In order to generate stable mammalian cell lines, the NF-kappaB/SV40/SEAP cassette is removed from the above NF-kappaB/SEAP vector using restriction enzymes *Sal* I and *Not* I, and inserted into a vector containing neomycin resistance. Particularly, the NF-kappaB/SV40/SEAP cassette was inserted into pGFP-1 (Clontech), replacing the GFP gene, after restricting pGFP-1 with *Sal* I and *Not* I.

Once NF-kappaB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF-a (0.1,1, 10 ng) is added to wells H9, H10, and H11, with a 5-10 fold activation typically observed.

### Example 17: Assay for SEAP Activity.

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 µl of 2.5x dilution buffer into Optiplates containing 35 µl of a supernatant. Seal the plates with a plastic sealer and incubate at 65°C for 30 min. Separate the Optiplates to avoid uneven heating.

Cool the samples to room temperature for 15 minutes. Empty the dispenser and prime with the Assay Buffer. Add 50 µl Assay Buffer and incubate at room temperature 5 min. Empty the dispenser and prime with the Reaction Buffer (see the table below). Add 50 µl Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluminescence indicates reporter activity.

**Table III:**

| **Reaction Buffer Formulation:** | | |
|---|---|---|
| # of plates (ml) | Rxn buffer diluent | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| . 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

### Example 18: High-Throughput Screening Assay Identifying Changes in Small Molecule Concentration and Membrane Permeability.

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-3, used here.

For adherent cells, seed the cells at 10,000-20,000 cells/well in a Co-star black 96-well plate with clear bottom. The plate is incubated in a CO₂ incubator for 20 hours. The adherent cells are washed two times in Biotek washer with 200 µl of HBSS (Hank's Balanced Salt Solution) leaving 100 ul of buffer after the final wash.

A stock solution of 1 mg/ml fluo-3 is made in 10% pluronic acid DMSO. To load the cells with fluo-3, 50 µl of 12 µg/ml fluo-3 is added to each well. The plate is incubated at 37°C in a CO₂ incubator for 60 min. The plate is washed four times in the Biotek washer with HBSS leaving 100 µl of buffer.

For non-adherent cells, the cells are spun down from culture media. Cells are re-suspended to 2-5x10⁶ cells/ml with HBSS in a 50-ml conical tube. Four µl of 1 mg/ml fluo-3 solution in 10% pluronic acid DMSO is added to each 1 ml of cell suspension. The tube is then placed in a 37°C water bath for 30-60 min. The cells are washed twice with HBSS, resuspended to 1x10⁶ cells/ml, and dispensed into a microplate, 100 µl/well. The plate is centrifuged at 1000 rpm for 5 min. The plate is then washed once in Denley CellWash with 200 µl, followed by an aspiration step to 100 µl final volume.

For a non-cell based assay, each well contains a fluorescent molecule, such as fluo-3. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event caused by the a molecule, either IL-21 or a molecule induced by IL-21, which has resulted in an increase in the intracellular Ca²⁺ concentration.

### Example 19: High-Throughput Screening Assay Identifying Tyrosine Kinase Activity.

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., *src*, *yes*, *lck*, *lyn*, *fyn*) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, identifying whether IL-21 or a molecule induced by IL-21 is capable of activating tyrosine kinase signal transduction pathways is of interest. Therefore, the following protocol is designed to identify such molecules capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4°C. Cell growth on these plates is assayed by seeding 5,000 cells/well in growth medium and indirect quantitation of cell number through use of alamar Blue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/well) and cultured overnight in complete medium. Cells are quiesced by incubation in serum-free basal medium for 24 hr. After 5-20 minutes, treatment with EGF (60ng/ml) or 50 µl of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4°C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4°C at 16,000 x *g*.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10 µl of 5 µM Biotinylated Peptide, then 10 µl ATP/Mg²⁺ (5 mM ATP/50 mM MgCl₂), then 10 µl of 5x Assay Buffer (40 mM imidazole hydrochloride, pH 7.3, 40 mM b-glycerophosphate, 1 mM EGTA, 100 mM MgCl₂, 5 mM MnCl₂, 0.5 mg/ml BSA), then 5 µl of Sodium Vanadate (1 mM), and then 5 µl of water. Mix the components gently and preincubate the reaction mix at 30°C for 2 min. Initial the reaction by adding 10 µl of the control enzyme or the filtered supernatant.

The tyrosine kinase assay reaction is then terminated by adding 10 µl of 120 mm EDTA and place the reactions on ice.

Tyrosine kinase activity is determined by transferring 50 µl aliquot of reaction mixture to a microtiter plate (MTP) module and incubating at 37°C for 20 min. This allows the streptavadin coated 96 well plate to associate with the biotinylated peptide. Wash the MTP module with 300 µl/well of PBS four times. Next add 75 µl of anti-phospotyrosine antibody conjugated to horse radish peroxidase(anti-P-Tyr-POD (0.5 µl/ml)) to each well and incubate at 37°C for one hour. Wash the well as above.

Next add 100 µl of peroxidase substrate solution (Boehringer Mannheim) and incubate at room temperature for at least 5 min (up to 30 min). Measure the absorbance of the sample at 405 nm by using ELISA reader. The level of bound peroxidase activity is quantitated using an ELISA reader and reflects the level of tyrosine kinase activity.

### Example 20: High-Throughput Screening Assay Identifying Phosphorylation Activity.

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1 ml of protein G (1 µg/ml) for 2 hr at room temp (RT). The plates are then rinsed with PBS and blocked with 3% BSA/PBS for 1 hr at RT. The protein G plates are then treated with 2 commercial monoclonal antibodies (100 ng/well) against Erk-1 and Erk-2 (1 hr at RT; available from Santa Cruz Biotechnology). To detect other molecules, this step can easily be modified by substituting a monoclonal antibody detecting any of the above described molecules. After 3-5 rinses with PBS, the plates are stored at 4°C until use.

A431 cells are seeded at 20,000/well in a 96-well Loprodyne filterplate and cultured overnight in growth medium. The cells are then starved for 48 hr in basal medium (DMEM) and then treated with EGF (6 ng/well) or 50 µl of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10 ng/well) is used in place of A431 extract. Plates are then treated with a commercial polyclonal (rabbit) antibody (1 µg/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation by IL-21 or a molecule induced by IL-21.

### Example 21: Method of Determining Alterations in the IL-21 Gene.

RNA isolated from entire families or individual patients presenting with a phenotype of interest (such as a disease) is be isolated. cDNA is then generated from these RNA samples using protocols known in the art (see, Sambrook, *et al., supra*) The cDNA is then used as a template for PCR, employing primers surrounding regions of interest in SEQ ID NO:1. Suggested PCR conditions consist of 35 cycles at 95°C for 30 seconds; 60-120 seconds at 52-58°C; and 60-120 seconds at 70°C, using buffer solutions described by Sidransky and colleagues (*Science* **252:**706 (1991)).

PCR products are then sequenced using primers labeled at the 5' end with T4 polynucleotide kinase, employing SequiTherm Polymerase (Epicentre Technologies). The intron-exon borders of selected exons of IL-21 are also determined and genomic PCR products analyzed to confirm the results. PCR products harboring suspected mutations in IL-21 are then cloned and sequenced to validate the results of the direct sequencing.

PCR products of IL-21 are cloned into T-tailed vectors as described by Holton and Graham (*Nucl. Acids Res.* **19:**1156 (1991)) and sequenced with T7 polymerase (United States Biochemical). Affected individuals are identified by mutations in IL-21 not present in unaffected individuals.

Genomic rearrangements are also observed as a method of determining alterations in the IL-21 gene. Genomic clones isolated according to Example 2 are nick-translated with digoxigenindeoxy-uridine 5'-triphosphate (Boehringer Manheim), and FISH performed as described by Johnson and coworkers (*Methods Cell Biol*. **35:**73-99 (1991)). Hybridization with the labeled probe is carried out using a vast excess of human cot-1 DNA for specific hybridization to the IL-21 genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters (Johnson, C., *et al., Genet. Anal. Tech. Appl*. **8:**75 (1991)). Image collection, analysis and chromosomal fractional length measurements are performed using the ISee Graphical Program System. (Inovision Corporation, Durham, NC). Chromosome alterations of the genomic region of IL-21 (hybridized by the probe) are identified as insertions, deletions, and translocations. These IL-21 alterations are used as a diagnostic marker for an associated disease.

### Example 22: Method of Detecting Abnormal Levels of IL-21 in a Biological Sample.

IL-21 polypeptides can be detected in a biological sample, and if an increased or decreased level of IL-21 is detected, this polypeptide is a marker for a particular phenotype. Methods of detection are numerous, and thus, it is understood that one skilled in the art can modify the following assay to fit their particular needs.

For example, antibody-sandwich ELISAs are used to detect IL-21 in a sample, preferably a biological sample. Wells of a microtiter plate are coated with specific antibodies to IL-21, at a final concentration of 0.2 to 10 µg/ml. The antibodies are either monoclonal or polyclonal and are produced by the method described in Example 10. The wells are blocked so that non-specific binding of IL-21 to the well is reduced.

The coated wells are then incubated for > 2 hours at RT with a sample containing IL-21. Preferably, serial dilutions of the sample should be used to validate results. The plates are then washed three times with deionized or distilled water to remove unbounded IL-21.

Next, 50 µl of specific antibody-alkaline phosphatase conjugate, at a concentration of 25-400 ng, is added and incubated for 2 hours at room temperature. The plates are again washed three times with deionized or distilled water to remove unbounded conjugate.

Add 75 µl of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot IL-21 polypeptide concentration on the X-axis (log scale) and fluorescence or absorbance of the Y-axis (linear scale). Interpolate the concentration of the IL-21 in the sample using the standard curve.

### Example 23: Formulating a Polypeptide.

The IL-21 composition will be formulated and dosed in a fashion consistent with good medical practice, taking into account the clinical condition of the individual patient (especially the side effects of treatment with the IL-21 polypeptide alone), the site of delivery, the method of administration, the scheduling of administration, and other factors known to practitioners. The "effective amount" for purposes herein is thus determined by such considerations.

As a general proposition, the total pharmaceutically effective amount of IL-21 administered parenterally per dose will be in the range of about 1 µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, IL-21 is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions containing IL-21 are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

IL-21 is also suitably administered by sustained-release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U., *et al., Biopolymers* **22:**547-556 (1983)), poly (2- hydroxyethyl methacrylate) (Langer, R., *et al., J. Biomed. Mater. Res.* **15**:167-277 (1981); Langer, R. *Chem. Tech*. **12:**98-105 (1982)), ethylene vinyl acetate (Langer, R., *et al*.) or poly-D- (-)-3-hydroxybutyric acid (EP 133,988). Sustained-release compositions also include liposomally entrapped IL-21 polypeptides. Liposomes containing the IL-21 are prepared by methods known *per se* (DE 3,218,121; Epstein, *et al., Proc. Natl. Acad. Sci. USA* **82**:3688-3692 (1985); Hwang, *et al., Proc. Natl. Acad. Sci. USA* **77**:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324). Ordinarily, the liposomes are of the small (about 200-800 Angstroms) unilamellar type in which the lipid content is greater than about 30 mol. percent cholesterol, the selected proportion being adjusted for the optimal secreted polypeptide therapy.

For parenteral administration, in one embodiment, IL-21 is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting IL-21 uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

IL-21 is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

IL-21 used for therapeutic administration can be sterile. Sterility is readily accomplished by filtration through sterile filtration membranes (e.g., 0.2 micron membranes). Therapeutic polypeptide compositions generally are placed into a container having a sterile access port, for example, an intravenous solution bag or vial having a stopper pierceable by a hypodermic injection needle.

IL-21 polypeptides ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous IL-21 polypeptide solution, and the resulting mixture is lyophilized. The infusion solution is prepared by reconstituting the lyophilized IL-21 polypeptide using bacteriostatic Water-For-Injection (WFI).

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, IL-21 may be employed in conjunction with other therapeutic compounds.

### Example 24: Method of Treating Decreased Levels of IL-21.

The present invention relates to a method for treating an individual in need of a decreased level of IL-21 activity in the body comprising, administering to such an individual a composition comprising a therapeutically effective amount of IL-21 antagonist. Preferred antagonists for use in the present invention are IL-21-specific antibodies.

Moreover, it will be appreciated that conditions caused by a decrease in the standard or normal expression level of IL-21 in an individual can be treated by administering IL-21, preferably in the secreted form. Thus, the invention also provides a method of treatment of an individual in need of an increased level of IL-21 polypeptide comprising administering to such an individual a pharmaceutical composition comprising an amount of IL-21 to increase the activity level of IL-21 in such an individual.

For example, a patient with decreased levels of IL-21 polypeptide receives a daily dose 0.1-100 µg/kg of the polypeptide for six consecutive days. Preferably, the polypeptide is in the secreted form. The exact details of the dosing scheme, based on administration and formulation, are provided in Example 23.

### Example 25: Method of Treating Increased Levels of IL-21.

The present invention also relates to a method for treating an individual in need of an increased level of IL-21 activity in the body comprising administering to such an individual a composition comprising a therapeutically effective amount of IL-21 or an agonist thereof.

Antisense technology is used to inhibit production of IL-21. This technology is one example of a method of decreasing levels of IL-21 polypeptide, preferably a secreted form, due to a variety of etiologies, such as cancer.

For example, a patient diagnosed with abnormally increased levels of IL-21 is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

### Example 26: Method of Treatment Using Gene Therapy.

One method of gene therapy transplants fibroblasts, which are capable of expressing IL-21 polypeptides, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37°C for approximately one week.

At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T., *et al*., *DNA* **7:**219-25 (1988)), flanked by the long terminal repeats of the Moloney murine sarcoma virus, is digested with *Eco* RI and *Hin* dIII and subsequently treated with calf intestinal phosphatase. The linear vector is fractionated on agarose gel and purified, using glass beads.

The cDNA encoding IL-21 can be amplified using PCR primers which correspond to the 5' and 3' end sequences respectively as set forth in Example 1. Preferably, the 5' primer contains an *Eco* RI site and the 3' primer includes a *Hin* dIII site. Equal quantities of the Moloney murine sarcoma virus linear backbone and the amplified *Eco* RI and *Hin* dIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector contains properly inserted IL-21.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the IL-21 gene is then added to the media and the packaging cells transduced with the vector. The packaging cells now produce infectious viral particles containing the IL-21 gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether IL-21 protein is produced.

The engineered fibroblasts are then transplanted onto the host, either alone or after having been grown to confluence on cytodex 3 microcarrier beads.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference.

Further, the Sequence Listing submitted herewith, and each of the Sequence Listings submitted with U.S. Provisional Application Serial No. 60/087,340, filed on May 29, 1998, copending U.S. Provisional Application Serial No. 60/099,805, filed on September 10, 1998, and copending U.S. Provisional Application Serial No. 60/131,965, filed on April 30, 1999 (to each of which the present application claims benefit of the filing dates under 35 U.S.C. § 119(e)), in both computer and paper forms are hereby incorporated by reference in their entireties.

## Claims

1. A polynucleotide comprising a nucleic acid selected from the group consisting of:
(a) a nucleic acid encoding a polypeptide comprising amino acid residues +1 to +160 as set forth in SEQ ID NO:4;
(b) a nucleic acid encoding a polypeptide comprising amino acid residues +19 to +27, +30 to 38, +40 to 48, +57 to +64, +58 to +67, +72 to +77, +99 to +105, +105 to +113, +121 to +128, +129 to +137, +139 to +147, +151 to +159, +47 to +160, +56 to +160 or +65 to +160 as set forth in SEQ ID NO:4;
(c) a nucleic acid encoding a polypeptide comprising amino acid residues n² to +160 of SEQ ID NO:4, where n² is an integer from +1 to +28;
(d) a nucleic acid encoding a polypeptide comprising amino acid residues +1 to m² of SEQ ID NO:4, where m² is an integer from +129 to +160;
(e) a nucleic acid encoding a polypeptide comprising amino acid residues n² to m² of SEQ ID NO:4, where n² is an integer from +1 to +28 and m² is an integer from +129 to +160;
(f) a nucleic acid encoding a polypeptide comprising a fragment of amino acid residues +1 to +160 of SEQ ID NO:4, wherein the fragment modulates immune system cell proliferation and/or differentiation;
(g) a nucleic acid comprising part of the coding sequence of SEQ ID NO:3 encoding at least the mature or a secreted form of the polypeptide;
(h) a nucleic acid comprising at least 30 contiguous nucleotides of SEQ ID NO:3;
(i) the nucleic acid of (h) comprising at least 50 contiguous nucleotides of SEQ ID NO:3;
(j) a nucleic acid encoding a polypeptide comprising at least 30 contiguous amino acid residues of SEQ ID NO:4;
(k) the nucleic acid of (j) encoding a polypeptide comprising at least 50 contiguous amino acid residues of SEQ ID NO:4;
(I) a nucleic acid encoding a polypeptide which is at least 90%, 95%, 96%, 97%, 98% or 99% identical to the polypeptide encoded by the nucleic acid of any one of (a) to (k), wherein said polypeptide modulates immune system cell proliferation and/or differentiation;
(m) a nucleic acid which is at least 90%, 95%, 96%, 97%, 98% or 99% identical to the nucleic acid of any one of (a) to (k), wherein said nucleic acid encodes a polypeptide which modulates immune system cell proliferation and/or differentiation;
(n) a nucleic acid which hybridizes to the complement of the nucleic acid set forth in any one of (a) to (k), wherein said hybridization occurs under conditions consisting of hybridization in a buffer consisting of 50% formamide, 5x SSC, 50 mM sodium phosphate (pH 7.6), 5x Denhardt's solution, 10% dextran sulfate, and 20µg/ml denatured, sheared salmon sperm DNA at 42°C and wash in a solution consisting of 0.1 x SSC at 65°C;
(o) the nucleic acid of any one of (a) to (n), wherein said nucleic acid does not encode an N-terminal methionine; and
(p) the nucleic acid of (a) to (n), wherein said nucleic acid encodes an N-terminal methionine;
or a polynucleotide complementary to the nucleic acid of (a) to (p).

2. A polynucleotide comprising a nucleic acid selected from the group consisting of :
(a) a nucleic acid encoding a polypeptide comprising a full length amino acid sequence encoded by the cDNA contained in ATCC Deposit No. 209665;
(b) a nucleic acid encoding a polypeptide comprising an amino acid sequence of the conserved domain I, domain II, domain III, domain IV and/or an antigenic epitope of the polypeptide having the amino acid sequence encoded by the cDNA contained in ATCC Deposit No. 209665;
(c) a nucleic acid encoding a polypeptide comprising a portion of the full-length amino acid sequence encoded by the cDNA contained in ATCC Deposit No. 209665, wherein said portion excludes one or more amino acid residues from the N-terminus up to the cysteine residue at position 29 of said full-length amino acid sequence;
(d) a nucleic acid encoding a polypeptide comprising a portion of the full-length amino acid sequence encoded by the cDNA contained in ATCC Deposit No. 209665, wherein said portion excludes one or more amino acid residues from the C-terminus up to the proline residue at position 129 of said full-length amino acid sequence;
(e) a nucleic acid encoding a polypeptide comprising a portion of the full-length amino acid sequence encoded by the cDNA contained in ATCC Deposit No. 209665, wherein said portion excludes one or more amino acid residues from the N-terminus up to the cysteine residue at position 29 and one or more amino acid residues from the C-terminus up to the proline residue at position 129 of said full-length amino acid sequence;
(f) a nucleic acid encoding a polypeptide comprising a fragment of the full-length amino acid sequence encoded by the cDNA contained in ATCC Deposit No. 209665, wherein the fragment modulates immune system cell proliferation and/or differentiation;
(g) a nucleic acid comprising the coding sequence of the cDNA contained in ATCC Deposit No. 209665 encoding at least the mature or a secreted form of the polypeptide;
(h) a nucleic acid comprising at least 30 contiguous nucleotides of the cDNA contained in ATCC Deposit No. 209665;
(i) the nucleic acid of (h) comprising at least 50 contiguous nucleotides of the cDNA contained in ATCC Deposit No. 209665;
(j) a nucleic acid encoding a polypeptide comprising at least 30 contiguous amino acid residues encoded by the cDNA contained in ATCC Deposit No. 209665;
(k) the nucleic acid of (j) encoding a polypeptide comprising at least 50 contiguous amino acid residues encoded by the cDNA contained in ATCC Deposit No. 209665;
(I) a nucleic acid encoding a polypeptide which is at least 90%, 95%, 96%, 97%, 98% or 99% identical to the polypeptide encoded by the nucleic acid of any one of (a) to (k), wherein said polypeptide modulates immune system cell proliferation and/or differentiation;
(m) a nucleic acid which is at least 90%, 95%, 96%, 97%, 98% or 99% identical to the nucleic acid of any one of (a) to (k), wherein said nucleic acid encodes a polypeptide which modulates immune system cell proliferation and/or differentiation;
(n) a nucleic acid which hybridizes to the complement of the nucleic acid set forth in any one of (a) to (k), wherein said hybridization occurs under conditions consisting of hybridization in a buffer consisting of 50% formamide, 5x SSC, 50 mM sodium phosphate (pH 7.6), 5x Denhardt's solution, 10% dextran sulfate, and 20µg/ml denatured, sheared salmon sperm DNA at 42°C and wash in a solution consisting of 0.1x SCC at 65°C;
(o) the nucleic acid of any one of (a) to (n), wherein said nucleic acid encodes an N-terminal methionine; and
(p) the nucleic acid of any one of (a) to (n), wherein said nucleic acid encodes an N-terminal methionine;
or a polynucleotide complementary to the nucleic acid of (a) to (p).

3. A polynucleotide comprising a nucleic acid selected from the group consisting of:
(a) a nucleic acid encoding a polypeptide comprising amino acid residues +1 to +173 as set forth in SEQ ID NO:32;
(b) a nucleic acid encoding a polypeptide comprising acid residues +70 to +77, +85 to +90, +112 to +118, +134 to +141, +18 to +23 or +60 to +65 as set forth in SEQ ID NO:32;
(c) a nucleic acid encoding a polypeptide comprising amino acid residues n⁶ to +173 of SEQ ID NO:32, where n⁶ is an integer from +1 to +168, wherein said polypeptide modulates immune system cell proliferation and/or differentiation;
(d) a nucleic acid encoding a polypeptide comprising amino acid residues +1 to m⁶ of SEQ ID NO:32, where m⁶ is an integer from +6 to +173, wherein said polypeptide modulates immune system cell proliferation and/or differentiation;
(e) a nucleic acid encoding a polypeptide comprising amino acid residues n⁶ to m⁶ of SEQ ID NO:32, where n⁶ is an integer from +1 to +168 and m⁶ is an integer from +6 to +173, wherein said polypeptide modulates immune system cell proliferation and/or differentiation;
(f) a nucleic acid encoding a polypeptide comprising a fragment of amino acid residues +1 to +173 of SEQ ID NO:32, wherein the fragment modulates immune system cell proliferation and/or differentiation;
(g) a nucleic acid comprising part of the coding sequence of SEQ ID NO:31 encoding at least the mature or a secreted form of the polypeptide;
(h) a nucleic acid comprising at least 30 contiguous nucleotides of SEQ ID NO:31;
(i) the nucleic acid of (h) comprising at least 50 contiguous nucleotides of SEQ ID NO:31;
(j) a nucleic acid encoding a polypeptide comprising at least 30 contiguous amino acid residues of SEQ ID NO:32;
(k) the nucleic acid of (j) encoding a polypeptide comprising at least 50 contiguous amino acid residues of SEQ ID NO:32;
(l) a nucleic acid encoding a polypeptide which is at least 90%, 95%, 96%, 97%, 98% or 99% identical to the polypeptide encoded by the nucleic acid of any one of (a) to (k), wherein said polypeptide modulates immune system cell proliferation and/or differentiation;
(m) a nucleic acid which is at least 90%, 95%, 96%, 97%, 98% or 99% identical to the nucleic acid of any one of (a) to (k), wherein said nucleic acid encodes a polypeptide which modulates immune system cell proliferation and/or differentiation;
(n) a nucleic acid which hybridizes to the complement of the nucleic acid set forth in any one of (a) to (k), wherein said hybridization occurs under conditions consisting of hybridization in a buffer consisting of 50% formamide, 5x SSC, 50 mM sodium phosphate (pH 7.6), 5x Denhardt's solution, 10% dextran sulfate, and 20 µg/ml denatured, sheared salmon sperm DNA at 42°C and wash in a solution consisting of 0.1x SSC at 65°C;
(o) the nucleic acid of any one of (a) to (n), wherein said nucleic acid does not encode an N-terminal methionine; and
(p) the nucleic acid of any one of (a) to (n), wherein said nucleic acid encodes an N-terminal methionine;
or a polynucleotide complementary to the nucleic acid of (a) to (p).

4. A polynucleotide comprising a nucleic acid selected from the group consisting of:
(a) a nucleic acid encoding a polypeptide comprising a full-length amino acid sequence encoded by the cDNA contained in ATCC Deposit No. PTA-70;
(b) a nucleic acid encoding a polypeptide comprising an amino acid sequence of the conserved domain I, domain II, domain III, domain IV and/or an antigenic epitope of the polypeptide having the amino acid sequence encoded by the cDNA contained in ATCC Deposit No. PTA-70;
(c) a nucleic acid encoding a polypeptide comprising a fragment of the full-length amino acid sequence encoded by the cDNA contained in ATCC Deposit No. PTA-70, wherein the fragment modulates immune system cell proliferation and/or differentiation;
(d) a nucleic acid comprising the coding sequence of the cDNA contained in ATCC Deposit No. PTA-70 encoding at least the mature or a secreted form of the polypeptide;
(e) a nucleic acid comprising at least 30 contiguous nucleotides of the cDNA contained in ATCC Deposit No. PTA-70;
(f) the nucleic of (e) comprising at least 50 contiguous nucleotides of the cDNA contained in ATCC Deposit No. PTA-70;
(g) a nucleic acid encoding a polypeptide comprising at least 30 contiguous amino acid residues encoded by the cDNA contained in ATCC Deposit No. PTA-70;
(h) the nucleic acid of (g) encoding a polypeptide comprising at least 50 contiguous amino acid residues encoded by the cDNA contained in ATCC Deposit No. PTA-70;
(i) a nucleic acid encoding a polypeptide which is at least 90%, 95%, 96%, 97%, 98% or 99% identical to the polypeptide encoded by the nucleic acid of any one of (a) to (h), wherein said polypeptide modulates immune system cell proliferation and/or differentiation;
(j) a nucleic acid which is at least 90%, 95%, 96%, 97%, 98% or 99% identical to the nucleic acid of any one of (a) to (h), wherein said nucleic acid encodes a polypeptide which modulates immune system cell proliferation and/or differentiation;
(k) a nucleic acid which hybridizes to the complement of the nucleic acid set forth in any one of (a) to (h), wherein said hybridization occurs under conditions consisting of hybridization in a buffer consisting of 50% formamide, 5x SSC, 50 mM sodium phosphate (pH 7.6), 5x Denhardt's solution, 10% dextran sulfate, and 20 µg/ml denatured, sheared salmon sperm DNA at 42°C and wash in a solution consisting of 0.1x SSC at 65°C;
(l) the nucleic acid of any one of (a) to (k), wherein said nucleic acid does not encode an N-terminal methionine; and
(m) the nucleic acid of any one of (a) to (k), wherein said nucleic acid encodes an N-terminal methionine;
or a polynucleotide complementary to the nucleic acid of (a) to (m).

5. The polynucleotide of any one of claims 1 to 4, wherein said polynucleotide is DNA, RNA, cDNA or genomic DNA.

6. The polynucleotide of any one of claims 1 to 5, wherein said polynucleotide is double stranded or single stranded.

7. The polynucleotide of any one of claims 1 to 6 fused to a heterologous polynucleotide.

8. The polynucleotide of claim 7, wherein the heterologous polynucleotide encodes a heterologous polypeptide.

9. The polynucleotide of claim 8, wherein said heterologous polypeptide is fused to a polypeptide encoded by the polynucleotide of any one of 1 to 6.

10. The polynucleotide of any one of claims 1 to 9, wherein said polynucleotide is immobilized.

11. The polynucleotide of any one of claims 1 to 10, wherein said polynucleotide is labeled.

12. A vector comprising the polynucleotide of any one of claims 1 to 11.

13. The vector of claim 12, wherein the polynucleotide is operably linked to a regulatory control sequence.

14. A method of producing a host cell comprising genetically engineering cells with the polynucleotide of any one of claims 1 to 11 or the vector of claim 12 or 13.

15. A host cell genetically engineered with the polynucleotide of any one of claims 1 to 11, comprising the vector of claim 12 or 13 or produced by the method of claim 14.

16. The host cell of claim 15, wherein the polynucleotide is operably associated with a heterologous regulatory control sequence.

17. The host cell of claim 15 or 16, wherein said host cell is a prokaryotic cell, eukaryotic cell, fungal cell, insect cell, animal cell, mammalian cell, Cos cell, CHO cell or E. coli cell.

18. A method of producing a polypeptide comprising culturing the host cell of any one of claims 15 to 17 under conditions such that the polypeptide encoded by said polynucleotide is expressed; and recovering said polypeptide.

19. A method of producing a polypeptide comprising expressing a polypeptide encoded by the polynucleotide of any one of claims 1 to 11 and recovering said polypeptide.

20. A polypeptide comprising an amino acid sequence encoded by a polynucleotide of any one of claims 1 to 11.

21. The polypeptide of claim 20, wherein said polypeptide is chemically synthesized or is obtainable from the host cell of any one of claims 15 to 17 or which is obtainable by the method of claim 18 or 19.

22. The polypeptide of claim 20 or 21, wherein the polypeptide is labeled, modified or pegylated.

23. The polypeptide of claim 22, wherein the label is a toxin, radioisotope or fluorescent label.

24. The polypeptide of any one of claims 20 to 23 fused to a heterologous polypeptide.

25. The polypeptide of any one of claims 20 to 24, wherein said polypeptide lacks an N-terminal methionine.

26. The polypeptide of any one of claims 20 to 24, wherein said polypeptide has an N-terminal methionine.

27. The polypeptide of one of claims 20 to 26, wherein said polypeptide is immobilized.

28. An antibody specifically recognizing the polypeptide of any one of claims 20 to 27 or which is an anti-idiotypic antibody of the polypeptide of any one of claims 20 to 27.

29. The antibody of claim 28 which is polyclonal, monoclonal, chimeric, a single chain, single chain Fv, human antibody, humanized antibody or Fab fragment.

30. The antibody of claim 28 or 29, wherein said antibody is labeled.

31. The antibody of claim 30, wherein said label is a toxin, radioisotope, or fluorescent label.

32. The antibody of any one of claims 28 to 31, wherein said antibody is immobilized.

33. A polynucleotide which specifically hybridizes to a polynucleotide of any one of claims 1 to 11.

34. An antagonist of the polypeptide of any one of claims 20 to 27 which is an antibody of any one of claims 28 to 32 capable of antagonizing the biological activity of said polypeptide or an antisense molecule or triple helix forming molecule capable of binding and inhibiting expression of a polynucleotide of any one of claims 1 to 11.

35. A composition comprising the polynucleotide of any one of claims 1 to 11, the polypeptide of any one of claims 20 to 27, the antibody of any one of claims 28 to 32 or the antagonist of claim 34.

36. A pharmaceutical composition comprising the polynucleotide of any one of claims 1 to 11, the polypeptide of any one of claims 20 to 27, the antibody of any one of claims 28 to 32 or the antagonist of claim 34 and optionally a pharmaceutically acceptable carrier.

37. A diagnostic composition comprising the polynucleotide of any one of claims 1 to 11 and 33, the polypeptide of any one of claims 20 to 27 or the antibody of any one of claims 28 to 32.

38. Use of the polynucleotide of any one of claims 1 to 11 and 33, the polypeptide of any one of claims 20 to 27, the antibody of any one of claims 28 to 32 or the antagonist of claim 34 for the preparation of a pharmaceutical composition for treating deficiencies or disorders of the immune system or of hematopoietic cells, for modulating hemostatic or thrombolytic activity, for treating autoimmune disorders, allergic reactions or conditions, anaphylaxis, hypersensitivity to an antigenic molecule or blood group incompatibility, for treating or preventing organ rejection or graft-versus-host-disease, for treating inflammation, hyperproliferative disorders or infections or for the regeneration of tissues.

39. A method of diagnosing a pathological condition or a susceptibility to a pathological condition in a subject related to the expression or activity of a secreted protein comprising:
(a) determining the presence or absence of a mutation in the polynucleotide of any one of claims 1 to 11; and
(b) diagnosing a pathological condition or a susceptibility to a pathological condition based on the presence or absence of said mutation.

40. A method of diagnosing a pathological condition or a susceptibility to a pathological condition in a subject related to the expression or activity of a secreted protein comprising:
(a) determining the presence or amount of expression of the polypeptide of any one of claims 20 to 27 in a biological sample; and
(b) diagnosing a pathological condition or a susceptibility to a pathological condition based on the presence or amount of expression of the polypeptide.

41. A method for identifying a binding partner to the polypeptide of any one of claims 20 to 27 comprising:
(a) contacting said polypeptide with a candidate binding partner; and
(b) determining whether the candidate binding partner effects an activity of the polypeptide and thereby identifying a binding partner.

42. A method for identifying an agonist or antagonist of the polypeptide of any one of claims 20 to 27 comprising:
(a) incubating a candidate compound with said polypeptide;
(b) assaying a biological activity of the polypeptide of (a); and
(c) determining whether said biological activity has been changed by incubating said candidate compound with said polypeptide and thereby identifying the candidate compound as an agonist or antagonist.

43. A method for the production of a pharmaceutical composition comprising the steps of the method of claim 41 or 42 and furthermore the step of formulating the compound identified in step (b) of the method of claim 41 or in step (c) of the method of claim 42 in a pharmaceutically acceptable form.

44. A method of identifying an activity in a biological assay, wherein the method comprises:
(a) expressing the polynucleotide of any one of claims 1 to 11 in a cell;
(b) isolating the supernatant;
(c) detecting an activity in a biological assay; and
(d) identifying the protein in the supernatant having the activity.

45. Use of the polynucleotide of any one of claims 1 to 11 and 33, the polypeptide of any one of claims 20 to 27 or the antibody of any one of claims 28 to 32 for screening compounds which bind IL-22.

46. Use of the polynucleotide of any one of claims 1 to 11 and 33, the polypeptide of any one of claims 20 to 27 or the antibody of any one of claims 28 to 32 for screening compounds which are antagonists of IL-22.

47. Use of the polynucleotide of any one of claims 1 to 11 and 33, the polypeptide of any one of claims 20 to 27 or the antibody of any one of claims 28 to 32 for screening compounds which are agonists of IL-22.

48. Use of the polynucleotide of any one of claims 1 to 11 and 33, the polypeptide of any one of claims 20 to 27 or the antibody of any one of claims 28 to 32 for detecting compounds that bind the polynucleotide of any one of claims 1 to 11 and 33, the polypeptide of any one of claims 20 to 27 or the antibody of any one of claims 28 to 32.
